(19) Europäisches Patentamt
European Patent Office
Office européen des brevets

(11) **EP 4 180 423 A1**

(12) **EUROPEAN PATENT APPLICATION**
published in accordance with Art. 153(4) EPC

(43) Date of publication:
**17.05.2023 Bulletin 2023/20**

(21) Application number: **21837785.1**

(22) Date of filing: **28.06.2021**

(51) International Patent Classification (IPC):
***C07D 215/233*** $^{(2006.01)}$ ***A61K 31/47*** $^{(2006.01)}$
***A61K 31/352*** $^{(2006.01)}$ ***C07D 311/22*** $^{(2006.01)}$
***C07D 407/12*** $^{(2006.01)}$

(52) Cooperative Patent Classification (CPC):
**A61K 31/352; A61K 31/47; C07D 215/233; C07D 311/22; C07D 407/12;** Y02A 50/30

(86) International application number:
**PCT/KR2021/008079**

(87) International publication number:
**WO 2022/010150 (13.01.2022 Gazette 2022/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.07.2020 KR 20200083622**

(71) Applicant: **ILAb Co., Ltd.**
**Seongnam-si, Gyeonggi-do 13486 (KR)**

(72) Inventor: **PARK, Kyung Yeon**
**Bucheon-si, Gyeonggi-do 14405 (KR)**

(74) Representative: **Vossius & Partner**
**Patentanwälte Rechtsanwälte mbB**
**Siebertstraße 3**
**81675 München (DE)**

(54) **NOVEL TNF ACTIVITY INHIBITOR COMPOUND, AND PHARMACEUTICALLY ACCEPTABLE SALT THEREOF**

(57) The present invention relates to a novel TNF activity inhibitor compound and a pharmaceutically acceptable salt thereof, a method for prevention, alleviation or treatment comprising administering the compound of the present invention, a method for preparing the compound of the present invention, and a pharmaceutical composition comprising the compound.

【Fig. 1】

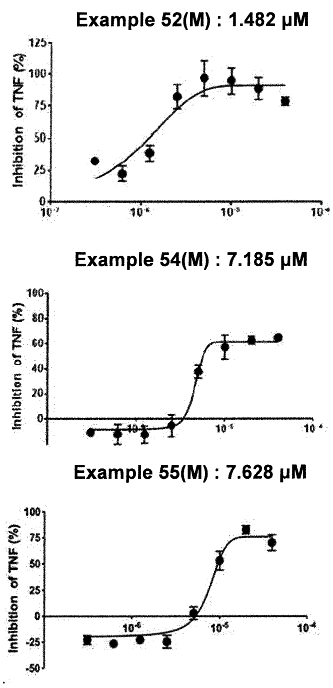


EP 4 180 423 A1

## Description

[Technical Field]

The present invention relates to a novel TNF activity inhibitor compound(s) and a pharmaceutically acceptable salt thereof, a method for prevention, alleviation or treatment comprising administering the compound of the present invention, a method for preparing the compound of the present invention, and a pharmaceutical composition comprising the compound.

[Background Technology]

It has been known that tumor necrosis factor (TNF), especially TNF, is released from inflammatory cells and causes a variety of cytotoxic, immune and inflammatory responses. TNF is the main cause of autoimmune inflammatory diseases, such as rheumatoid arthritis, inflammatory bowel disease, psoriasis, ankylosing spondylitis and the like, and is known to cause severe sepsis and septic shock when it is released into the blood and acts on the whole body. As described above, since TNF is a factor widely related to the immune system of a living body, a drug inhibiting TNF is being actively developed. To date, various anti-TNF blockbuster biologics such as etanercept (Enbrel®), adalimumab (Humira®), and infliximab (Remicade®) have been actively used as a therapeutic agent for various TNF-related diseases, including rheumatoid arthritis. Although anti-TNF biopharmaceuticals show excellent efficacy in a short period of time, they have obvious disadvantages - they are expensive and require repeated injections, so patients are highly reluctant; about one-third of the patients have no therapeutic effect; even patients who responded to the drug develop tolerance within a few years due to immunogenic side effect; and they have difficulty in storage because low temperature storage is essential - and an unmet medical need.

As a strategy to overcome this, there have been efforts to discover small molecule substances which act like antibodies by binding directly to cytokines or their receptors and can be administered orally [He et al. (2005). Science 310(5750): 1022-1025]. However, the binding of cytokines and their receptors occurs over a large area as a protein-protein binding, and it is not effective to inhibit it with a small molecule substance so far.

Though a small molecule substance that directly binds to TNF and disassembles a TNF trimer has been published in a paper [He et al. (2005). Science 310(5750): 1022-1025], it seems that further studies were stopped due to weak activity. On the other hand, a number of small molecule signal transduction inhibitors that block intracellular signal transduction are under development, and papers on TNF expression or secretion inhibitors, whose mechanisms are not clear, have been published. However, there is no *in vivo* activity data, and there have been no successful cases of the development of drugs with small molecules that directly inhibit the binding between TNF and TNF receptors (TNFR1 or TNFR2).

[Prior Art Document]

[Patent]

(Patent 1) Korean Patent No. 10-1282801
(Patent 2) Korean Patent No. 10-1934651
(Patent 3) Korean Patent No. 10-1982667

[Detailed Description of the Invention]

[Technical Subject]

An object of the present invention is to provide a novel compound exhibiting an excellent TNF activity inhibitory effect.

Another object of the present invention is to provide a method for effectively preventing or treating TNF-related diseases by exhibiting an excellent TNF activity inhibitory effect, or a pharmaceutical composition for the same.

Another object of the present invention is to provide a health functional food composition that can effectively prevent or improve TNF-related diseases by exhibiting an excellent TNF activity inhibitory effect.

Another object of the present invention is to provide a reagent composition that inhibits the activity of TNF *in vitro.*

[Technical Solution]

In order to achieve the above objects, the present invention provides a compound of Formula I below or a pharmaceutically acceptable salt thereof.

[Formula I]

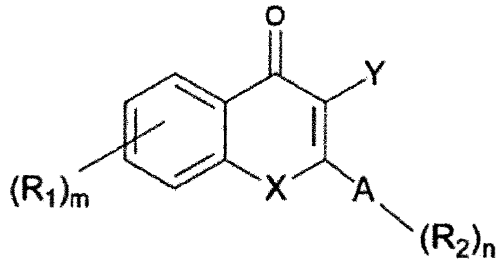

wherein,

X is $-C(R_3)_2-$, $-N(R_3)-$, -O- or -S-,
Y is $-OR_a$, $-SR_a$, $-C(O)R_a$, $-C(O)OR_a$, $-C(O)N(R_a)(R_b)$, $-N(R_a)(R_b)$, $-N(R_a)C(O)R_b$, - $N(R_a)C(O)OR_b$, or $-N(R_a)S(O)OR_b$,
A is $C_3$-$C_8$ cycloalkyl; 4- to 10-membered heterocycloalkyl containing 1 to 4 heteroatoms independently selected from the group consisting ofN, O and S; $C_6$-$C_{10}$ aryl; or 5- to 10-membered heteroaryl containing 1 to 4 heteroatoms independently selected from the group consisting of N, O and S,
$R_1$, $R_2$ and $R_3$ may be the same or different and are each independently H, halo, cyano, - ORc, nitro, $C_1$-$C_6$ alkyl, $C_2$-$C_6$ alkenyl, $C_2$-$C_6$ alkynyl, -SRc, $-C(O)R_c$, $-C(O)OR_c$, -C(O)N(Rc)(Rd), $-N(R_c)(R_d)$, $-N(R_c)C(O)R_d$, $-N(R_c)C(O)OR_d$, or $-N(R_c)S(O)OR_d$,
$R_a$, $R_b$, $R_c$ and $R_d$ may be the same or different and are each independently H or $C_1$-$C_6$ alkyl, and
m and n are each independently an integer from 0 to 4.

In another aspect, the present invention provides a compound of Formula III below or a pharmaceutically acceptable salt thereof.

[Formula III] $M_1$-L-$M_2$

wherein,

$M_1$ and $M_2$ may be the same or different, and are each independently a monomer radical of Formula Ia below, and
L is a linking group covalently bonding $M_1$ and $M_2$, and is selected from Formulas IVa to IVc below,

[Formula Ia]

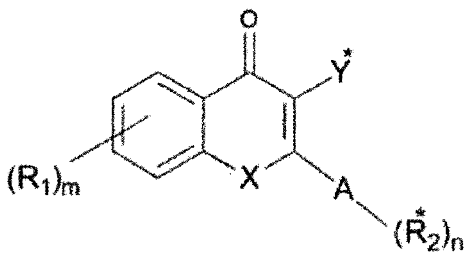

(wherein,

X is $-C(R_3)_2-$, $-N(R_3)-$, -O- or -S-,
Y is $-OR_a$, $-SR_a$, $-C(O)R_a$, $-C(O)OR_a$, $-C(O)N(R_a)(R_b)$, $-N(R_a)(R_b)$, $-N(R_a)C(O)R_b$, - $N(R_a)C(O)OR_b$, or $-N(R_a)S(O)OR_b$,

A is $C_3$-$C_8$ cycloalkyl; 4- to 10-membered heterocycloalkyl containing 1 to 4 heteroatoms independently selected from the group consisting ofN, O and S; $C_6$-$C_{10}$ aryl; or 5- to 10-membered heteroaryl containing 1 to 4 heteroatoms independently selected from the group consisting of N, O and S,

$R_1$, $R_2$ and $R_3$ may be the same or different and each independently H, halo, cyano, -$OR_c$, nitro, $C_1$-$C_6$ alkyl, $C_2$-$C_6$ alkenyl, $C_2$-$C_6$ alkynyl, -SRc, -C(O)$R_c$, -C(O)$OR_c$, -C(O)N($R_c$)($R_d$), - N($R_c$)($R_d$), -N($R_c$)C(O)$R_d$, -N($R_c$)C(O)$OR_d$, or -N($R_c$)S(O)$OR_d$,

$R_a$, $R_b$, $R_c$ and $R_d$ may be the same or different and are each independently H or $C_1$-$C_6$ alkyl,

m and n are each independently an integer of 0 to 4,

the monomer unit $M_1$ or $M_2$ is bonded to the linking group L through a bonding site * located in either Y or $R_2$, and when Y or $R_2$ has the above bonding site *, Y or $R_2$ contains N, O or S atoms, and at the bonding site *, -C(O)-, -NH-, -N($C_1$-$C_6$ alkyl)-, -O- or -S- radical is located),

[Formula IVa] *R-Z-R'*

[Formula IVb] *-[$(CH_2)_2$-O]$_p$-$(CH_2)_2$-*

[Formula IVc]

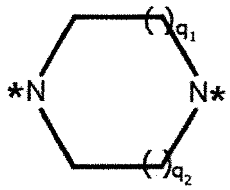

(wherein,

* represents a bonding site between the monomer unit $M_1$ or $M_2$ and the linking group L,

R or R' is each independently $C_1$-$C_{10}$ alkylene, $C_2$-$C_{10}$ alkenylene, or $C_2$-$C_{10}$ alkynylene;

Z is a single bond; -O-; -S-; -NH-; -N($C_1$-$C_6$ alkyl)-; $C_3$-$C_8$ cycloalkylene; 4- to 10-membered heterocycloalkyl containing 1 to 4 heteroatoms independently selected from the group consisting of N, O and S; $C_6$-$C_{10}$ aryl; or 5- to 10-membered heteroaryl containing 1 to 4 heteroatoms independently selected from the group consisting of N, O and S;

p is an integer from 1 to 5, and

$q_1$ and $q_2$ are each independently an integer from 0 to 5, but $q_1$ and $q_2$ are not 0 at the same time).

The present invention provides a pharmaceutical composition for preventing or treating TNF-related diseases, comprising the compound or a pharmaceutically acceptable salt, solvate, racemate or stereoisomer thereof as an active ingredient.

In another aspect, the present invention provides a method for preventing, improving or treating a TNF-related disease, comprising administering to a subject the compound or a pharmaceutically acceptable salt, solvate, racemate or stereoisomer thereof in a pharmaceutically effective amount.

In another aspect, the present invention provides a health functional food composition for preventing or improving TNF-related diseases, comprising the compound or a pharmaceutically acceptable salt, solvate, racemate or stereoisomer thereof as an active ingredient.

In another aspect, the present invention provides a reagent composition for inhibiting TNF activity *in vitro,* comprising the compound or a pharmaceutically acceptable salt, solvate, racemate or stereoisomer thereof as an active ingredient.

[Effect of the Invention]

The novel compounds of the present invention exhibit highly selective inhibitory activity against TNF, and are useful for preventing, improving or treating TNF-related diseases.

The conventional medicaments used for TNF-related diseases are protein-based TNF-inhibiting biopharmaceuticals, which are expensive, have difficulties in storage because low-temperature storage is essential, and have

problems such as tolerance. In addition, these biopharmaceuticals were inconvenient to co-administer with other therapeutic compounds, and it was virtually impossible to develop a combination drug. On the other hand, the TNF-inhibiting compound provided by the present invention not only exhibits excellent efficacy, but also has advantages such as low cost, non-invasive oral administration, non-immunogenicity, and no need for refrigeration. Further, it is very easy to develop a co-administration with other conventional therapeutic compounds or a combination drug, so it can be usefully used as a composition for preventing, improving, or treating TNF-related diseases.

[Brief Description of the Drawings]

Fig. 1 is a graph showing the results of measuring TNF-induced apoptosis inhibitory activity by treating mouse fibroblasts with the compound of the present invention. The horizontal axis represents the treatment concentration (M) of each compound.
Fig. 2 is a graph showing the results of measuring the TNF response inhibitory activity by treating HEK-Blue TNF cells with the compound of the present invention.
Fig. 3 is a graph showing the results of measuring the direct binding activity of the compound of the present invention and TNF, TNFR1 or TNFR2 using a surface plasmon resonance (SPR) analysis method.
Figs. 4 and 5 are graphs showing the results of measuring TNF-TNFR1 or TNF-TNFR2 binding inhibitory activity of the compound of the present invention using a fluorescence resonance energy transfer (FRET) assay.
Fig. 6a shows that the degree of ubiquitination of the upstream protein RIP 1 of the TNF signal transduction pathway is reduced by treatment with the compound of the present invention.
Fig. 6b shows that the levels of the sub-proteins of the TNF signal transduction pathway, phospho-p65, phospho-IKK$\alpha/\beta$, phospho-SAPK/JNK, and phospho-AKT, are reduced by treatment with the compounds of the present invention.
Fig. 7 is a graph showing the death inhibitory activity of TNF-induced mice pretreated with the compound of the present invention.

[Best Mode for Carrying Out the Invention]

Hereinafter, the present invention will be described in more detail.

<u>**Definition**</u>

Unless defined otherwise, all technical terms used herein have the same meaning as commonly understood by a person skilled in the art to which this invention belongs. Moreover, numerical values recited herein are intended to include the meaning of "about" unless explicitly stated otherwise.

Definitions of residues and substituents as used herein are provided below. Unless otherwise specified, each residue has the following definitions and is used in the sense commonly understood by a person skilled in the art.

In accordance with convention used in the art, "

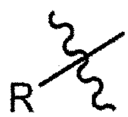 "

is used herein to indicate that a moiety or substituent "R" is attached to the backbone structure.

As used herein, the term "hydrocarbyl" refers to a monovalent group of hydrocarbons, including, but not limited to, aryl, carbocycle, alkyl, alkenyl, alkynyl, and combinations thereof, as defined below. A hydrocarbyl group essentially refers to a group bonded through a carbon atom that does not have an =O or =S substituent and typically has one or more carbon-hydrogen bonds and a basic carbon backbone, but optionally with substituents containing heteroatoms. Thus, groups such as methyl, ethoxyethyl, 2-pyridyl, and even trifluoromethyl are considered as hydrocarbyl for the purposes of this application, but acetyl (having =O substituents on the linking carbon) and ethoxy (the basic backbone is linked via oxygen rather than carbon) groups are not included.

The term "alkyl," as used herein, is a hydrocarbon having primary, secondary, tertiary and/or quaternary carbon atoms and includes saturated aliphatic groups which may be straight-chain or branched. For example, an alkyl group may have 1 to 20 carbon atoms (i.e., $C_1$-$C_{20}$ alkyl), 1 to 10 carbon atoms (i.e., $C_1$-$C_{10}$ alkyl), or 1 to 6 carbon atoms (i.e., $C_1$-$C_6$ alkyl). Unless otherwise defined, in a preferred embodiment, alkyl refers to $C_1$-$C_6$ alkyl. Examples of suitable alkyl

groups include methyl (Me, $-CH_3$), ethyl (Et, $-CH_2CH_3$), 1-propyl (n-Pr, n-propyl, - $CH_2CH_2CH_3$), 2-propyl (i-Pr, i-propyl, $-CH(CH_3)_2$), 1-butyl (n-Bu, n-butyl, $-CH_2CH_2CH_2CH_3$), 2-methyl-1-propyl (i-Bu, i-butyl, $-CH_2CH(CH_3)_2$), 2-butyl (s-Bu, s-butyl, $-CH(CH_3)CH_2CH_3$), 2-methyl-2-propyl (t-Bu, t-butyl, $-C(CH_3)_3$), 1-pentyl (n-pentyl, $-CH_2CH_2CH_2CH_2CH_3$), 2-pentyl ($-CH(CH_3)CH_2CH_2CH_3$), 3-pentyl ($-CH(CH_2CH_3)_2$), 2-methyl-2-butyl ($-C(CH_3)_2CH_2CH_3$), 3-methyl-2-butyl ($-CH(CH_3)CH(CH_3)_2$), 3-methyl-1-butyl ($-CH_2CH_2CH(CH_3)_2$), 2-methyl-1-butyl ($-CH_2CH(CH_3)CH_2CH_3$), 1-hexyl ($-CH_2CH_2CH_2CH_2CH_2CH_3$), 2-hexyl ($-CH(CH_3)CH_2CH_2CH_2CH_3$), 3-hexyl ($-CH(CH_2CH_3)(CH_2CH_2CH_3)$), 2-methyl-2-pentyl ($-C(CH_3)_2CH_2CH_2CH_3$), 3-methyl-2-pentyl ($-CH(CH_3)CH(CH_3)CH_2CH_3$), 4-methyl-2-pentyl ($-CH(CH_3)CH_2CH(CH_3)_2$), 3-methyl-3-pentyl ($-C(CH_3)(CH_2CH_3)_2$), 2-methyl-3-pentyl ($-CH(CH_2CH_3)CH(CH_3)_2$), 2,3-dimethyl-2-butyl ($-C(CH_3)_2CH(CH_3)_2$), 3,3-dimethyl-2-butyl ($-CH(CH_3)C(CH_3)_3$), heptyl ($-(CH_2)_6CH_3$) and octyl ($-(CH_2)_7CH_3$), but are not limited thereto.

As used herein, the term "$C_{x-y}$" or "$C_x$-$C_y$", when used in conjunction with a chemical moiety such as acyl, acyloxy, alkyl, alkenyl, alkynyl or alkoxy, is considered to include a group containing x to y carbons within that moiety. For example, a ($C_1$-$C_6$)alkyl group contains 1 to 6 carbon atoms in the alkyl chain.

The term "acyl," as used herein, refers to the groups -C(=O)-alkyl, -C(=O)-carbocycle, and -C(=O)-heterocycle, wherein alkyl, carbocycle, or heterocycle moieties are as defined herein. Non-limiting examples of "acyl" include $-C(=O)CH_3$, $-C(=O)CH_2CH_3$, $-C(=O)CH(CH_3)_2$, - $C(=O)C(CH_3)_3$, - C(=O)-phenyl, -C(=O)-cyclopropyl, -C(=O)-cyclobutyl, -C(=O)-cyclopentyl, - C(=O)-cyclohexyl, and - a C(=O)-pyridyl group.

As used herein, the term "acylamino" refers to an amino group substituted with an acyl group, and may be represented, for example, as hydrocarbyl-C(O)NH-, preferably alkyl-C(O)NH-.

As used herein, the term "acyloxy" refers to an oxy (O) group substituted with an acyl group, and may be represented, for example, as hydrocarbyl-C(O)O-, preferably alkyl-C(O)O-.

The term "alkoxy," as used herein, refers to a group of the general formula (alkyl-O-) wherein the alkyl group as defined above is attached to the parent compound through an oxygen atom. The alkyl moiety of an alkoxy group may have, for example, 1 to 20 carbon atoms (i.e., $C_1$-$C_{20}$ alkoxy), 1 to 12 carbon atoms (i.e., $C_1$-$C_{12}$ alkoxy), 1 to 10 carbon atoms (i.e., $C_1$-$C_{10}$ alkoxy), or 1 to 6 carbon atoms (i.e., $C_1$-$C_6$ alkoxy). Examples of suitable alkoxy groups include methoxy ($-O-CH_3$ or -OMe), ethoxy ($-OCH_2CH_3$ or -OEt), and t-butoxy ($-OC(CH_3)_3$ or -O-tBu) but are not limited thereto.

The term "alkoxyalkyl," as used herein, refers to an alkyl group substituted with an alkoxy group, and may be represented by the general formula (-alkyl-O-alkyl).

As used herein, the term "alkylamino" refers to an amino group substituted with one or more alkyl groups.

As used herein, the term "alkylthio" refers to a thio (S) group substituted with an alkyl group and may be represented by the general formula (alkyl-S-).

The term "alkenyl," as used herein, is a hydrocarbon, which may be straight-chain or branched, having primary, secondary, tertiary and/or quaternary carbon atoms and one or more unsaturated region, i.e., a carbon-carbon $sp^2$ double bond. For example, an alkenyl group may have 2 to 20 carbon atoms (i.e., $C_2$-$C_{20}$ alkenyl), 2 to 12 carbon atoms (i.e., $C_2$-$C_{12}$ alkenyl), 2 to 10 carbon atoms (i.e., $C_2$-$C_{10}$ alkenyl), or 2 to 6 carbon atoms (i.e., $C_2$-$C_6$ alkenyl). Examples of suitable alkenyl groups include, but are not limited to, ethenyl ($-CH=CH_2$), 2-propenyl ($-CH_2CH=CH_2$), and 5-hexenyl ($-CH_2CH_2CH_2CH_2CH=CH_2$).

The term "alkynyl," as used herein, is a hydrocarbon, which may be straight-chain or branched, having primary, secondary, tertiary and/or quaternary carbon atoms and one or more carbon-carbon sp triple bonds. For example, an alkynyl group may have 2 to 20 carbon atoms (i.e., $C_2$-$C_{20}$ alkynyl), 2 to 12 carbon atoms (i.e., $C_2$-$C_{12}$ alkynyl), 2 to 10 carbon atoms (i.e., $C_2$-$C_{10}$ alkynyl), or 2 to 6 carbon atoms (i.e., $C_2$-$C_6$ alkynyl). Examples of suitable alkynyl groups include, but are not limited to, ethynyl (-C=CH) and 2-propynyl (-CHC=CH).

The term "alkylene," as used herein, refers to a saturated hydrocarbon group, which may be branched or straight-chain, having two valences derived by the removal of two hydrogen atoms from the same carbon atom or two different carbon atoms of a parent alkane. For example, an alkylene group can have 1 to 20 carbon atoms, 1 to 10 carbon atoms, or 1 to 6 carbon atoms. Examples of typical alkylene groups include, but are not limited to, methylene ($-CH_2-$) or ethylene ($-CH_2-CH_2-$).

The term "alkenylene," as used herein, refers to a double-bonded hydrocarbon group, which may be branched or straight chained, having two valences derived by the removal of two hydrogen atoms from the same carbon atom or two different carbon atoms of a parent alkene. For example, an alkenylene group can have 2 to 20 carbon atoms, 2 to 10 carbon atoms, or 2 to 6 carbon atoms. Examples of typical alkenylene groups include, but are not limited to, 1,2-ethenylene (-CH=CH-).

The term "alkynylene," as used herein, refers to a triple-bonded hydrocarbon group, which may be branched or straight-chain, having two valences derived by the removal of two hydrogen atoms from two different carbon atoms of a parent alkyne. For example, an alkynylene group can have 2 to 20 carbon atoms, 2 to 10 carbon atoms, or 2 to 6 carbon atoms. Examples of typical alkynylene groups include, but are not limited to, ethynylene (-C=C-), propynylene ($-CH_2C\equiv C-$), and 4-pentynylene ($-CH_2CH_2CH_2C\equiv C-$).

The term "amide," as used herein, refers to a group

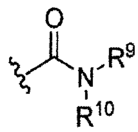

wherein $R^9$ and $R^{10}$ each independently represent a hydrogen or hydrocarbyl group, or $R^9$ and $R^{10}$, together with the N atom to which they are bonded, may form a heterocycle having 4 to 8 atoms in the ring structure.

The terms "amine" and "amino," as used herein, refer to unsubstituted or substituted amines and salts thereof, for example,

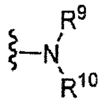 or 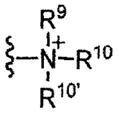 ,

wherein $R^9$, $R^{10}$ and $R^{10'}$ each independently represent a hydrogen or hydrocarbyl group, or $R^9$ and $R^{10}$, together with the N atom to which they are bonded, may form a heterocycle having 4 to 8 atoms in the ring structure). In certain embodiments, amino is $-NH_2$.

As used herein, the term "aminoalkyl" refers to an alkyl group substituted with an amino group.

As used herein, the term "aryl" includes substituted or unsubstituted monovalent or divalent aromatic hydrocarbon groups, which can be monocyclic, bicyclic or polycyclic, wherein each atom of the ring is carbon. Preferably, the aryl ring is a 6- to 20-membered ring, a 6- to 14-membered ring, a 6- to 10-membered ring, or more preferably a 6-membered ring. An aryl group can be a polycyclic ring system having two or more cyclic rings in which two or more carbons are common to two adjacent rings, wherein at least one of the rings can be aromatic, and the other cyclic ring can be, for example, a cycloalkyl, cycloalkenyl, cycloalkynyl, aryl, heteroaryl, and/or heterocycloalkyl. Aryl groups include, but are not limited to, phenyl, naphthyl, phenanthryl, anthracenyl, indenyl, indanyl, anilyl, and the like.

As used herein, the term "aralkyl" or the term "arylalkyl" refers to an alkyl group substituted with an aryl group. Examples of typical arylalkyl groups include, but are not limited to, benzyl, 2-phenylethan-1-yl, naphthylmethyl, 2-naphthylethan-1-yl, naphthobenzyl, 2-naphthophenylethan-1-yl, and the like, each of which may be substituted or unsubstituted). Arylalkyl groups can have from 7 to 20 carbon atoms. For example, its alkyl moiety may have 1 to 6 carbon atoms and its aryl moiety may have 6 to 14 carbon atoms.

The term "arylalkenyl," as used herein, refers to an alkenyl group in which one of the hydrogen atoms bonded to a carbon atom, typically a terminal or other $sp^3$ carbon atom (or may be a $sp^2$ carbon atom), is replaced by an aryl group. The aryl moiety of arylalkenyl can be, for example, any of the aryl group described herein, and the alkenyl moiety of arylalkenyl can include, for example, any of the alkenyl groups described herein. An arylalkenyl group can have from 8 to 20 carbon atoms. For example, its alkenyl moiety may have 2 to 6 carbon atoms and its aryl moiety may have 6 to 14 carbon atoms.

The term "arylalkynyl," as used herein, refers to an alkynyl group in which one of the hydrogen atoms bonded to a carbon atom, typically a terminal or other $sp^3$ carbon atom (or may be a sp carbon atom), is replaced by an aryl group. The aryl moiety of an arylalkynyl can be, for example, any of the aryl groups described herein, and the alkynyl moiety of an arylalkynyl can include, for example, any of the alkynyl groups described herein. An arylalkynyl group can have from 8 to 20 carbon atoms. For example, its alkynyl moiety may have 2 to 6 carbon atoms and its aryl moiety may have 6 to 14 carbon atoms.

The term "carbocycle", "carbocyclyl", "carbocyclic" or "cycloalkyl," as used herein, refers to a non-aromatic saturated or unsaturated, monovalent or a bivalent ring, which can be monocyclic, bicyclic or polycyclic, wherein each atom in the ring is carbon. A cycloalkyl group can have 3 to 7 carbon atoms as a monocycle, 7 to 12 carbon atoms as a bicycle, and up to about 20 carbon atoms as a polycycle. Examples of cycloalkyl include, but are not limited to, cyclopropyl, cyclobutyl, cyclopentyl, cyclohexyl, cycloheptyl, cyclooctyl, and the like.

As used herein, the term "carbocyclylalkyl", or "cycloalkylalkyl", or "(cycloalkyl)alkyl" refers to an alkyl group substituted with a carbocycle group or a cycloalkyl group.

As used herein, the term "carbonate" refers to the formula $-OCO_2-$.

As used herein, the term "carboxy" refers to a group represented by the formula $-CO_2H$.

As used herein, the term "carbamate" refers to a general formula

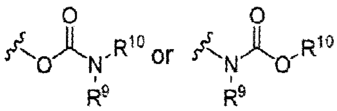

wherein $R^9$ and $R^{10}$ independently represent hydrogen or a hydrocarbyl group.

As used herein, the term "ester" refers to the general formula $-C(O)OR^9$, wherein $R^9$ represents a hydrocarbyl group.

As used herein, the term "ether" refers to a hydrocarbyl group linked to another hydrocarbyl group through an oxygen. Thus, the ether substituent of a hydrocarbyl group may be hydrocarbyl-O-. Ethers may be symmetric or asymmetric. Examples of ethers include, but are not limited to, heterocycle-O-heterocycle and aryl-O-heterocycle. Ethers include "alkoxyalkyl" groups, which may be represented by the general formula alkyl-O-alkyl.

As used herein, the terms "halo" and "halogen" mean halogen and include chloro, fluoro, bromo, and iodo.

The term "haloalkyl," as used herein, is an alkyl group in which at least one of the hydrogen atoms of the alkyl group as defined above has been replaced by a halogen atom. The alkyl moiety of a haloalkyl group may contain from 1 to 20 carbon atoms (i.e., $C_1$-$C_{20}$ haloalkyl), from 1 to 12 carbon atoms (i.e., $C_1$-$C_{12}$ haloalkyl), from 1 to 10 carbon atoms (i.e., $C_1$-$C_{10}$ haloalkyl), or 1 to 6 carbon atoms (i.e., $C_1$-$C_6$ haloalkyl). Exemplary haloalkyl groups include, but are not limited to, $-CF_3$, $-CHF_2$, $-CFH_2$, and $-CH_2CF_3$.

The term "heteroalkyl," as used herein, refers to an alkyl group in which one or more carbon atoms are replaced by a heteroatom such as O, N or S. For example, when a carbon atom of an alkyl group attached to the parent molecule is replaced by a heteroatom (e.g., O, N or S), the resulting heteroalkyl group may be each an alkoxy group (e.g., $-OCH_3$), an amine group (e.g., - $NHCH_3$, $-N(CH_3)_2$, etc.), or a thioalkyl group (e.g., $-SCH_3$). When a non-terminal carbon atom of an alkyl group not attached to the parent molecule is replaced by a heteroatom (e.g., O, N or S), the resulting heteroalkyl group can be each an alkyl ether (e.g., $-CH_2CH_2-O-CH_3$, etc.), an alkylamine (e.g., $-CH_2NHCH_3$, $-CH_2N(CH_3)_2$, etc.), or a thioalkyl ether (e.g., $-CH_2-S-CH_3$). When the terminal carbon atom of the alkyl group is replaced by a heteroatom (e.g., O, N or S), the resulting heteroalkyl group can be each a hydroxyalkyl group (e.g., - $CH_2CH_2-OH$), an aminoalkyl group (e.g., $-CH_2NH_2$), or a thioalkyl group (e.g., $-CH_2CH_2-SH$). For example, a heteroalkyl group can have 1 to 20 carbon atoms, 1 to 10 carbon atoms, or 1 to 6 carbon atoms.

The term "arylheteroalkyl," as used herein, refers to a group in which a carbon atom of a heteroalkyl group or a hydrogen atom attached to a heteroatom is replaced by an aryl group. For example, an arylheteroalkyl group may have the general formula -alkylene-O-aryl, -alkylene-O-alkylene-aryl, -alkylene-NH-aryl, -alkylene-NH-alkylene-aryl, -alkylene-S-aryl, -alkylene-S-alkylene-aryl, and the like.

As used herein, the term "heteroaryl" refers to a substituted or unsubstituted monovalent or divalent aromatic group, which is monocyclic, bicyclic or polycyclic, containing one or more heteroatoms in the ring. Non-limiting examples of suitable heteroatoms that may be contained in the aromatic ring include oxygen, sulfur and nitrogen. In polycyclic heteroaryl ring systems, the ring system has at least two cyclic rings in which at least two carbons are common to two adjacent rings, wherein at least one of the rings is heteroaromatic and the other cyclic rings can be, for example, cycloalkyl, cycloalkenyl, cycloalkynyl, aryl, heteroaryl, and/or heterocyclyl. Heteroaryl groups include, for example, benzofuran, benzothiophene, pyrrole, furan, thiophene, imidazole, indole, isoindole, isoxazole, isothiazole, oxazole, thiazole, quinoline, isoquinoline, pyrazole, pyridine, pyrazine, pyridazine, and pyrimidine radicals and the like.

As used herein, the term "heteroarylalkyl" refers to an alkyl group in which a hydrogen atom is replaced by a heteroaryl group. Non-limiting examples of heteroarylalkyl groups include $-CH_2$-pyridinyl, $-CH_2$-pyrrolyl, $-CH_2$-oxazolyl, $-CH_2$-indolyl, $-CH_2$-isoindolyl, $-CH_2$-furanyl, - $CH_2$-thienyl, $-CH_2$-benzofuranyl, $-CH_2$-benzothiophenyl, $-CH_2$-carbazolyl, $-CH_2$-imidazolyl, - $CH_2$-thiazolyl, $-CH_2$-isoxazolyl, $-CH_2$-pyrazolyl, $-CH_2$-isothiazolyl, $-CH_2$-quinolyl, $-CH_2$-isoquinolyl, $-CH_2$-pyridazyl, $-CH_2$-pyrimidyl, $-CH_2$-pyrazyl, $-CH(CH_3)$-pyridinyl, $-CH(CH_3)$-pyrrolyl, $-CH(CH_3)$-oxazolyl, $-CH(CH_3)$-indolyl, $-CH(CH_3)$-isoindolyl, $-CH(CH_3)$-furanyl, - $CH(CH_3)$-thienyl, $-CH(CH_3)$-benzofuranyl, $-CH(CH_3)$-benzothiophenyl, $-CH(CH_3)$-carbazolyl, - $CH(CH_3)$-imidazolyl, $-CH(CH_3)$-thiazolyl, $-CH(CH_3)$-isoxazolyl, $-CH(CH_3)$-pyrazolyl, - $CH(CH_3)$-isothiazolyl, $-CH(CH_3)$-quinolyl, $-CH(CH_3)$-isoquinolyl, $-CH(CH_3)$-pyridazyl, - $CH(CH_3)$-pyrimidyl, $-CH(CH_3)$-pyrazyl, and the like.

As used herein, the terms "heterocyclyl," "heterocycle," "heterocyclic," and "heterocycloalkyl" refer to a substituted or unsubstituted, monovalent or divalent, saturated or partially saturated non-aromatic ring structure, preferably a 3- to 10-membered ring, more preferably a 3- to 7-membered ring, in which the ring structure contains at least 1 heteroatom, preferably 1 to 4 heteroatoms, more preferably 1 to 2 heteroatom(s). The terms "heterocyclyl," "heterocycle," "heterocyclic," and "heterocycloalkyl" also include polycyclic ring systems having two or more cyclic rings in which two or more carbons are common to two adjacent rings, wherein at least one of the rings is heterocyclic and the other cyclic ring can be, for example, cycloalkyl, cycloalkenyl, cycloalkynyl, aryl, heteroaryl, and/or heterocyclyl. Bicyclic and polycyclic heterocyclic ring systems may be fused, bridged, or spiro ring systems. Heterocyclic groups include, for example, piperidine, piperazine, pyrrolidine, morpholine, lactone, lactam radicals and the like. Further exemplary heterocyclic

groups include, but are not limited to, dihydropyridyl, dihydroindolyl, tetrahydropyridyl (piperidyl), tetrahydrothiophenyl, sulfur-oxidized tetrahydrothiophenyl, indolenyl, piperidinyl, 4- piperidinyl, pyrrolidinyl, 2-pyrrolidonyl, pyrrolinyl, tetrahydrofuranyl, tetrahydroquinolinyl, tetrahydroisoquinolinyl, decahydroquinolinyl, octahydroisoquinolinyl, 6H-1,2,5-thiadiazinyl, 2H,6H-1,5,2-dithiazinyl, pyranyl, chromenyl, xanthenyl, phenoxatinyl, 2H-pyrrolyl, 3H-indolyl, 4H-quinolizinyl, phthalazinyl, naphthyridinyl, quinoxalinyl, quinazolinyl, cinnolinyl, pteridinyl, 4aH-carbazolyl, carbazolyl, β-carbolinyl, phenanthridinyl, acridinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, piperazinyl , quinuclidinyl, morpholinyl, and oxazolidinyl.

As used herein, the term "heterocyclylalkyl" refers to an alkyl group in which one of the hydrogen atoms bonded to a carbon atom, typically a terminal or other $sp^3$ carbon atom, is replaced by a heterocyclyl group.

As used herein, the term "heterocyclylalkenyl" refers to an alkenyl group in which one of the hydrogen atoms bonded to a carbon atom, typically a terminal or other $sp^3$ carbon atom (or may be a $sp^2$ carbon atom), is replaced by a heterocyclyl group (i.e., a heterocyclyl-alkenylene moiety).

As used herein, the term "heterocyclylalkynyl" refers to alkynyl group in which one of the hydrogen atoms bonded to a carbon atom, typically a terminal or other $sp^3$ carbon atom (or may be a sp carbon atom), is replaced by a heterocyclyl group (i.e., a heterocyclyl-alkynylene moiety).

As used herein, the term "hydroxyalkyl" refers to an alkyl group substituted with a hydroxy group.

The term "lower" as used herein, when used in conjunction with chemical moieties such as acyl, acyloxy, alkyl, alkenyl, alkynyl or alkoxy, is considered to contain a group having up to 10 atoms, preferably up to 6 atoms, in the substituent. "Lower alkyl" refers to an alkyl group containing, for example, up to 10, preferably up to 6 carbon atoms.

As used herein, the terms "polycyclyl," "polycycle," and "polycyclic" refer to two or more rings (e.g., cycloalkyl, cycloalkenyl, cycloalkynyl, aryl, heteroaryl, and/or heterocyclyl), in which two or more atoms are common to two adjacent rings, e.g., the rings are "fused rings." In certain embodiments, each ring of the polycycle contains 3 to 10 atoms, preferably 5 to 7 atoms in the ring.

As used herein, the term "sulfate" refers to the formula $-OSO_3H$, or a pharmaceutically acceptable salt thereof.

As used herein, the term "sulfonamide" refers to a group represented by the general formula

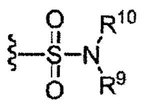 or 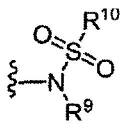 ,

wherein $R^9$ and $R^{10}$ independently represent hydrogen or a hydrocarbyl group.

As used herein, the term "sulfoxide" refers to the formula -S(O)-.

As used herein, the term "sulfonate" refers to the formula $-SO_3H$, or a pharmaceutically acceptable salt thereof.

As used herein, the term "sulfone" refers to the formula $-S(O)_2-$.

As used herein, the term "silyloxy" refers to the general formula $-O-SiR_3$, wherein each R is independently an alkyl, aryl, or heteroaryl group. Non-limiting examples of silyloxy include $Si(CH_3)_3$, $-O-Si(CH_3)_2tBu$, $-O-Si(tBu)_2CH_3$, $-O-Si(tBu)_3$, $-O-Si(CH_3)_2Ph$, $-O-Si(Ph)_2CH_3$, and $-O-Si(Ph)_3$.

As used herein, the term "thioalkyl" refers to an alkyl group substituted with a thiol group.

As used herein, the term "thioester" refers to the general formula $-C(O)SR^9$ or $-SC(O)R^9$, wherein $R^9$ represents a hydrocarbyl group.

As used herein, the term "thioether" corresponds to an ether in which the oxygen has been replaced by sulfur.

The term "urea" may be represented by the general formula and $R^{10}$ independently represent hydrogen or a hydrocarbyl group.

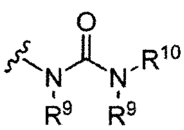 ,

wherein $R^9$

The term "ester thereof" refers to any ester of a compound in which any -COOH functional group of a molecule is modified to a -COOR functional group, or any -OH functional group of a molecule is modified to -OC(=O)R. In the above, the R moiety of the ester can be any carbon-containing group that forms a stable ester moiety, which includes, but is not limited to, alkyl, alkenyl, alkynyl, cycloalkyl, cycloalkylalkyl, aryl, arylalkyl, heterocyclyl, heterocyclylalkyl, and substituted derivatives thereof.

All groups defined hereinabove may be substituted in one or more positions with one or more groups as defined herein unless otherwise stated.

The term "substituted" for alkyl, alkylene, aryl, arylalkyl, heterocyclyl, etc., for example "substituted alkyl," "substituted alkylene," "substituted aryl," "substituted arylalkyl," "substituted heterocyclyl," and "substituted carbocyclyl (e.g., substituted cycloalkyl)" refer to alkyl, alkylene, aryl, arylalkyl, heterocyclyl, or carbocyclyl (e.g., cycloalkyl), in which at least one hydrogen atoms are each independently replaced by a non-hydrogen substituent. A substituent may include any of the substituents described herein, for example, halogen, hydroxyl, alkyl, hydroxyalkyl, haloalkyl, cyanoalkyl, alkoxyalkyl, carbonyl (e.g., carboxyl, alkoxycarbonyl, formyl, or acyl), thiocarbonyl (e.g., thioester, thioacetate, or thioformate), alkoxyl, phosphoryl, phosphate, phosphonate, phosphinate, amino, amido, amidin, imine, cyano, nitro , azido, sulfhydryl, alkylthio, sulfate, sulfonate, sulfamoyl, sulfonamido, sulfonyl, heterocyclyl, aralkyl, or aromatic or heteroaromatic moieties. It will be understood by a person skilled in the art that a substituted moiety on the hydrocarbon chain may itself be optionally substituted.

In certain embodiments, the compounds of the present invention may be racemates. In certain embodiments, the compounds of the present invention may be enriched for one enantiomer. For example, a compound of the present invention may have an enantiomeric excess (ee) of at least 30%, 40%, 50%, 60%, 70%, 80%, 90%, or even 95% or more.

Certain compounds useful in the methods and compositions of the present disclosure may have one or more stereocenters within their structure. Such stereocenters may exist in either R or S configuration, and the R and S designations are used in accordance with the rules described in the document [Pure Appl. Chem. (1976), 45, 11-30]. This disclosure contemplates a compound, salt, prodrug thereof, or all stereoisomeric forms, such as enantiomeric and diastereomeric forms, including all possible mixtures of stereoisomers.

Certain compounds of the present invention have more than one stereocenter. Accordingly, the compounds of the present invention may be enriched for one or more diastereomers. For example, a compound of the present invention may have a diastereomeric excess (de) of at least 30%, 40%, 50%, 60%, 70%, 80%, 90%, or even 95% or greater. In certain embodiments, the compounds of the present invention have substantially one isomeric configuration at one or more stereocenters and multiple isomeric configurations at the remaining stereo centers.

As used herein, a single bond depicted without stereochemistry does not represent the stereochemistry of the compound. Compounds of formula I provide examples of compounds for which stereochemistry is not indicated.

Moreover, certain compounds containing an alkenyl group may exist as Z (zusammen) or E (entgegen) isomers. In each case, the present disclosure includes both mixtures and separate individual isomers.

Some of the compounds may also exist in tautomeric forms. Such forms, although not expressly indicated in the formulas described herein, are intended to be included within the scope of the present disclosure.

The phrase "pharmaceutically acceptable" is well accepted in the art. In certain embodiments, the term includes compositions, excipients, adjuvants, polymers and other substances and/or dosage forms, which are suitable for use in contact with tissues of humans and animals without undue toxicity, irritation, allergic reaction, or other problems or complications, commensurate with a reasonable benefit/harm ratio within the scope of sensible medical judgment.

"Pharmaceutically acceptable salt" or "salt" is used herein to refer to an acid addition salt or a base addition salt suitable or compatible with the treatment of a patient. Exemplary inorganic acids that form suitable salts include hydrochloric acid, hydrobromic acid, sulfuric acid and phosphoric acid, as well as metal salts such as sodium monohydrogen orthophosphate and potassium hydrogen sulfate. Exemplary organic acids that form suitable salts include mono-, di- and tricarboxylic acids such as glycolic acid, lactic acid, pyruvic acid, malonic acid, succinic acid, glutaric acid, fumaric acid, malic acid, tartaric acid, citric acid, ascorbic acid, maleic acid, benzoic acid, phenylacetic acid, cinnamic acid and salicylic acid, as well as sulfonic acids such as p-toluene sulfonic acid and methanesulfonic acid. Monoacid or diacid salts may be formed, and such salts may exist in hydrated, solvated or substantially anhydrous form. In general, acid addition salts of compounds of the present invention are more soluble in water and various hydrophilic organic solvents and generally exhibit higher melting points compared to their free base forms. The selection of an appropriate salt is known to a person skilled in the art. Other non-pharmaceutically acceptable salts, such as oxalates, can be used in the isolation of compounds of the present invention, for example, for laboratory use or for subsequent conversion to pharmaceutically acceptable acid addition salts. Exemplary inorganic bases that form suitable salts include lithium, sodium, potassium, calcium, magnesium, or barium hydroxide. Exemplary organic bases that form suitable salts include aliphatic, cycloaliphatic or aromatic organic amines such as methylamine, trimethylamine and picoline or ammonia. Thus, in some instances, contemplated salts of the present invention include alkyl, dialkyl, trialkyl or tetra-alkyl ammonium salts. In certain embodiments, contemplated salts of the present invention include, but are not limited to, L-arginine, benentamine, benzathine, betaine, calcium hydroxide, choline, deanol, diethanolamine, diethylamine, 2-(diethylamino)ethanol, ethanolamine, ethylenediamine, N-methylglucamine, hydrabamine, 1H-imidazole, lithium, L-lysine, magnesium, 4-(2-hydroxyethyl)morpholine, piperazine, potassium, 1-(2-hydroxyethyl)pyrrolidine, sodium, triethanolamine, tromethamine, and zinc salts. In certain embodiments, contemplated salts of the present invention include, but are not limited to, Na, Ca, K, Mg, Zn or other metal salts.

The selection of an appropriate salt is known to a person skilled in the art.

Pharmaceutically acceptable acid addition salts may also exist in various solvates, such as solvates with water, methanol, ethanol, dimethylformamide, and the like. Mixtures of such solvates may also be prepared. The source of

these solvates may be from, or incidentally included in, the solvent of preparation or crystallization.

A "prodrug" or "pharmaceutically acceptable prodrug" refers to a compound that, after administration, is metabolized in a host, for example, hydrolyzed or oxidized to form a compound of the present invention. Typical examples of prodrugs include compounds having a biologically labile or cleavable protecting group on the functional moiety of the active compound. Prodrugs include compounds that may be oxidized, reduced, aminationed, deaminationed, hydroxylated, dehydroxylated, hydrolysed, dehydrolyzed, alkylated, dealkylated, acylated, deacylated, phosphorylated or dephosphorylated to yield the active compounds. Examples of prodrugs that use esters or phosphoramidates as biologically labile or cleavable protecting groups are disclosed in US Pat. Nos. 6,875,751, 7,585,851, and 7,964,580, the disclosures of which are incorporated herein by reference. The prodrugs of the present disclosure are metabolized to produce the compounds of the present invention. The present disclosure includes within its scope prodrugs of the compounds described herein. Existing methods for the selection and preparation of suitable prodrugs are described, for example, in ["Design of Prodrugs" Ed. H. Bundgaard, Elsevier, 1985].

In certain embodiments, a therapeutic agent of a compound of the present invention may be enriched to provide predominantly one enantiomer of the compound. An enantiomerically enriched mixture may comprise, for example, at least 60 mol %, or more preferably at least 75, 90, 95, or even 99 mol % of one enantiomer. In certain embodiments, a compound enriched for one enantiomer is substantially free of the other enantiomer, wherein "substantially free" means that the material is, for example, less than 10%, or less than 5%, or less than 4%, or less than 3%, or less than 2%, or less than 1% relative to the amount of the other enantiomer in the composition or mixture of compounds. For example, if a composition or mixture of compounds contains 98 moles of the first enantiomer and 2 moles of the second enantiomer, it contains 98 mole% of the first enantiomer and only 2 mole% of the second enantiomer.

**Novel TNF activity inhibitor compounds**

In the following, representative embodiments of the compounds of the present invention are specifically disclosed. The compounds of the present invention are suitable for formulation as any pharmaceutical composition, or for use in any method disclosed herein, such as a prophylactic or therapeutic method.

Unless stated otherwise herein, a compound of the above formula as an active ingredient of a therapeutic agent includes any pharmaceutically acceptable salts thereof, all of which are to be considered included within the scope of the present invention. For convenience of explanation, it may be simply abbreviated as a compound of the above formula or a compound of the present invention.

In one embodiment, the present invention provides a compound of Formula I below or a pharmaceutically acceptable salt thereof.

[Formula I]

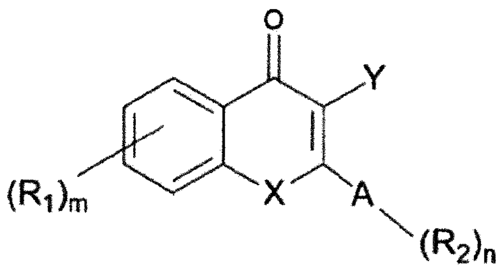

wherein,

X is $-C(R_3)_2-$, $-N(R_3)-$, -O- or -S-,

Y is $-OR_a$, $-SR_a$, $-C(O)R_a$, $-C(O)OR_a$, $-C(O)N(R_a)(R_b)$, $-N(R_a)(R_b)$, $-N(R_a)C(O)R_b$, $-N(R_a)C(O)OR_b$, or $-N(R_a)S(O)OR_b$,

A is $C_3$-$C_8$ cycloalkyl; 4- to 10-membered heterocycloalkyl containing 1 to 4 heteroatoms independently selected from the group consisting of N, O and S; $C_6$-$C_{10}$ aryl; or 5- to 10-membered heteroaryl containing 1 to 4 heteroatoms independently selected from the group consisting of N, O and S,

$R_1$, $R_2$ and $R_3$ may be the same or different and are each independently H, halo, cyano, - ORc, nitro, $C_1$-$C_6$ alkyl, $C_2$-$C_6$ alkenyl, $C_2$-$C_6$ alkynyl, $-SR_c$, $-C(O)R_c$, $-C(O)OR_c$, $-C(O)N(R_c)(R_d)$, $-N(R_c)(R_d)$, $-N(R_c)C(O)R_d$, $-N(R_c)C(O)OR_d$, or $-N(R_c)S(O)OR_d$,

$R_a$, $R_b$, $R_c$ and $R_d$ may be the same or different and are each independently H or $C_1$-$C_6$ alkyl, and

m and n are each independently an integer from 0 to 4.

In a preferred embodiment, X is - $N(R_3)$- or -O-, and $R_3$ may be H or $C_1$-$C_6$ alkyl.

In a preferred embodiment, Y may be -$OR_a$, -C(O)$R_a$, -C(O)O$R_a$, or -C(O)N($R_a$)($R_b$).

In a preferred embodiment, A may be phenyl.

In another preferred embodiment, $R_1$ and $R_2$ can be the same or different and each independently can be H, halo or -$OR_c$.

In one embodiment, the present invention provides novel chromenone or quinolone derivative compounds. In a representative embodiment, the derivative compound may be a compound of Formula II below or a pharmaceutically acceptable salt thereof.

[Formula II]

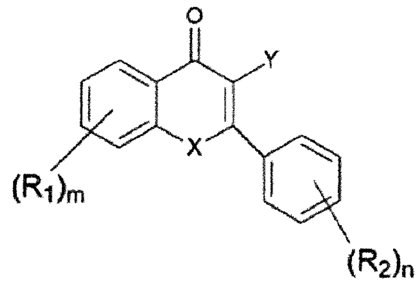

wherein,

X is -N($R_3$)- or -O-,
Y is -$OR_a$, -C(O)$R_a$, -C(O)O$R_a$, or -C(O)N($R_a$)($R_b$),
$R_1$ and $R_2$ can be the same or different, and are each independently H, halo, or -$OR_c$,
$R_3$ is H or $C_1$-$C_6$ alkyl,
$R_a$, $R_b$ and $R_c$ can be the same or different, and are each independently H or $C_1$-$C_6$ alkyl, and
m and n are each independently an integer 1 or 2.

The compounds of Formulas I to II include, but are not limited to, a compound selected from the group consisting of the following compounds (1) to (47) or a pharmaceutically acceptable salt thereof.

(1) 2-(2,4-difluorophenyl)-3-carboxy-7-chloro-4-(1*H*)-quinolone;
(2) 2-(2,5-difluorophenyl)-3-carboxy-7-chloro-4-(1*H*)-quinolone;
(3) 2-(3,4-difluorophenyl)-3-carboxy-7-chloro-4-(1*H*)-quinolone;
(4) 2-(3,5-difluorophenyl)-3-carboxy-7-chloro-4-(1*H*)-quinolone;
(5) 2-(2,6-difluorophenyl)-3-carboxy-7-chloro-4-(1*H*)-quinolone;
(6) 2-(2,5-difluorophenyl)-3-carboxy-6-chloro-4-(1*H*)-quinolone;
(7) 2-(3,4-difluorophenyl)-3-carboxy-6-chloro-4-(1*H*)-quinolone;
(8) 2-(3,5-difluorophenyl)-3-carboxy-6-chloro-4-(1*H*)-quinolone;
(9) 2-(2,6-difluorophenyl)-3-carboxy-6-chloro-4-(1*H*)-quinolone;
(10) 2-(2,5-difluorophenyl)-3-carboxy-8-chloro-4-(1*H*)-quinolone;
(11) 2-(2,5-difluorophenyl)-3-carboxy-7-bromo-4-(1*H*)-quinolone;
(12) 2-(3,4-difluorophenyl)-3 -carboxy-7-bromo-4-(1*H*)-quinolone;
(13) 2-(3,5-difluorophenyl)-3-carboxy-7 -bromo-4-(1*H*)-quinolone;
(14) 2-(2,6-difluorophenyl)-3-carboxy-7-bromo-4-(1*H*)-quinolone;
(15) 2-(2,5-difluorophenyl)-3-carboxy-7-methoxy-4-(1*H*)-quinolone;
(16) 2-(3,5-difluorophenyl)-3-carboxy-7-methoxy-4-(1*H*)-quinolone;
(17) 2-(2,6-difluorophenyl)-3-carboxy-7-methoxy-4-(1*H*)-quinolone;
(18) 2-(2,5-difluorophenyl)-3-carboxy-7-fluoro-4-(1*H*)-quinolone;
(19) 1-methyl-2-(2,4-difluorophenyl)-3-carboxy-7-chloro-4-(1*H*)-quinolone;
(20) 1-methyl-2-(2,5-difluorophenyl)-3-carboxy-7-chloro-4-(1*H*)-quinolone;
(21) 1-methyl-2-(3,4-difluorophenyl)-3-carboxy-7-chloro-4-(1*H*)-quinolone;
(22) 1-methyl-2-(3,5-difluorophenyl)-3-carboxy-7-chloro-4-(1*H*)-quinolone;
(23) 1-methyl-2-(2,6-difluorophenyl)-3-carboxy-7-chloro-4-(1*H*)-quinolone;
(24) 1-methyl-2-(2,5-difluorophenyl)-3-carboxy-6-chloro-4-(1*H*)-quinolone;
(25) 1-methyl-2-(3,4-difluorophenyl)-3-carboxy-6-chloro-4-(1*H*)-quinolone;

(26) 1 -methyl-2-(3,5-difluorophenyl)-3-carboxy-6-chloro-4-(1*H*)-quinolone;
(27) 1-methyl-2-(2,6-difluorophenyl)-3-carboxy-6-chloro-4-(1*H*)-quinolone;
(28) 1-methyl-2-(2,5-difluorophenyl)-3-carboxy-8-chloro-4-(1*H*)-quinolone;
(29) 1 -methyl-2-(2,5-difluorophenyl)-3-carboxy-7-bromo-4-(1*H*)-quinolone;
(30) 1-methyl-2-(3,5-difluorophenyl)-3-carboxy-7-bromo-4-(1*H*)-quinolone;
(31) 1-methyl-2-(2,6-difluorophenyl)-3-carboxy-7-bromo-4-(1*H*)-quinolone;
(32) 7-chloro-2-(2,5-difluorophenyl)-3-hydroxy-4*H*-chromen-4-one;
(33) 7-chloro-2-(3,5-difluorophenyl)-3-hydroxy-4*H*-chromen-4-one;
(34) 7-chloro-2-(3,4-difluorophenyl)-3-hydroxy-4*H*-chromen-4-one;
(35) 2-(2,5-difluorophenyl)-7-fluoro-3-hydroxy-4*H*-chromen-4-one;
(36) 2-(3,5-difluorophenyl)-7-fluoro-3-hydroxy-4*H*-chromen-4-one;
(37) 2-(2,3-difluorophenyl)-7-fluoro-3-hydroxy-4*H*-chromen-4-one;
(38) 2-(2,5-difluorophenyl)-3-hydroxy-7-methoxy-4*H*-chromen-4-one;
(39) 2-(3,5-difluorophenyl)-3-hydroxy-7-methoxy-4*H*-chromen-4-one;
(40) 7-chloro-2-(2,5-difluorophenyl)-3-methoxy-4*H*-chromen-4-one;
(41) 7-chloro-2-(3,5-difluorophenyl)-3-methoxy-4*H*-chromen-4-one;
(42) 2-(2,5-difluorophenyl)-7-fluoro-3-methoxy-4*H*-chromen-4-one;
(43) 2-(2,4-difluorophenyl)-7-fluoro-3-methoxy-4*H*-chromen-4-one;
(44) 2-(3,5-difluorophenyl)-7-fluoro-3-methoxy-4*H*-chromen-4-one;
(45) 2-(2,5-difluorophenyl)-3,7-dimethoxy-4*H*-chromen-4-one;
(46) 2-(3,5-difluorophenyl)-3,7-dimethoxy-4*H*-chromen-4-one; and
(47) 2-(2,4-difluorophenyl)-3,7-dimethoxy-4*H*-chromen-4-one.

In one embodiment, the present invention provides a dimer compound of the compound of Formula I or II. In a representative embodiment, the dimer may be a compound of Formula III below or a pharmaceutically acceptable salt thereof.

[Formula III] $M_1$-L-$M_2$

wherein,

$M_1$ and $M_2$ may be the same or different, and are each independently a monomer radical of Formula Ia below, and L is a linking group covalently bonding $M_1$ and $M_2$, and is selected from the following Formulas IVa to IVc.

[Formula Ia]

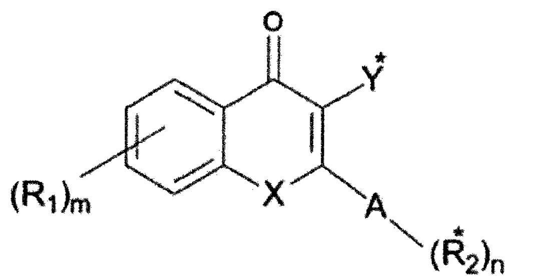

(wherein,

X is -C($R_3$)$_2$-, -N($R_3$)-, -O- or -S-,
Y is -O$R_a$, -S$R_a$, -C(O)$R_a$, -C(O)O$R_a$, -C(O)N($R_a$)($R_b$), -N($R_a$)($R_b$), -N($R_a$)C(O)$R_b$, - N($R_a$)C(O)O$R_b$, or -N($R_a$)S(O)O$R_b$,
A is $C_3$-$C_8$ cycloalkyl; 4- to 10-membered heterocycloalkyl containing 1 to 4 heteroatoms independently selected from the group consisting of N, O and S; $C_6$-$C_{10}$ aryl; or 5- to 10-membered heteroaryl containing 1 to 4 heteroatoms independently selected from the group consisting of N, O and S,
$R_1$, $R_2$ and $R_3$ may be the same or different and are each independently H, halo, cyano, - O$R_c$, nitro, $C_1$-$C_6$ alkyl, $C_2$-$C_6$ alkenyl, $C_2$-$C_6$ alkynyl, -S$R_c$, -C(O)$R_c$, -C(O)O$R_c$, -C(O)N($R_c$)($R_d$), -N($R_c$)($R_d$), -N($R_c$)C(O)$R_d$, -N($R_c$)C(O)O$R_d$, or -N($R_c$)S(O)O$R_d$,
$R_a$, $R_b$, $R_c$ and $R_d$ may be the same or different and are each independently H or $C_1$-$C_6$ alkyl,

m and n are each independently an integer from 0 to 4, the monomer unit $M_1$ or $M_2$ is bonded to the linking group L through a bonding site * located in either Y or $R_2$, and when Y or $R_2$ has the above bonding site *, Y or $R_2$ contains N, O or S atoms, and -C(O)-, -NH-, -N($C_1$-$C_6$ alkyl)- , -O- or -S- radicals are located at the bonding site *).

[Formula IVa] *R-Z-R'*

[Formula IVb] $*-[(CH_2)_2-O]_p-(CH_2)_2-*$

[Formula IVc]

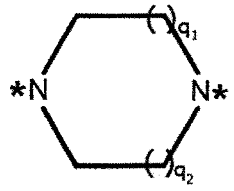

(wherein,

* represents a bonding site between the monomer unit $M_1$ or $M_2$ and the linking group L,
R or R' is each independently $C_1$-$C_{10}$ alkylene, $C_2$-$C_{10}$ alkenylene, or $C_2$-$C_{10}$ alkynylene;
Z is a single bond; -O-; -S-; -NH-; -N($C_1$-$C_6$ alkyl)-; $C_3$-$C_8$ cycloalkylene; 4- to 10-membered heterocycloalkyl containing 1 to 4 heteroatoms independently selected from the group consisting of N, O and S; $C_6$-$C_{10}$ aryl; or 5- to 10-membered heteroaryl containing 1 to 4 heteroatoms independently selected from the group consisting of N, O and S;
p is an integer from 1 to 5, and
$q_1$ and $q_2$ are each independently an integer of 0 to 5, but $q_1$ and $q_2$ are not 0 at the same time).

Linker L is also referred to herein as "Linker".

In a preferred embodiment, $M_1$ and $M_2$ may be the same or different, and each independently may be a chromenone or quinolone derivative, more preferably, a monomer radical of the following Formula IIa.

[Formula IIa]

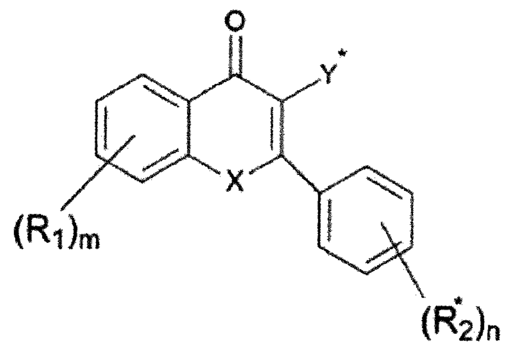

wherein,

X is N-$R_3$ or O,
Y is -$OR_a$, -C(O)$OR_a$, or -C(O)N($R_a$)($R_b$),
$R_1$ and $R_2$ can be the same or different, and are each independently H, halo, or -$OR_c$,
$R_3$ is H or $C_1$-$C_6$ alkyl,
$R_a$, $R_b$ and $R_c$ can be the same or different, and are each independently H or $C_1$-$C_6$ alkyl,

m and n are each independently an integer 1 or 2.

In a preferred embodiment, the linking group L may be a $C_2$-$C_4$ alkylene group, $-(CH_2)_2-O-(CH_2)_2-$, or

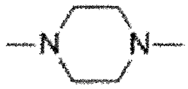 .

The compound of Formula III includes, but is not limited to, a compound selected from the group consisting of the following compounds (48) to (55) or a pharmaceutically acceptable salt thereof.

(48) 2,2'-((ethane-1,2-diylbis(oxy))bis(2-fluoro-4,1-phenylene))bis(7-chloro-4-oxo-4H-chloromene-3-carboxylic acid);
(49) 2,2'-((propane-1,3-diylbis(oxy))bis(2-fluoro-4,1-phenylene))bis(7-chloro-4-oxo-4H-chloromene-3-carboxylic acid);
(50) 2,2'-((butane-1,4-diylbis(oxy))bis(2-fluoro-4,1-phenylene))bis(7-chloro-4-oxo-4H-chloromene-3-carboxylic acid);
(51) 2,2'-(((oxybis(ethane-2,1-diyl))bis(oxy))bis(2-fluoro-4,1-phenylene))bis(7-chloro-4 -oxo-4H-chromene-3-carboxylic acid);
(52) N,N'-(ethane-1,2-diyl)bis(7-chloro-2-(2,5-difluorophenyl)-4-oxo-4H-chromene-3-carboxamide);
(53) N,N'-(propane-1,3-diyl)bis(7-chloro-2-(2,5-difluorophenyl)-4-oxo-4H-chromene-3-carboxamide);
(54) N,N'-(butane-1,4-diyl)bis(7-chloro-2-(2,5-difluorophenyl)-4-oxo-4H-chromene-3-carboxamide); and
(55) 3,3'-(piperazine-1,4-dicarbonyl)bis(7-chloro-2-(2,5-difluorophenyl)-4H-chromene-4-one).

The compounds of the present invention may exist in the form of salts, particularly pharmaceutically acceptable salts. As the salt, salts generally used in the art, such as acid addition salts formed with pharmaceutically acceptable free acids, can be used without limitation. As used herein, the term "pharmaceutically acceptable salt" refers to any organic or inorganic addition salt of a compound of the present invention, wherein the side effects induced by the salt are relatively non-toxic to the patient and do not compromise the beneficial effects of the compound at a concentration exhibiting innocuous effective activity.

Organic and inorganic acids can be used as free acids. Pharmaceutically acceptable salts include inorganic acids such as hydrochloric acid, sulfuric acid, nitric acid, phosphoric acid, hydrobromic acid, hydroiodic acid and the like; organic carbonic acids such as tartaric acid, formic acid, citric acid, acetic acid, trichloroacetic acid, trifluoroacetic acid, gluconic acid, benzoic acid, lactic acid, mandelic acid, fumaric acid, maleic acid, salicylic acid and the like; or acid addition salts formed with sulfonic acids such as methanesulfonic acid, ethanesulfonic acid, benzenesulfonic acid, p-toluenesulfonic acid and the like. Examples of pharmaceutically acceptable salts of carboxylic acids include metal salts or alkaline earth metal salts formed with lithium, sodium, potassium, calcium, magnesium and the like, amino acid salts such as lysine, arginine, guanidine and the like, organic salts such as dicyclohexylamine, N-methyl-D-glucamine, tris(hydroxymethyl)methylamine, diethanolamine, choline, triethylamine, etc. The compounds according to the present invention can also be converted into their salts by conventional methods.

In addition, the compounds of the present invention include, without limitation, pharmaceutically acceptable salts thereof, as well as all possible optical isomers. Stereoisomers of the compounds of the present invention can be prepared using methods known in the art.

In addition, the compounds of the present invention may be prepared in crystalline or amorphous form. When the compound is prepared in crystalline form, it may optionally be hydrated or solvated.

Other terms have the same meanings as commonly understood in the art to which this invention pertains.

## Therapeutic use

The compound according to the present invention or a pharmaceutically acceptable salt thereof may inhibit the activity of TNF by directly binding to TNF and inhibiting cell signal transduction by TNF. Accordingly, the compound according to the present invention exhibits an effect of preventing or treating a TNF-related disease.

In one embodiment, the present invention relates to a pharmaceutical composition for preventing or treating a TNF-related disease, comprising a compound of the present invention, or a pharmaceutically acceptable salt thereof, as an active ingredient.

In another embodiment, the present invention relates to a method for preventing or treating a TNF-related disease, comprising administering a compound of the present invention, or a pharmaceutically acceptable salt thereof.

In another embodiment, the present invention relates to the use of a compound of the present invention, or a

pharmaceutically acceptable salt thereof, for the prevention or treatment of a TNF-related disease.

Specifically, the TNF-related disease may be any one selected from the group consisting of autoimmune diseases, inflammatory diseases, cardiovascular diseases, metabolic diseases, immune disorders, neurological diseases, ophthalmic diseases, skin diseases, mental diseases, infectious diseases and cancer, but is not limited thereto.

More specifically, the TNF-related disease may be any one selected from the group consisting of rheumatoid arthritis, juvenile rheumatoid arthritis, inflammatory bowel disease, Crohn's disease, ulcerative colitis, psoriasis, plaque psoriasis, pediatric plaque psoriasis, psoriatic arthritis, polyarticular juvenile idiopathic arthritis, Behcet's enteritis, ankylosing spondylitis, axial spondylarthritis, pediatric osteochondritis-related arthritis, polymyalgia rheumatica, multiple sclerosis, thyroiditis, delayed hypersensitivity, allergy, contact dermatitis, atopic dermatitis, systemic lupus erythematosus, systemic sclerosis, adult-onset Still's disease, asthma, autoimmune thyroid disorder, Sjogren's syndrome, Kawasaki disease, pancreatitis, nephritis, hepatitis, pneumonia, chronic obstructive pulmonary disease, otitis media, angioproliferative nephritis, myelodysplastic syndrome, osteoarthritis, sarcoidosis, granuloma annulus, Wegener's granulomatosis, lupus, hemolytic uremic syndrome, arteriosclerosis, vasculitis, heart failure, stroke, myocardial infarction, myocardial ischemia-reperfusion injury, sexual dysfunction, obesity, hypertension, diabetes and diabetic complications, hyperlipidemia, preeclampsia, kidney disease, liver disease, kidney damage, liver damage, snake bite, allograft rejection, organ transplantation, graft-versus-host disease, dementia, Alzheimer's disease, Parkinson's disease, amyotrophic lateral sclerosis, pain, central nervous system disease, uveitis, Behcet's disease, diabetic macular edema, macular degeneration, orbitopathy, glaucoma, hidradenitis suppurativa, multicentral reticulocytosis, pityriasis rubra pilaris, eosinophilic fasciitis, panniculitis, necrobiosis lipoidica diabeticorum, cicatricial pemphigoid, pyoderma gangrenosum, Sweet's syndrome, subcorneal pustular dermatosis, scleroderma, neutrophilic dermatosis, toxic epidermal necrolysis, pustular dermatosis, dermatomyositis, polymyositis, bullous dermatosis, erythema nodosum, alopecia, depression, bipolar disorder, anxiety disorder, tuberculosis, viral infection, bacterial infection, fungal infection, protozoan infection, cerebral malaria, sepsis, septic shock, prostate cancer, skin cancer, colorectal cancer, kidney cancer, pancreatic cancer, ovarian cancer, breast cancer, bladder cancer, prostate cancer, lymphoma, glioma, osteosarcoma, leukemia, multiple myeloma and cachexia, but the present invention is not limited thereto.

According to the results of *in vitro* and *in vivo* experiments, the novel compound of the present invention inhibits apoptosis induced by TNF, and exhibits an $IC_{50}$ value of about 0.1 to 50 μM. In addition, the compound of the present invention directly binds to TNF with a higher binding affinity than TNFR1 or TNFR2, and inhibits the binding of TNF-TNFR1 or TNF-TNFR2 (in each case, exhibits an IC50 value of about 1 to 100 μM). Furthermore, it inhibits cell signal transduction by TNF. Accordingly, the compounds of the present invention exhibit high selective inhibitory activity against TNF, and are useful for preventing, improving or treating TNF-related diseases.

The present invention also provides a com health functional food position for preventing or improving TNF-related diseases, comprising the compound of the present invention or a pharmaceutically acceptable salt thereof as an active ingredient. In another embodiment, the present invention provides a reagent composition for inhibiting the activity of TNF *in vitro* comprising a compound of the present invention or a pharmaceutically acceptable salt thereof as an active ingredient.

## General Synthesis Methods

The compounds according to the invention can be prepared from readily available starting materials using modifications to the specific synthetic protocols described below well known to those skilled in the art.

In one embodiment, the quinolone derivative compound of the present invention may be prepared according to Scheme 1 below.

[Scheme 1]

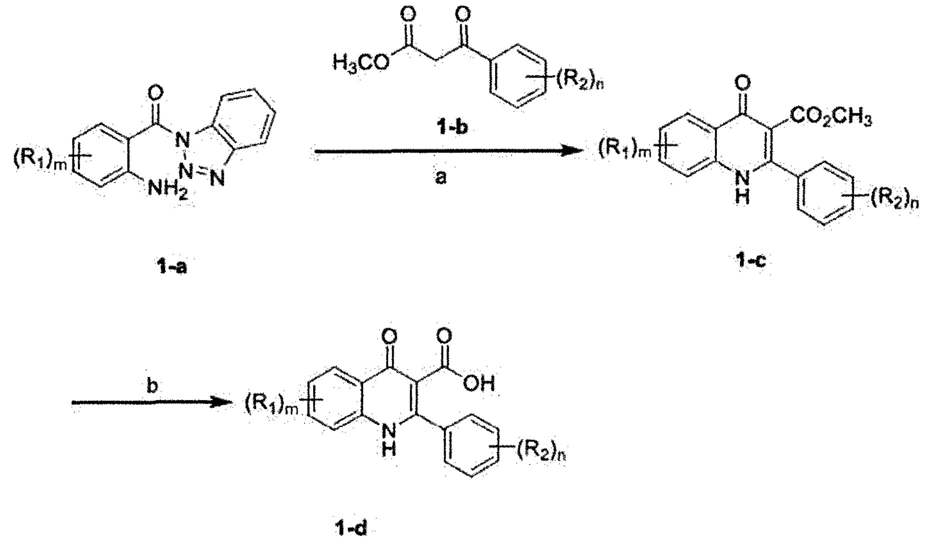


1-a 1-b 1-c 1-d

wherein the substituents $R_1$ and $R_2$, and the integers m and n are as defined above.

Anthranilic acid benzotriazole derivative 1-a and benzoyl acetate derivative 1-b are dissolved in an appropriate solvent and stirred under a nitrogen atmosphere. t-BuOK, t-BuONa, or MeONa is added dropwise, and then heated to reflux. Upon the completion of the reaction, the solvent is removed by distillation under reduced pressure, and then extracted using an appropriate extraction solvent. The organic layer is dried, filtered, and distilled under reduced pressure. Impurities are removed to obtain intermediate compound 1-c.

Intermediate compound 1-c is subjected to ester hydrolysis using a basic aqueous solution to obtain the final compound 1-d.

In another embodiment, the quinolone derivative compound of the present invention may be prepared according to Scheme 2 below.

[Scheme 2]

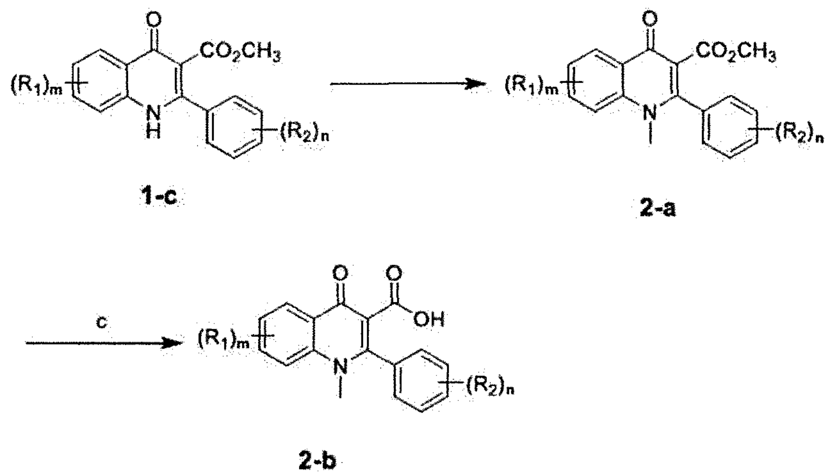


1-c 2-a 2-b

wherein the substituents $R_1$ and $R_2$, and the integers m and n are as defined above.

After dissolving compound 1-c in a solvent, NaH is added and stirred under appropriate reaction conditions. Compound 1-c may be prepared, for example, through Scheme 1. After the dropwise addition of iodomethane, the mixture was stirred under appropriate reaction conditions, and the solvent was removed when the reaction is completed. Then, after extraction with an extraction solvent, the organic layer is dried and filtered to remove the solvent. Intermediate compound 2-a is obtained through the final purification process.

After dissolving compound 2-a in a basic aqueous solution, it is stirred under appropriate reaction conditions

to undergo ester hydrolysis. Upon completion of the reaction, the solvent is removed, the pH is adjusted by adding an acidic aqueous solution dropwise, and the resulting solid is filtered to obtain the final compound 2-b.

In one embodiment, the chromenone derivative compound of the present invention may be prepared according to Scheme 3 below.

[Scheme 3]

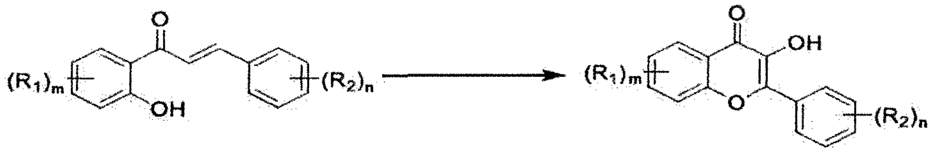

3-a 3-b

wherein the substituents $R_1$ and $R_2$, and the integers m and n are as defined above.

After dissolving chalcone compound 3-a in a solvent, an additional solvent is added at an appropriate temperature and stirred. Upon completion of the reaction, the acidic aqueous solution is slowly added dropwise to the reaction mixture to adjust the pH, followed by further stirring. The resulting solid is filtered to obtain the final compound 3-b.

In another embodiment, the chromenone derivative compound of the present invention may be prepared according to Scheme 4 below.

[Scheme 4]

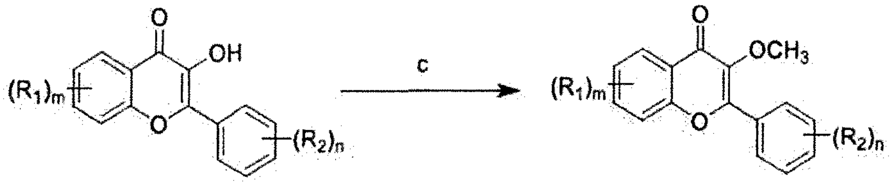


3-b 4-a

wherein the substituents $R_1$ and $R_2$, and the integers m and n are as defined above.

After dissolving compound 3-b in a solvent, $K_2CO_3$/MeI or NaOH/$(CH_3O)_2SO_2$ is added to the reaction mixture and stirred under appropriate reaction conditions. Compound 3-b may be prepared, for example, through Scheme 3. Upon completion of the reaction, the solvent is removed by distillation under reduced pressure, and the resulting solid is washed with a solvent and filtered under reduced pressure or purified by column chromatography to obtain the final compound 4-a.

In one embodiment, the dimer compound of the present invention may be prepared according to Scheme 5 below.

[Scheme 5]

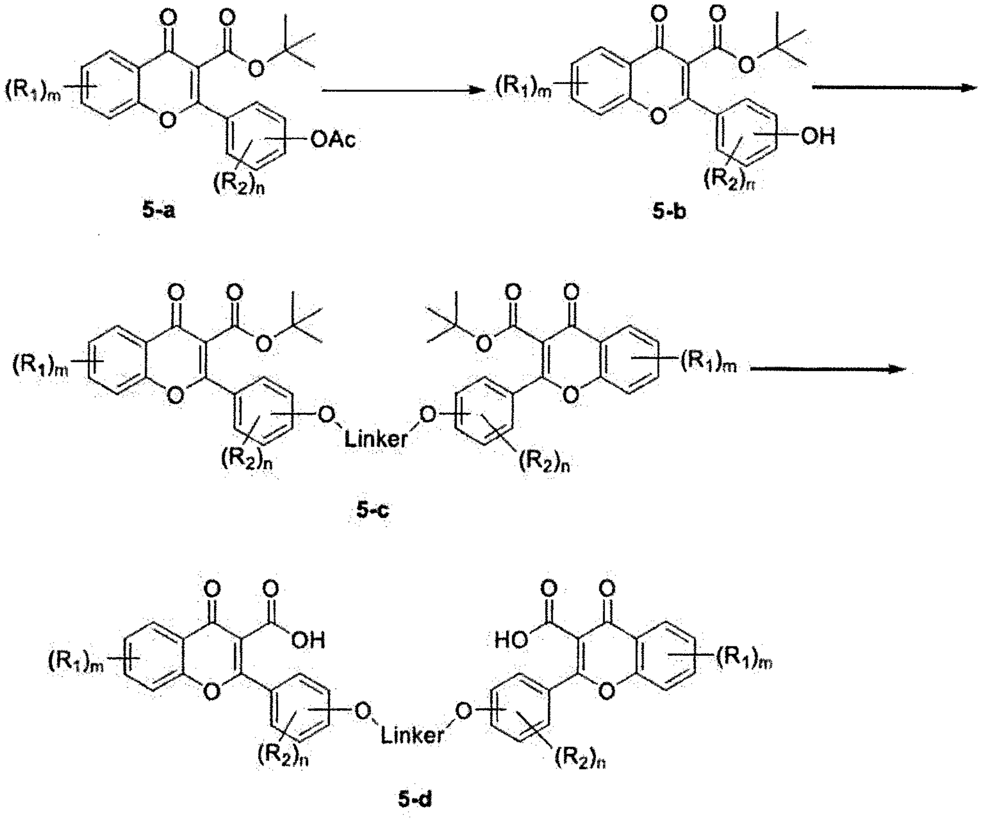

5-a 5-b 5-c 5-d

In the above formula, Linker refers to the linking group L, the substituents $R_1$ and $R_2$, the linking group L, and the integers m and n are as defined above.

After dissolving compound 5-a in a solvent, dimethylamine is added and the mixture is stirred under appropriate reaction conditions. When the reaction is completed, the solvent is removed by distillation under reduced pressure, and the resulting solid is washed with a solvent and filtered under reduced pressure or purified by column chromatography to obtain compound 5-b. Compound 5-c is synthesized by reacting compound 5-b with various di-p-tosylate derivatives or dibromoalkane compounds. The tert-butyl group of compound 5-c is deprotected using an acid to obtain the final compound 5-d.

[Scheme 6]

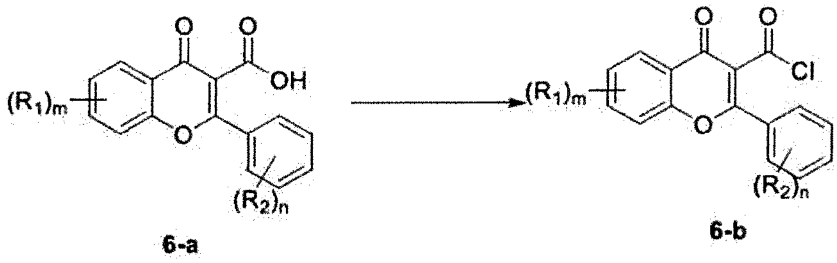

6-a 6-b

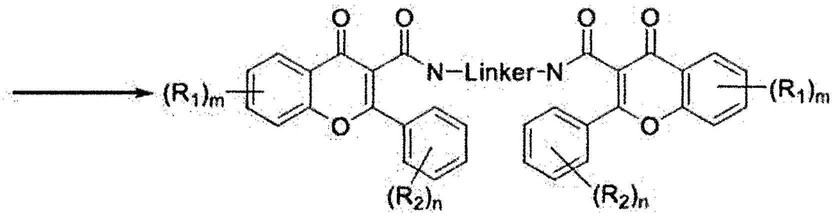

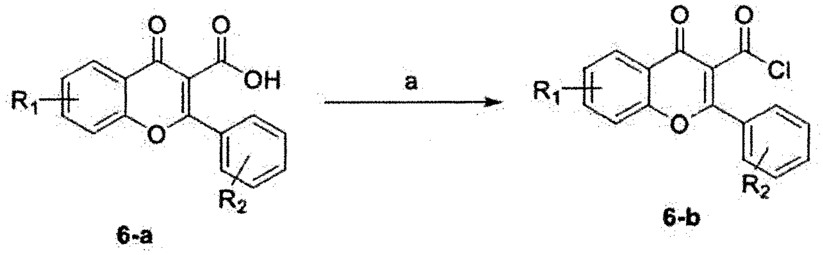


6-a 6-b

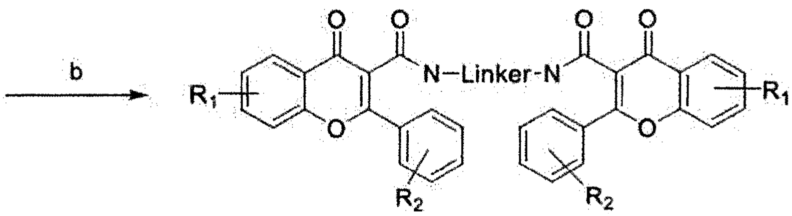


In the above formula, Linker refers to the linking group L, the substituents $R_1$ and $R_2$, the linking group L, and the integers m and n are as defined above.

The acid moiety of compound 6-a is chlorinated to obtain acid chloride, which is reacted with various alkyldiamine derivatives to obtain the final compound 6-c.

[Modes for Carrying Out the Invention]

## Examples

Hereinafter, to help the understanding of the present invention, examples will be described in detail. However, the following examples are provided to more completely explain the present invention to those with average knowledge in the art, and are only illustrative of the contents of the present invention, so that the scope of the present invention is not limited to the following examples.

**<Synthesis 1: Preparation of quinolone derivative compounds of Examples 1 to 18 >**

Based on Scheme 1, the quinolone derivative compounds of Examples 1 to 18 were prepared. Specifically, anthranilic acid benzotriazole derivative 1-a (1.0 eq) and benzoyl acetate derivative 1-b (1.0 eq) were dissolved in anhydrous THF and stirred under a nitrogen atmosphere for an appropriate time, for example, about 1 hour. t-BuOK (1.1 eq) was added dropwise and stirred for an appropriate time, e.g., about 3 to 5 hours. When the reaction was completed, the solvent was removed by distillation under reduced pressure, followed by extraction using dichloromethane

and distilled water. The organic layer was dried over anhydrous $MgSO_4$, filtered, and distilled under reduced pressure. Impurities were removed by dissolving in diethyl ether to obtain quinolone ester compound 1-c.

The final compound 1-d was synthesized by ester hydrolysis of compound 1-c in MeOH solution using 1 N aqueous NaOH solution.

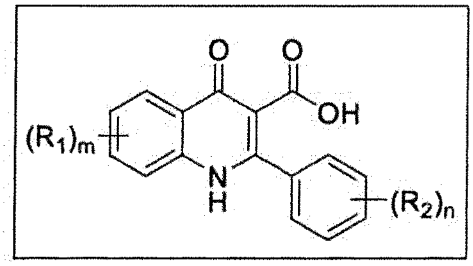

| | | |
|---|---|---|
| **1,** | $(R_1)_m$ = 7-chloro | $(R_2)_n$ = 2,4-difluoro |
| **2,** | $(R_1)_m$ = 7-chloro | $(R_2)_n$ = 2,5-difluoro |
| **3,** | $(R_1)_m$ = 7-chloro | $(R_2)_n$ = 3,4-difluoro |
| **4,** | $(R_1)_m$ = 7-chloro | $(R_2)_n$ = 3,5-difluoro |
| **5,** | $(R_1)_m$ = 7-chloro | $(R_2)_n$ = 2,6-difluoro |
| **6,** | $(R_1)_m$ = 6-chloro | $(R_2)_n$ = 2,5-difluoro |
| **7,** | $(R_1)_m$ = 6-chloro | $(R_2)_n$ = 3,4-difluoro |
| **8** | $(R_1)_m$ = 6-chloro | $(R_2)_n$ = 3,5-difluoro |
| **9,** | $(R_1)_m$ = 6-chloro | $(R_2)_n$ = 2,6-difluoro |
| **10,** | $(R_1)_m$ = 8-chloro | $(R_2)_n$ = 2,5-difluoro |
| **11,** | $(R_1)_m$ = 7-bromo | $(R_2)_n$ = 2,5-difluoro |
| **12,** | $(R_1)_m$ = 7-bromo | $(R_2)_n$ = 3,4-difluoro |
| **13,** | $(R_1)_m$ = 7-bromo | $(R_2)_n$ = 3,5-difluoro |
| **14,** | $(R_1)_m$ = 7-bromo | $(R_2)_n$ = 2,6-difluoro |
| **15,** | $(R_1)_m$=7-methoxy | $(R_2)_n$ = 2,5-difluoro |
| **16,** | $(R_1)_m$=7-methoxy | $(R_2)_n$ = 3,5-difluoro |
| **17,** | $(R_1)_m$=7-methoxy | $(R_2)_n$ = 2,6-difluoro |
| **18** | $(R_1)_m$ = 7-fluoro | $(R_2)_n$ = 2,5-difluoro |

### <Example 1> 2-(2,4-difluorophenyl)-3-carboxy-7-chloro-4-(1*H*)-quinolone

The title compound was prepared using the preparation method described in Synthesis 1. Specifically, (2-amino-4-chlorophenyl)(1*H*-benzo[*d*][1,2,3]triazol-1-yl)methanone (939 mg, 4.4 mmol, 1.0 eq) and methyl 3-(2,4-difluorophenyl)-3-oxopropanoate (1.2 g, 4.4 mmol, 1.0 eq) were stirred in anhydrous THF (10 mL) for 1 h, t-BuOK (541 mg, 4.8 mmol, 1.1 eq) was added dropwise and stirred for 5 hours, and the quinolone ester compound (20 mg, 0.08 mmol) obtained by terminating the reaction was stirred in MeOH (1 mL) and 1 N NaOH (1 mL) for 22 hours. As a result, 9.4 mg (49.0%) of the final target compound was obtained.

$^1H$ NMR (500 MHz, DMSO-$d_6$): $\delta$ 15.74 (s, 1H), 13.42 (s, 1H), 8.35 - 8.33 (m, 1H), 7.81 (s, 1H), 7.72 - 7.67 (m, 2H), 7.49 (td, *J* = 9.8, 2.1 Hz, 1H), 7.33 (td, *J* = 8.4, 2.1 Hz, 1H); $^{13}C$ NMR (125 MHz, DMSO-$d_6$): $\delta$ 178.16, 164.70, 163.41, 159.27, 151.31, 139.53, 1138.76, 131.32, 127.50, 126.77, 122.45, 119.57, 118.66, 111.48, 108.43, 104.00.

### <Example 2> 2-(2,5-difluorophenyl)-3-carboxy-7-chloro-4-(1*H*)-quinolone

The title compound was prepared using the preparation method described in Synthesis 1. Specifically, (2-amino-4-chlorophenyl)(1*H*-benzo[*d*][1,2,3]triazol-1-yl)methanone (300 mg, 1.4 mmol, 1.0 eq) and methyl 3-(2,5-difluorophenyl)-3-oxopropanoate (382 mg, 1.4 mmol, 1.0 eq) were stirred in anhydrous THF (5 mL) for 1 h, t-BuOK (172.8 mg, 1.5 mmol, 1.1 eq) was added dropwise and stirred for 5 hours, and the quinolone ester compound (50.0 mg, 0.14 mmol) obtained by terminating the reaction was stirred in MeOH (1.5 mL) and 1 N NaOH (1.5 mL) for 21 hours to obtain 31.4 mg (65.4%) of the final target compound.

$^1H$ NMR (500 MHz, DMSO-$d_6$): δ 15.68 (s, 1H), 13.50 (s, 1H), 8.35 (d, *J*= 8.8 Hz, 1H), 7.81 (d, *J* = 1.9 Hz, 1H), 7.70 (dd,*J* = 8.8, 1.9 Hz, 1H), 7.62 - 7.58 (m, 1H), 7.52 - 7.44 (m, 2H); $^{13}C$ NMR (125 MHz, DMSO-$d_6$): δ 178.18, 164.59, 157.67, 154.96, 150.72, 139.48, 138.88, 127.53, 126.92, 122.48, 118.67, 118.39, 118.20, 116.97, 116.78, 108.30.

**<Example 3> 2-(3,4-difluorophenyl)-3-carboxy-7-chloro-4-(1*H*)-quinolone**

The title compound was prepared using the preparation method described in Synthesis 1. Specifically, (2-amino-4-chlorophenyl)(1*H*-benzo[*d*][1,2,3]triazol-1-yl)methanone (300 mg, 1.4 mmol, 1.0 eq) and methyl 3-(3,4-difluorophenyl)-3-oxopropanoate (382 mg, 1.4 mmol, 1.0 eq) were stirred in anhydrous THF (5 mL) for 1 h, t-BuOK (172.8 mg, 1.5 mmol, 1.1 eq) was added dropwise and stirred for 5 hours, and the quinolone ester compound (57.7 mg, 0.17 mmol) obtained by terminating the reaction was stirred in MeOH (2 mL) and 1 N NaOH (2 mL) for 24 hours. As a result, 34.9 mg (63.0%) of the final target compound was obtained.

$^1H$ NMR (500 MHz, DMSO-$d_6$): δ 15.55 (s, 1H), 13.15 (s, 1H), 8.31 (d, *J* = 8.6 Hz, 1H), 7.80 (d, *J*= 1.9 Hz, 1H), 7.79 - 7.75 (m, 1H), 7.68 - 7.63 (m, 2H), 7.46 - 7.44 (m, 1H); $^{13}C$ NMR (125 MHz, DMSO-$d_6$): δ 178.04, 164.98, 155.13, 150.49, 148.52, 139.27, 138.56, 132.10, 127.44, 126.47, 125.78, 122.39, 118.50, 118.19, 117.31, 108.27.

**<Example 4> 2-(3,5-difluorophenyl)-3-carboxy-7-chloro-4-(1*H*)-quinolone**

The title compound was prepared using the preparation method described in Synthesis 1. Specifically, (2-amino-4-chlorophenyl)(1*H*-benzo[*d*][1,2,3]triazol-1-yl)methanone (300 mg, 1.4 mmol, 1.0 eq) and methyl 3-(3,5-difluorophenyl)-3-oxopropanoate (382 mg, 1.4 mmol, 1.0 eq) were stirred in anhydrous THF (5 mL) for 1 h, t-BuOK (172.8 mg, 1.5 mmol, 1.1 eq) was added dropwise and stirred for 4 hours, and the quinolone ester compound (53.0 mg, 0.15 mmol) obtained by terminating the reaction was stirred in MeOH (1.5 mL) and 1 N NaOH (1.5 mL) for 20 hours to obtain 40.2 mg (72.7%) of the final target compound.

$^1H$ NMR (500 MHz, DMSO-$d_6$): δ 15.52 (s, 1H), 13.23 (s, 1H), 8.32 (d, *J*= 8.7 Hz, 1H), 7.80 (d, *J* = 1.9 Hz, 1H), 7.67 (dd, *J* = 8.7, 1.9 Hz, 1H), 7.51 (tt, *J* = 9.5, 2.2 Hz, 1H), 7.42 - 7.40 (m, 2H); $^{13}C$ NMR (125 MHz, DMSO-$d_6$): δ 178.05, 164.82, 161.77, 154.76, 139.23, 138.67, 138.03, 127.45, 126.59, 122.42, 118.50, 112.08, 108.14, 105.05.

**<Example 5> 2-(2,6-difluorophenyl)-3-carboxy-7-chloro-4-(1*H*)-quinolone**

The title compound was prepared using the preparation method described in Synthesis 1. Specifically, (2-amino-4-chlorophenyl)(1*H*-benzo[*d*][1,2,3]triazol-1-yl)methanone (500 mg, 2.3 mmol, 1.0 eq) and methyl 3-(2,6-difluorophenyl)-3-oxopropanoate (635.4 mg, 2.3 mmol, 1.0 eq) were stirred in anhydrous THF (5 mL) for 1 h, t-BuOK (294.9 mg, 2.6 mmol, 1.1 eq) was added dropwise and stirred for 5 hours, and the quinolone ester compound (67.0 mg, 0.19 mmol) obtained by terminating the reaction was stirred in MeOH (2 mL) and 1 N NaOH (2 mL) for 24 hours to obtain 27.4 mg (39.3%) of the final target compound.

$^1H$ NMR (500 MHz, DMSO-$d_6$): δ 15.68 (s, 1H), 13.74 (s, 1H), 8.36 (d, *J* = 8.8 Hz, 1H), 7.80 (d, *J* = 1.8 Hz, 1H), 7.74 - 7.68 (m, 2H), 7.35 (t, *J* = 8.1 Hz, 2H); $^{13}C$ NMR (125 MHz, DMSO-$d_6$): δ 178.23, 164.44, 158.84, 146.33, 139.66, 139.08, 132.80, 132.73, 127.59, 127.11, 122.58, 118.71, 111.81, 108.91.

**<Example 6> 2-(2,5-difluorophenyl)-3-carboxy-6-chloro-4-(1*H*)-quinolone**

The title compound was prepared using the preparation method described in Synthesis 1. Specifically, (2-amino-5-chlorophenyl)(1*H*-benzo[*d*][1,2,3]triazol-1-yl)methanone (300 mg, 1.4 mmol, 1.0 eq) and methyl 3-(2,5-difluorophenyl)-3-oxopropanoate (382 mg, 1.4 mmol, 1.0 eq) were stirred in anhydrous THF (5 mL) for 1 h, t-BuOK (172.8 mg, 1.5 mmol, 1.1 eq) was added dropwise and stirred for 4 hours, and the quinolone ester compound (50.0 mg, 0.14 mmol) obtained by terminating the reaction was stirred in MeOH (1.5 mL) and 1 N NaOH (1.5 mL) for 19 hours. As a result, 26.3 mg (56.0%) of the final target compound was obtained.

$^1H$ NMR (500 MHz, DMSO-$d_6$): δ 15.62 (s, 1H), 13.61 (s, 1H), 8.29 (d, *J* = 2.4 Hz, 1H), 7.99 (dd, *J* = 9.3, 2.4 Hz, 1H), 7.84 (d, *J* = 9.3 Hz, 1H), 7.61 - 7.58 (m, 1H), 7.51 - 7.42 (m, 2H); $^{13}C$ NMR (125 MHz, DMSO-$d_6$): δ 178.09, 165.07, 158.15, 155.47, 150.82, 137.91, 135.03, 131.60, 125.36, 124.58, 122.47, 118.84, 118.66, 117.44, 117.25, 108.67.

**<Example 7> 2-(3,4-difluorophenyl)-3-carboxy-6-chloro-4-(1*H*)-quinolone**

The title compound was prepared using the preparation method described in Synthesis 1. Specifically, (2-amino-5-chlorophenyl)(1*H*-benzo[*d*][1,2,3]triazol-1-yl)methanone (300 mg, 1.4 mmol, 1.0 eq) and methyl 3-(3,4-difluorophenyl)-3-oxopropanoate (382 mg, 1.4 mmol, 1.0 eq) were stirred in anhydrous THF (5 mL) for 1 h, t-BuOK (172.8 mg, 1.5 mmol, 1.1 eq) was added dropwise and stirred for 5 hours, and the quinolone ester compound (52.7 mg, 0.15 mmol)

obtained by terminating the reaction was stirred in MeOH (2 mL) and 1 N NaOH (2 mL) for 22 hours to obtain 25.3 mg (50.2%) of the final target compound.

$^1$H NMR (500 MHz, DMSO-$d_6$): δ 15.52 (s, 1H), 13.28 (s, 1H), 8.25 (d, *J* = 2.4 Hz, 1H), 7.96 (dd, *J* = 8.9, 2.4 Hz, 1H), 7.84 (d, *J* = 8.9 Hz, 1H), 7.80 - 7.75 (m, 1H), 7.64 (dt, *J* = 10.7, 8.5 Hz, 1H), 7.46 - 7.44 (m, 1H); $^{13}$C NMR (125 MHz, DMSO-$d_6$): δ 177.41, 164.99, 154.81, 150.46, 148.50, 137.25, 134.20, 132.14, 130.66, 125.80, 124.78, 123.96, 121.88, 118.20, 117.25, 108.14.

**<Example 8> 2-(3,5-difluorophenyl)-3-carboxy-6-chloro-4-(1*H*)-quinolone**

The title compound was prepared using the preparation method described in Synthesis 1. Specifically, (2-amino-5-chlorophenyl)(1*H*-benzo[*d*][1,2,3]triazol-1-yl)methanone (300 mg, 1.4 mmol, 1.0 eq) and methyl 3-(3,5-difluorophenyl)-3-oxopropanoate (382 mg, 1.4 mmol, 1.0 eq) were stirred in anhydrous THF (5 mL) for 1 h, t-BuOK (172.8 mg, 1.5 mmol, 1.1 eq) was added dropwise and stirred for 5 hours, and the quinolone ester compound (47.6 mg, 0.14 mmol) obtained by terminating the reaction was stirred in MeOH (1.5 mL) and 1 N NaOH (1.5 mL) for 21 hours to obtain 32.4 mg (65.5%) of the final target compound.

$^1$H NMR (500 MHz, DMSO-$d_6$): δ 15.48 (s, 1H), 13.35 (s, 1H), 8.26 (d, *J*= 2.4 Hz, 1H), 7.97 (dd, *J* = 8.9, 2.4 Hz, 1H), 7.84 (d, *J* = 8.9 Hz, 1H), 7.50 (tt, *J* = 9.5, 2.1 Hz, 1H), 7.41 - 7.40 (m 2H); $^{13}$C NMR (125 MHz, DMSO-$d_6$): δ 177.44, 164.81, 161.74, 154.45, 138.05, 137.21, 134.31, 130.78, 124.82, 123.98, 121.87, 112.11, 108.00, 104.98.

**<Example 9> 2-(2,6-difluorophenyl)-3-carboxy-6-chloro-4-(1*H*)-quinolone**

The title compound was prepared using the preparation method described in Synthesis 1. Specifically, (2-amino-5-chlorophenyl)(1*H*-benzo[*d*][1,2,3]triazol-1-yl)methanone (500 mg, 2.3 mmol, 1.0 eq) and methyl 3-(2,6-difluorophenyl)-3-oxopropanoate (635.4 mg, 2.3 mmol, 1.0 eq) were stirred in anhydrous THF (5 mL) for 1 h, t-BuOK (294.9 mg, 2.6 mmol, 1.1 eq) was added dropwise and stirred for 5 hours, and the quinolone ester compound (42.3 mg, 0.12 mmol) obtained by terminating the reaction was stirred in MeOH (2 mL) and 1 N NaOH (2 mL) for 20 hours to obtain 21.7 mg (49.3%) of the final target compound.

$^1$H NMR (500 MHz, DMSO-$d_6$): δ 15.62 (s, 1H), 13.86 (s, 1H), 8.31 (d, *J*= 2.4 Hz, 1H), 8.01 (dd, *J* = 8.9, 2.4 Hz, 1H), 7.84 (d, *J* = 8.9 Hz, 1H), 7.74 - 7.68 (m, 1H), 7.34 (t, *J* = 8.1 Hz, 2H); $^{13}$C NMR (125 MHz, DMSO-$d_6$): δ 177.65, 164.43, 158.86, 145.90, 137.61, 134.66, 132.70, 131.31, 124.97, 124.18, 121.97, 111.80, 108.75.

**<Example 10> 2-(2,5-difluorophenyl)-3-carboxy-8-chloro-4-(1*H*)-quinolone**

The title compound was prepared using the preparation method described in Synthesis 1. Specifically, (2-amino-3-chlorophenyl)(1*H*-benzo[*d*][1,2,3]triazol-1-yl)methanone (300 mg, 1.4 mmol, 1.0 eq) and methyl 3-(2,4-difluorophenyl)-3-oxopropanoate (382 mg, 1.4 mmol, 1.0 eq) were stirred in anhydrous THF (5 mL) for 1 h, t-BuOK (172.8 mg, 1.5 mmol, 1.1 eq) was added dropwise and stirred for 5 hours, and the quinolone ester compound (56.0 mg, 0.16 mmol) obtained by terminating the reaction was stirred in MeOH (1.5 mL) and 1 N NaOH (1.5 mL) for 22 hours. As a result, 19.2 mg (35.8%) of the final target compound was obtained.

$^1$H NMR (500 MHz, DMSO-$d_6$): δ 15.22 (s, 1H), 12.51 (s, 1H), 8.33 (dd, *J* = 7.9, 1.2 Hz, 1H), 8.10 (dd, *J*= 7.9, 1.2 Hz, 1H), 7.63 (t, *J* = 7.9 Hz, 1H), 7.49 - 7.45 (m, 1H), 7.44 - 7.41 (m, 1H), 7.39 - 7.35 (m, 1H); $^{13}$C NMR (125 MHz, DMSO-$d_6$): δ 177.56, 164.70, 157.60, 155.37, 151.24, 136.12, 134.48, 126.55, 125.64, 124.54, 123.19, 117.81, 117.63, 117.20, 116.37, 109.60.

**<Example 11> 2-(2,5-difluorophenyl)-3-carboxy-7-bromo-4-(1*H*)-quinolone**

The title compound was prepared using the preparation method described in Synthesis 1. Specifically, (2-amino-4-bromophenyl)(1*H*-benzo[*d*][1,2,3]triazol-1-yl)methanone (450 mg, 2.1 mmol, 1.0 eq) and methyl 3-(2,4-difluorophenyl)-3-oxopropanoate (666 mg, 2.1 mmol, 1.0 eq) were stirred in anhydrous THF (5 mL) for 1 h, t-BuOK (259 mg, 2.31 mmol, 1.1 eq) was added dropwise and stirred for 5 hours, and the quinolone ester compound (48.6 mg, 0.12 mmol) obtained by terminating the reaction was stirred in MeOH (2.5 mL) and 1 N NaOH (2.5 mL) for 20 hours to obtain 24.9 mg (53.3%) of the final target compound.

$^1$H NMR (500 MHz, DMSO-$d_6$): δ 15.66 (s, 1H), 13.45 (s, 1H), 8.26 (d, *J* = 8.7 Hz, 1H), 7.97 (d, *J*= 1.6 Hz, 1H), 7.82 (dd, *J* = 8.7, 1.6 Hz, 1H), 7.61 - 7.57 (m, 1H), 7.53 - 7.43 (m, 2H); $^{13}$C NMR (125 MHz, DMSO-$d_6$): δ 178.31, 164.57, 157.67, 154.98, 150.62, 139.58, 129.57, 127.79, 127.41, 124.24, 122.76, 121.75, 118.27, 116.89, 116.79, 108.36.

**<Example 12> 2-(3,4-difluorophenyl)-3-carboxy-7-bromo-4-(1*H*)-quinolone**

The title compound was prepared using the preparation method described in Synthesis 1. Specifically, (2-amino-4-bromophenyl)(1*H*-benzo[*d*][1,2,3]triazol-1-yl)methanone (350 mg, 1.6 mmol, 1.0 eq) and methyl 3-(3,4-difluorophenyl)-3-oxopropanoate (518 mg, 1.6 mmol, 1.0 eq) were stirred in anhydrous THF (5 mL) for 1 h, t-BuOK (201 mg, 1.8 mmol, 1.1 eq) was added dropwise and stirred for 5 hours, and the quinolone ester compound (81.0 mg, 0.2 mmol) obtained by terminating the reaction was stirred in MeOH (1.5 mL) and 1 N NaOH (1.5 mL) for 17 hours to obtain 64.5 mg (78.1%) of the final target compound.

$^1$H NMR (500 MHz, DMSO-$d_6$): δ 15.51 (s, 1H), 13.11 (s, 1H), 8.24 (d, *J* = 8.7 Hz, 1H), 7.97 (d, *J* = 1.5 Hz, 1H), 7.79 - 7.75 (m, 2H), 7.69 (q, *J* = 9.5 Hz, 1H), 7.46 - 7.45 (m, 1H); $^{13}$C NMR (125 MHz, DMSO-$d_6$): δ 178.18, 164.93, 154.98, 150.47, 148.56, 139.33, 132.04, 129.04, 127.47, 127.35, 125.78, 122.68, 121.57, 118.19, 117.29, 108.35.

**<Example 13> 2-(3,5-difluorophenyl)-3-carboxy-7-bromo-4-(1*H*)-quinolone**

The title compound was prepared using the preparation method described in Synthesis 1. Specifically, (2-amino-4-bromophenyl)(1*H*-benzo[*d*][1,2,3]triazol-1-yl)methanone (400 mg, 1.9 mmol, 1.0 eq) and methyl 3-(3,5-difluorophenyl)-3-oxopropanoate (593 mg, 1.9 mmol, 1.0 eq) were stirred in anhydrous THF (5 mL) for 1 h, t-BuOK (230 mg , 2.1 mmol, 1.1 eq) was added dropwise and stirred for 5 hours, and the quinolone ester compound (80.0 mg, 0.2 mmol) obtained by terminating the reaction was stirred in MeOH (2 mL) and 1 N NaOH (2 mL) for 20 hours. As a result, 39.8 mg (51.6%) of the final target compound was obtained.

$^1$H NMR (500 MHz, DMSO-$d_6$): δ 15.50 (s, 1H), 13.19 (s, 1H), 8.24 (d, *J* = 8.7 Hz, 1H), 7.80 (s, 1H), 7.79 (dd, *J* = 8.7, 1.1 Hz, 1H), 7.50 (t, *J* = 9.5 Hz, 1H), 7.40 (d, *J* = 5.9 Hz, 2H); $^{13}$C NMR (125 MHz, DMSO-$d_6$): δ 178.17, 164.80, 161.77, 154.64, 139.35, 138.04, 129.25, 127.56, 127.35, 112.09, 108.20, 105.02.

**<Example 14> 2-(2,6-difluorophenyl)-3-carboxy-7-bromo-4-(1*H*)-quinolone**

The title compound was prepared using the preparation method described in Synthesis 1. Specifically, (2-amino-4-bromophenyl)(1*H*-benzo[*d*][1,2,3]triazol-1-yl)methanone (350 mg, 1.6 mmol, 1.0 eq) and methyl 3-(2,6-difluorophenyl)-3-oxopropanoate (518 mg, 1.6 mmol, 1.0 eq) were stirred in anhydrous THF (5 mL) for 1 h, t-BuOK (201 mg, 1.8 mmol, 1.1 eq) was added dropwise and stirred for 5 hours, and the quinolone ester compound (40.0 mg, 0.1 mmol) obtained by terminating the reaction was stirred in MeOH (2 mL) and 1 N NaOH (2 mL) for 23 hours. As a result, 23.6 mg (61.1%) of the final target compound was obtained.

$^1$H NMR (500 MHz, DMSO-$d_6$): δ 15.67 (s, 1H), 13.72 (s, 1H), 8.28 (d, *J* = 8.7 Hz, 1H), 7.96 (d, *J* = 1.4 Hz, 1H), 7.84 (dd, *J* = 8.7, 1.4 Hz, 1H), 7.74 - 7.68 (m, 1H), 7.35 (t, *J* = 8.1 Hz, 2H); $^{13}$C NMR (125 MHz, DMSO-$d_6$): δ 178.38, 164.48, 158.79, 146.20, 139.65, 132.82, 129.87, 128.10, 127.52, 122.81, 121.68, 111.80, 108.92.

**<Example 15> 2-(2,5-difluorophenyl)-3-carboxy-7-methoxy-4-(1*H*)-quinolone**

The title compound was prepared using the preparation method described in Synthesis 1. Specifically, (2-amino-4-methoxyphenyl)(1*H*-benzo[*d*][1,2,3]triazol-1-yl)methanone (350 mg, 1.6 mmol, 1.0 eq) and methyl 3-(2,5-difluorophenyl)-3-oxopropanoate (482 mg, 1.8 mmol, 1.0 eq) were stirred in anhydrous THF (5 mL) for 1 h, t-BuOK (202 mg, 1.8 mmol, 1.0 eq) was added dropwise and stirred for 3 hours, and the quinolone ester compound (83.0 mg, 0.24 mmol) obtained by terminating the reaction was stirred in MeOH (1.5 mL) and 1 N NaOH (1.5 mL) for 22 hours to obtain 47.8 mg (60.0%) of the final target compound.

$^1$H NMR (500 MHz, DMSO-$d_6$): δ 16.21 (s, 1H), 13.25 (s, 1H), 8.25 (d, *J* = 9.0 Hz, 1H), 7.58 (s, 1H), 7.48 - 7.43 (m, 2H), 7.26 (d, *J* = 9.0 Hz, 1H), 7.18 (s, 1H); $^{13}$C NMR (125 MHz, DMSO-$d_6$): δ 178.05, 164.94, 163.75, 157.65, 156.03, 149.97, 140.76, 127.08, 124.48, 117.99, 117.70, 116.84, 116.75, 107.08, 100.08, 55.86.

**<Example 16> 2-(3,5-difluorophenyl)-3-carboxy-7-methoxy-4-(1*H*)-quinolone**

The title compound was prepared using the preparation method described in Synthesis 1. Specifically, (2-amino-4-methoxyphenyl)(1*H*-benzo[*d*][1,2,3]triazol-1-yl)methanone (350 mg, 1.6 mmol, 1.0 eq) and methyl 3-(3,5-difluorophenyl)-3-oxopropanoate (482 mg, 1.8 mmol, 1.1 eq) were stirred in anhydrous THF (5 mL) for 1 h, t-BuOK (202 mg, 1.8 mmol, 1.1 eq) was added dropwise and stirred for 3 hours, and the quinolone ester compound (98.2 mg, 0.28 mmol) obtained by terminating the reaction was stirred in MeOH (1.5 mL) and 1 N NaOH (1.5 mL) for 22 hours to obtain 87.5 mg (90.0%) of the final target compound.

$^1$H NMR (500 MHz, DMSO-$d_6$): δ 16.16 (s, 1H), 13.02 (s, 1H), 8.24 (d, *J* = 9.0 Hz, 1H), 7.46 (t, *J* = 9.3 Hz, 1H), 7.37 (d, *J* = 6.6 Hz, 2H), 7.25 (dd, *J* = 9.0, 1.7 Hz, 1H), 7.19 (d, *J* = 1.7 Hz, 1H), 3.92 (s, 3H); $^{13}$C NMR (125 MHz, DMSO-

$d_6$): δ 178.65, 165.59, 164.14, 162.20, 155.08, 141.05, 138.98, 127.51, 118.16, 117.03, 112.61, 106.90, 105.19, 100.65, 56.34.

**<Example 17> 2-(2,6-difluorophenyl)-3-carboxy-7-methoxy-4-(1*H*)-quinolone**

The title compound was prepared using the preparation method described in Synthesis 1. Specifically, (2-amino-4-methoxyphenyl)(1*H*-benzo[*d*][1,2,3]triazol-1-yl)methanone (350 mg, 1.6 mmol, 1.0 eq) and methyl 3-(2,6-difluorophenyl)-3-oxopropanoate (482 mg, 1.8 mmol, 1.1 eq) were stirred in anhydrous THF (5 mL) for 1 h, t-BuOK (202 mg, 1.8 mmol, 1.1 eq) was added dropwise and stirred for 3 hours, and the quinolone ester compound (49.8 mg, 0.14 mmol) obtained by terminating the reaction was stirred in MeOH (1.5 mL) and 1 N NaOH (1.5 mL) for 22 hours to obtain 31.0 mg (64.9%) of the final target compound.

$^1$H NMR (500 MHz, DMSO-$d_6$): δ 16.22 (s, 1H), 13.52 (s, 1H), 8.27 (d, *J* = 9.1 Hz, 1H), 7.72 - 7.66 (m, 1H), 7.33 (t, *J* = 8.1 Hz, 2H), 7.29 (dd, *J* = 9.0, 2.3 Hz, 1H), 7.16 (d, *J* = 2.3 Hz, 1H), 3.94 (s, 3H); $^{13}$C NMR (125 MHz, DMSO-$d_6$): δ 178.56, 165.30, 164.41, 159.38, 145.97, 141.43, 132.99, 127.68, 118.24, 117.72, 112.24, 112.05, 108.20, 100.49, 56.40.

**<Example 18> 2-(2,5-difluorophenyl)-3-carboxy-7-fluoro-4-(1*H*)-quinolone**

The title compound was prepared using the preparation method described in Synthesis 1. Specifically, (2-amino-4-fluorophenyl)(1*H*-benzo[*d*][1,2,3]triazol-1-yl)methanone (350 mg, 1.6 mmol, 1.0 eq) and methyl 3-(2,5-difluorophenyl)-3-oxopropanoate (460 mg, 1.8 mmol, 1.1 eq) were stirred in anhydrous THF (5 mL) for 1 h, t-BuOK (202 mg , 1.8 mmol, 1.1 eq) was added dropwise and stirred for 3 hours, and the quinolone ester compound (79.4 mg, 0.24 mmol) obtained by terminating the reaction was stirred in MeOH (1.5 mL) and 1 N NaOH (1.5 mL) for 23 hours. As a result, 47.8 mg (52.6%) of the final target compound was obtained.

$^1$H NMR (500 MHz, DMSO-$d_6$): δ 15.75 (s, 1H), 13.49 (s, 1H), 8.43 (dd, *J* = 8.8, 6.1 Hz, 1H), 7.60 - 7.43 (m, 5H); $^{13}$C NMR (125 MHz, DMSO-$d_6$): δ 178.16, 165.03, 164.59, 157.67, 154.95, 150.82, 140.26, 128.86, 124.17, 120.86, 118.21, 116.85, 116.79, 115.65, 108.00, 104.78.

**<Synthesis 2: Preparation of quinolone derivative compounds of Examples 19 to 31>**

Based on Scheme 2, the quinolone derivative compounds of Examples 19 to 31 were prepared. Specifically, after dissolving compound 1-c (1.0 eq) in DMF, NaH (1.5 eq) was added and stirred at room temperature for an appropriate time, e.g., about 30 minutes. After iodomethane (2.0 eq) was added dropwise, the mixture was stirred at 60° C for an appropriate time, for example, about 3 to 5 hours, and when the reaction was completed, the solvent was removed by distillation under reduced pressure. After extraction with ethyl acetate and brine, the organic layer was dried over anhydrous $MgSO_4$ and filtered under reduced pressure to remove the solvent. Compound 2-a was obtained by purification by column chromatography (n-hexane/EtOAc = 10-15:1).

Compound 2-a was dissolved in MeOH and 1 N aqueous NaOH solution, and then stirred at 60° C under a nitrogen atmosphere for an appropriate time, for example, about 12 to 24 hours. When the reaction was completed, the solvent was removed by distillation under reduced pressure, and 1 N HCl aqueous solution was added dropwise to adjust the pH to 4 to 5, and the resulting solid was filtered with water to obtain the quinolone derivative compounds 2-b of Examples 19 to 31.

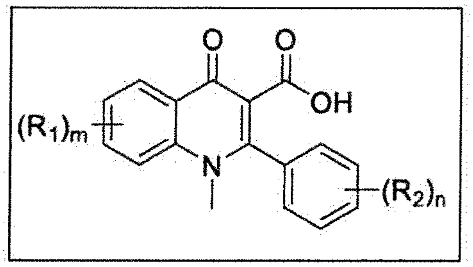

**19,** $(R_1)_m$ = 7-chloro $(R_2)_n$ = 2,4-difluoro
**20,** $(R_1)_m$ = 7-chloro $(R_2)_n$ = 2,5-difluoro
**21,** $(R_1)_m$ = 7-chloro $(R_2)_n$ = 3,4-difluoro

(continued)

| | | |
|---|---|---|
| **22.** | $(R_1)_m$ = 7-chloro | $(R_2)_n$ = 3,5-difluoro |
| **23,** | $(R_1)_m$ = 7-chloro | $(R_2)_n$ = 2,6-difluoro |
| **24,** | $(R_1)_m$ = 6-chloro | $(R_2)_n$ = 2,5-difluoro |
| **25,** | $(R_1)_m$ = 6-chloro | $(R_2)_n$ = 3,4-difluoro |
| **26,** | $(R_1)_m$ = 6-chloro | $(R_2)_n$ = 3,5-difluoro |
| **27,** | $(R_1)_m$ = 6-chloro | $(R_2)_n$ = 2,6-difluoro |
| **28,** | $(R_1)_m$ = 8-chloro | $(R_2)_n$ = 2,5-difluoro |
| **29,** | $(R_1)_m$ = 7-bromo | $(R_2)_n$ = 2,5-difluoro |
| **30,** | $(R_1)_m$ = 7-bromo | $(R_2)_n$ = 3,5-difluoro |
| **31,** | $(R_1)_m$ = 7-bromo | $(R_2)_n$ = 2,6-difluoro |

**<Example 19> 1-methyl-2-(2,4-difluorophenyl)-3-carboxy-7-chloro-4-(1*H*)-quinolone**

The title compound was prepared using the preparation method described in Synthesis 2. Specifically, 2-(2,4-difluorophenyl)-3-methoxycarbonyl-7-chloro-4-(1*H*)-quinolone (100 mg, 0.29 mmol) and NaH (17.2 mg, 0.43 mmol) were stirred in DMF (2 mL) for 30 min. After iodomethane (0.036 mL, 0.43 mmol) was added dropwise, the mixture was stirred for 4 h, and further stirred in MeOH (1.5 mL) and 1 N NaOH (1.5 mL) for 15 h. When the reaction was completed, the solvent was removed by distillation under reduced pressure, and 1 N HCl aqueous solution was added dropwise to adjust the pH to 4-5, and the resulting solid was filtered with water to obtain 24.8 mg (24.8%) of the final target compound.

$^1$H NMR (500 MHz, DMSO-$d_6$): δ 15.13 (s, 1H), 8.42 (d, *J*= 8.5 Hz, 1H), 8.21 (s, 1H), 7.75 (d, *J* = 8.5 Hz, 1H), 7.56 - 7.53 (m, 2H), 7.32 (t, *J* = 7.7 Hz, 1H), 3.65 (s, 3H); $^{13}$C NMR (125 MHz, DMSO-$d_6$): δ 176.27, 164.81, 164.36, 158.58, 152.38, 141.31, 139.37, 131.13, 127.77, 126.59, 123.70, 119.98, 118.43, 112.34, 112.17, 104.42, 38.07.

**<Example 20> 1-methyl-2-(2,5-difluorophenyl)-3-carboxy-7-chloro-4-(1*H*)quinolone**

The title compound was prepared using the preparation method described in Synthesis 2. Specifically, 2-(2,5-difluorophenyl)-3-methoxycarbonyl-7-chloro-4-(1*H*)-quinolone (100 mg, 0.29 mmol) and NaH (17.2 mg, 0.43 mmol) were stirred in DMF (2 mL) for 30 min. After iodomethane (0.036 mL, 0.43 mmol) was added dropwise, the mixture was stirred for 4 h, and further stirred in MeOH (1.5 mL) and 1 N NaOH (1.5 mL) for 12 h. When the reaction was completed, the solvent was removed by distillation under reduced pressure, and 1N HCl aqueous solution was added dropwise to adjust the pH to 4-5, and the resulting solid was filtered with water to obtain 41.4 mg (41.4%) of the final target compound.

$^1$H NMR (500 MHz, DMSO-$d_6$): δ 15.21 (s, 1H), 8.43 (d, *J* = 8.7 Hz, 1H), 8.23 (s, 1H), 7.76 (d, *J* = 8.7 Hz, 1H), 7.52 - 7.49 (m, 2H), 7.44 - 7.42 (m, 1H), 3.67 (s, 3H); $^{13}$C NMR (125 MHz, DMSO-$d_6$): δ 176.43, 164.65, 158.06, 154.51, 152.01, 141.24, 139.52, 127.79, 126.75, 123.66, 123.39, 118.68, 118.43, 117.37, 116.48, 111.70, 38.16.

**<Example 21> 1-methyl-2-(3,4-difluorophenyl)-3-carboxy-7-chloro-4-(1*H*)-quinolone**

The title compound was prepared using the preparation method described in Synthesis 2. Specifically, 2-(3,4-difluorophenyl)-3-methoxycarbonyl-7-chloro-4-(1*H*)-quinolone (105.7 mg, 0.3 mmol) and NaH (18.1 mg, 0.45 mmol) were stirred in DMF (2 mL) for 30 min. After iodomethane (0.038 mL, 0.6 mmol) was added dropwise, the mixture was stirred for 4 h, and further stirred in MeOH (2 mL) and 1 N NaOH (2 mL) for 22 h. When the reaction was completed, the solvent was removed by distillation under reduced pressure, and 1 N HCl aqueous solution was added dropwise to adjust the pH to 4-5, and the resulting solid was filtered with water to obtain 45.7 mg (43.5%) of the final target compound.

$^1$H NMR (500 MHz, DMSO-$d_6$): δ 15.04 (s, 1H), 8.40 (d, *J* = 8.7 Hz, 1H), 8.19 (d, *J* = 1.5 Hz, 1H), 7.72 (dd, *J* = 8.7, 1.5 Hz, 1H), 7.68 - 7.60 (m, 2H), 7.31 - 7.29 (m, 1H), 3.57 (s, 3H); $^{13}$C NMR (125 MHz, DMSO-$d_6$): δ 176.70, 165.54, 156.77, 151.11, 149.15, 141.75, 139.74, 132.20, 128.20, 126.79, 125.88, 124.18, 118.70, 118.45, 118.26, 112.53, 39.80.

**<Example 22> 1-methyl-2-(3,5-difluorophenyl)-3-carboxy-7-chloro-4-(1*H*)-quinolone**

The title compound was prepared using the preparation method described in Synthesis 2. Specifically, 2-(3,5-difluorophenyl)-3-methoxycarbonyl-7-chloro-4-(1*H*)-quinolone (100 mg, 0.29 mmol) and NaH (17.2 mg, 0.43 mmol) were stirred in DMF (2 mL) for 30 min. After iodomethane (0.036 mL, 0.43 mmol) was added dropwise, the mixture was stirred for 5 h and further stirred in MeOH (1.5 mL) and 1 N NaOH (1.5 mL) for 14 h. When the reaction was completed, the solvent was removed by distillation under reduced pressure, and 1 N HCl aqueous solution was added dropwise to

adjust the pH to 4-5, and the resulting solid was filtered with water to obtain 43.8 mg (43.8%) of the final target compound.

$^1$H NMR (500 MHz, DMSO-$d_6$): δ 15.15 (s, 1H), 8.41 (d, *J* = 8.7 Hz, 1H), 8.20 (d, *J* = 0.9 Hz, 1H), 7.74 (dd, *J* = 8.7, 0.9 Hz, 1H), 7.48 (t, *J* = 9.5 Hz, 1H), 7.27 (d, *J* = 5.7 Hz, 2H), 3.58 (s, 3H); $^{13}$C NMR (125 MHz, DMSO-$d_6$): δ 176.38, 164.83, 162.28, 156.13, 141.21, 139.38, 137.66, 127.72, 126.46, 123.63, 118.20, 111.77, 111.35, 104.98, 38.53.

**<Example 23> 1-methyl-2-(2,6-difluorophenyl)-3-carboxy-7-chloro-4-(1*H*)-quinolone**

The title compound was prepared using the preparation method described in Synthesis 2. Specifically, 2-(2,6-difluorophenyl)-3-methoxycarbonyl-7-chloro-4-(1*H*)-quinolone (100 mg, 0.29 mmol) and NaH (17.2 mg, 0.43 mmol) were stirred in DMF (2 mL) for 30 min. After iodomethane (0.036 mL, 0.43 mmol) was added dropwise, the mixture was stirred for 3 h and further stirred in MeOH (2 mL) and 1 N NaOH (2 mL) for 15 h. When the reaction was completed, the solvent was removed by distillation under reduced pressure, and 1N HCl aqueous solution was added dropwise to adjust the pH to 4-5, and the resulting solid was filtered with water to obtain 28.8 mg (28.8%) of the final target compound.

$^1$H NMR (500 MHz, DMSO-$d_6$): δ 15.21 (s, 1H), 8.44 (d, *J* = 8.7 Hz, 1H), 8.24 (d, *J* = 1.3 Hz, 1H), 7.78 (dd, *J* = 8.7, 1.3 Hz, 1H), 7.76 - 7.71 (m, 1H), 7.36 (t, *J* = 8.1 Hz, 2H), 3.76 (s, 3H); $^{13}$C NMR (125 MHz, DMSO-$d_6$): δ 176.43, 164.43, 158.35, 147.66, 141.33, 139.66, 133.41, 127.88, 127.02, 123.72, 118.65, 112.12, 111.22,37.81.

**<Example 24> 1-methyl-2-(2,5-difluorophenyl)-3-carboxy-6-chloro-4-(1*H*)-quinolone**

The title compound was prepared using the preparation method described in Synthesis 2. Specifically, (2,5-difluorophenyl)-3-methoxycarbonyl-6-chloro-4-(1*H*)-quinolone (100 mg, 0.29 mmol) and NaH (17.2 mg, 0.43 mmol) were stirred in DMF (2 mL) for 30 min. After iodomethane (0.036 mL, 0.43 mmol) was added dropwise, the mixture was stirred for 5 h, and further stirred in MeOH (2 mL) and 1 N NaOH (2 mL) for 18 h. When the reaction was completed, the solvent was removed by distillation under reduced pressure, and 1 N HCl aqueous solution was added dropwise to adjust the pH to 4-5, and the resulting solid was filtered with water to obtain 49.8 mg (49.8%) of the final target compound.

$^1$H NMR (500 MHz, DMSO-$d_6$): δ 15.13 (s, 1H), 8.34 (s, 1H), 8.15 - 8.14 (m, 1H), 8.08 - 8.06 (s, 1H), 7.53 - 7.51 (m, 2H), 7.46 - 7.42 (s, 1H), 3.69 (s, 3H); $^{13}$C NMR (125 MHz, DMSO-$d_6$): δ 175.79, 164.65, 158.05, 154.54, 151.60, 139.14, 134.31, 131.19, 126.13, 124.40, 123.34, 121.30, 118.69, 117.36, 116.52, 111.69, 39.12.

**<Example 25> 1-methyl-2-(3,4-difluorophenyl)-3-carboxy-6-chloro-4-(1*H*)-quinolone**

The title compound was prepared using the preparation method described in Synthesis 2. Specifically, 2-(3,4-difluorophenyl)-3-methoxycarbonyl-6-chloro-4-(1*H*)-quinolone (100 mg, 0.29 mmol) and NaH (17.2 mg, 0.43 mmol) were stirred in DMF (2 mL) for 30 min. After iodomethane (0.036 mL, 0.43 mmol) was added dropwise, the mixture was stirred for 5 h and further stirred in MeOH (2 mL) and 1 N NaOH (2 mL) for 18 h. When the reaction was completed, the solvent was removed by distillation under reduced pressure, and 1 N aqueous HCl solution was added dropwise to adjust the pH to 4-5, and the resulting solid was filtered with water to obtain 50.7 mg (50.7%) of the final target compound.

$^1$H NMR (500 MHz, DMSO-$d_6$): δ 14.91 (s, 1H), 8.33 (d, *J* = 2.5 Hz, 1H), 8.10 (d, *J* = 9.2 Hz, 1H), 8.02 (dd, *J* = 9.2, 2.5 Hz, 1H), 7.67 - 7.60 (m, 2H), 7.31 - 7.29 (m, 1H), 3.57 (s, 3H); $^{13}$C NMR (125 MHz, DMSO-$d_6$): δ 175.48, 165.07, 155.80, 150.60, 148.64, 139.17, 134.04, 131.61, 130.72, 126.20, 125.47, 124.32, 121.15, 117.95, 117.81, 112.15, 38.50.

**<Example 26> 1-methyl-2-(3,5-difluorophenyl)-3-carboxy-6-chloro-4-(1*H*)-quinolone**

The title compound was prepared using the preparation method described in Synthesis 2. Specifically, 2-(3,5-difluorophenyl)-3-methoxycarbonyl-6-chloro-4-(1*H*)-quinolone (103.7 mg, 0.3 mmol) and NaH (17.8 mg, 0.44 mmol) were stirred in DMF (2 mL) for 30 min. After iodomethane (0.037 mL, 0.59 mmol) was added dropwise, the mixture was stirred for 5 h, and further stirred in MeOH (1.5 mL) and 1 N NaOH (1.5 mL) for 14 h. When the reaction was completed, the solvent was removed by distillation under reduced pressure, and 1 N HCl aqueous solution was added dropwise to adjust the pH to 4-5, and the resulting solid was filtered with water to obtain 35.6 mg (33.9%) of the final target compound.

$^1$H NMR (500 MHz, DMSO-$d_6$): δ 15.03 (s, 1H), 8.35 (d, *J* = 2.3 Hz, 1H), 8.13 (d, *J* = 9.2 Hz, 1H), 8.05 (dd, *J* = 9.2, 2.3 Hz, 1H), 7.49 (tt, *J* = 9.5, 2.2 Hz, 1H), 7.30 - 7.27 (m, 2H), 3.60 (s, 3H); $^{13}$C NMR (125 MHz, DMSO-$d_6$): δ 175.68, 164.85, 162.25, 155.67, 139.13, 137.59, 134.18, 130.89, 126.15, 124.35, 121.15, 111.83, 111.47, 105.00, 38.55.

**<Example 27> 1-methyl-2-(2,6-difluorophenyl)-3-carboxy-6-chloro-4-(1*H*)-quinolone**

The title compound was prepared using the preparation method described in Synthesis 2. Specifically, 2-(2,6-difluorophenyl)-3-methoxycarbonyl-6-chloro-4-(1*H*)-quinolone (90 mg, 0.26 mmol) and NaH (15.4 mg, 0.39 mmol) were

stirred in DMF (2 mL) for 30 min. After iodomethane (0.032 mL, 0.51 mmol) was added dropwise, the mixture was stirred for 5 h, and further stirred in MeOH (2 mL) and 1 N NaOH (2 mL) for 24 h. When the reaction was completed, the solvent was removed by distillation under reduced pressure, and 1 N HCl aqueous solution was added dropwise to adjust the pH to 4-5, and the resulting solid was filtered with water to obtain 30.2 mg (33.2%) of the final target compound.

$^1$H NMR (500 MHz, $CDCl_3$): δ 15.61 (s, 1H), 8.62 (d, J = 2.5 Hz, 1H), 7.85 (dd, J = 9.1, 2.5 Hz, 1H), 7.72 (d, J = 9.1 Hz, 1H), 7.59 - 7.53 (m, 1H), 7.12 - 7.08 (m, 2H), 3.77 (s, 3H); $^{13}$C NMR (125 MHz, $CDCl_3$) δ 177.98, 164.68, 158.85, 149.73, 139.06, 134.86, 133.17, 132.77, 126.90, 126.65, 118.73, 111.98, 110.91, 37.42.

**<Example 28> 1-methyl-2-(2,5-difluorophenyl)-3-carboxy-8-chloro-4-(1*H*)-quinolone**

The title compound was prepared using the preparation method described in Synthesis 2. Specifically, 2-(2,5-difluorophenyl)-3-methoxycarbonyl-8-chloro-4-(1*H*)-quinolone (100 mg, 0.29 mmol) and NaH (17.2 mg, 0.43 mmol) were stirred in DMF (2 mL) for 30 min. After iodomethane (0.036 mL, 0.43 mmol) was added dropwise, the mixture was stirred for 5 h, and further stirred in MeOH (1 mL) and 1 N NaOH (1 mL) for 15 h. When the reaction was completed, the solvent was removed by distillation under reduced pressure, and 1 N HCl aqueous solution was added dropwise to adjust the pH to 4-5, and the resulting solid was filtered with water to obtain 11.6 mg (11.6%) of the final target compound.

$^1$H NMR (500 MHz, DMSO-$d_6$): δ 14.51 (s, 1H), 8.33 (dd, *J* = 7.9, 1.5 Hz, 1H), 8.10 (dd, *J* = 7.9, 1.5 Hz, 1H), 7.64 (t, *J* = 7.9 Hz, 1H), 7.54 - 7.49 (m, 3H), 4.15 (s, 3H); $^{13}$C NMR (125 MHz, DMSO-$d_6$): δ 176.58, 164.60, 158.09, 154.71, 153.98, 140.23, 137.11, 128.76, 126.94, 125.00, 123.38, 122.91, 119.29, 117.67, 117.23, 113.11, 44.47.

**<Example 29> 1-methyl-2-(2,5-difluorophenyl)-3-carboxy-7-bromo-4-(1*H*)-quinolone**

The title compound was prepared using the preparation method described in Synthesis 2. Specifically, 2-(2,5-difluorophenyl)-3-methoxycarbonyl-7-bromo-4-(1*H*)-quinolone (100 mg, 0.25 mmol) and NaH (15.2 mg, 0.38 mmol) were stirred in DMF (3.5 mL) for 30 min. After iodomethane (0.031 mL, 0.5 mmol) was added dropwise, the mixture was stirred for 5 h, and further stirred in MeOH (1.5 mL) and 1 N NaOH (1.5 mL) for 19 h. When the reaction was completed, the solvent was removed by distillation under reduced pressure, and 1 N HCl aqueous solution was added dropwise to adjust the pH to 4-5, and the resulting solid was filtered with water to obtain 27.0 mg (27.0%) of the final target compound.

$^1$H NMR (500 MHz, DMSO-$d_6$): δ 15.20 (s, 1H), 8.36 (s, 1H), 8.34 (s, 1H), 7.90 (d, *J* = 8.7 Hz, 1H), 7.50 (t, *J* = 5.7 Hz, 2H), 7.44 - 7.41 (m, 1H), 3.67 (s, 3H); $^{13}$C NMR (125 MHz, DMSO-$d_6$): δ 176.60, 164.60, 158.07, 154.52, 151.96, 141.28, 129.54, 128.61, 127.69, 123.97, 123.42, 121.29, 118.66, 117.35, 116.50, 111.71, 38.13.

**<Example 30> 1-methyl-2-(3,5-difluorophenyl)-3-carboxy-7-bromo-4-(1*H*)-quinolone**

The title compound was prepared using the preparation method described in Synthesis 2. Specifically, 2-(3,5-difluorophenyl)-3-methoxycarbonyl-7-bromo-4-(1*H*)-quinolone (100 mg, 0.25 mmol) and NaH (15.2 mg, 0.38) mmol) were stirred in DMF (3.5 mL) for 30 min. After iodomethane (0.031 mL, 0.5 mmol) was added dropwise, the mixture was stirred for 3 h, and further stirred in MeOH (2 mL) and 1 N NaOH (2 mL) for 19 h. When the reaction was completed, the solvent was removed by distillation under reduced pressure, and 1 N HCl aqueous solution was added dropwise to adjust the pH to 4-5, and the resulting solid was filtered with water to obtain 38.3 mg (38.3%) of the final target compound.

$^1$H NMR (500 MHz, DMSO-$d_6$): δ 15.14 (s, 1H), 8.35 (s, 1H), 8.33 (s, 1H), 7.83 (dd, *J* = 9.3, 1.4 Hz, 1H), 7.48 (t, *J* = 9.6 Hz, 1H), 7.26 (d, *J* = 5.9 Hz, 2H), 3.58 (s, 3H); $^{13}$C NMR (125 MHz, DMSO-$d_6$): δ 176.54, 164.78, 162.29, 156.07, 141.26, 137.67, 129.24, 128.44, 127.63, 123.94, 121.06, 111.77, 111.35, 104.95, 38.51.

**<Example 31> 1-methyl-2-(2,6-difluorophenyl)-3-carboxy-7-bromo-4-(1*H*)-quinolone**

The title compound was prepared using the preparation method described in Synthesis 2. Specifically, 2-(2,6-difluorophenyl)-3-methoxycarbonyl-7-bromo-4-(1*H*)-quinolone (75 mg, 0.19 mmol) and NaH (11.4 mg, 0.28 mmol) were stirred in DMF (3 mL) for 30 min. After iodomethane (0.024 mL, 0.38 mmol) was added dropwise, the mixture was stirred for 5 h, and further stirred in MeOH (2 mL) and 1 N NaOH (2 mL) for 19 h. When the reaction was completed, the solvent was removed by distillation under reduced pressure, and 1 N HCl aqueous solution was added dropwise to adjust the pH to 4-5, and the resulting solid was filtered with water to obtain 18.6 mg (24.8%) of the final target compound.

$^1$H NMR (500 MHz, DMSO-$d_6$): δ 15.21 (s, 1H), 8.37 (d, *J* = 8.6 Hz, 1H), 8.35 (d, *J* = 1.6 Hz, 1H), 7.91 (dd, *J* = 8.6, 1.6 Hz, 1H), 7.77 - 7.71 (m, 1H), 7.36 (t, *J* = 8.1 Hz, 2H), 3.76 (s, 3H); $^{13}$C NMR (125 MHz, DMSO-$d_6$): δ 176.57, 164.43, 158.35, 147.59, 141.33, 133.40, 129.81, 128.77, 127.75, 124.00, 121.52, 112.14, 111.23, 37.75.

**<Synthesis 3: Preparation of chromenone derivative compounds of Examples 32 to 39>**

Based on Scheme 3, the chromenone derivative compounds of Examples 32 to 39 were prepared. After dissolving chalcone compound (1.0 eq) in methanol (MeOH) or ethanol (EtOH), 5 M NaOH or KOH aqueous solution (2.0 eq) and $H_2O_2$ 30% aqueous solution (3.0 eq) were added and stirred for an appropriate time. Upon completion of the reaction, 1 N HCl aqueous solution was slowly added dropwise to the reactant while the pH was appropriately adjusted in the range of 1 to 5, followed by further stirring. The resulting solid was filtered to obtain the chromenone derivative compound 3-b of Examples 32 to 39.

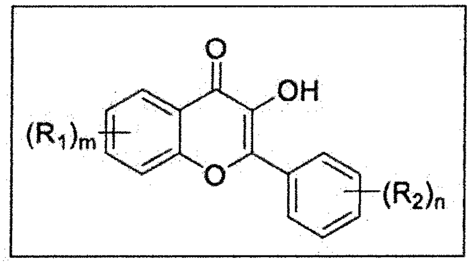

| | | |
|---|---|---|
| **32,** | $(R_1)_m$ = 7-chloro | $(R_2)_n$ = 2,5-difluoro |
| **33,** | $(R_1)_m$ = 7-chloro | $(R_2)_n$ = 3,5-difluoro |
| **34,** | $(R_1)_m$ = 7-chloro | $(R_2)_n$ = 3,4-difluoro |
| **35,** | $(R_1)_m$ = 7-fluoro | $(R_2)_n$ = 2,5-difluoro |
| **36,** | $(R_1)_m$ = 7-fluoro | $(R_2)_n$ = 3,5-difluoro |
| **37,** | $(R_1)_m$ = 7-fluoro | $(R_2)_n$ = 2,3-difluoro |
| **38,** | $(R_1)_m$=7-methoxy | $(R_2)_n$ = 2,5-difluoro |
| **39,** | $(R_1)_m$=7-methoxy | $(R_2)_n$= 3,5-difluoro |

**<Example 32> 7-chloro-2-(2,5-difluorophenyl)-3-hydroxy-4*H*-chromen-4-one**

The title compound was prepared using the preparation method described in Synthesis 3. Specifically, (*E*)-1-(4-chloro-2-hydroxyphenyl)-3-(2,5-difluorophenyl)prop-2-en-1-one (780 mg, 2.65 mmol ) was dissolved in EtOH, and 5 M NaOH (1.1 mL, 5.30 mmol) and $H_2O_2$ 30% aqueous solution (0.66 mL, 5.83 mmol) were added at 0 °C and stirred for 19 hours. Upon completion of the reaction, 1 N HCl aqueous solution was slowly added dropwise to the reaction mixture at 0 °C while adjusting the pH to 1, followed by stirring for 1 hour. The resulting solid was filtered to obtain 402.3 mg (49.0%) of the final product.

$^1H$ NMR (500 MHz, $CDCl_3$): δ 8.21 (d, *J* = 8.7 Hz, 1H), 7.58 (d, *J* = 1.8 Hz, 1H), 7.54-7.51 (m, 1H), 7.42 (dd, *J* = 1.8, 8.7 Hz, 1H), 7.22-7.19 (m, 2H), 6.67 (bs, 1H); $^{13}C$ NMR (125 MHz, DMSO-$d_6$): δ 172.24, 158.58, 156.66, 156.37, 155.07, 154.39, 142.19, 139.97, 138.31, 126.86, 125.39, 120.70, 120.03, 119.96, 119.90, 119.82, 119.26, 119.19, 119.06, 118.99, 118.31, 118.09, 118.02, 117.89, 117.82.

**<Example 33> 7-chloro-2-(3,5-difluorophenyl)-3-hydroxy-4H chromen-4-one**

The title compound was prepared using the preparation method described in Synthesis 3. Specifically, (*E*)-1-(4-chloro-2-hydroxyphenyl)-3-(3,5-difluorophenyl)prop-2-en-1-one (270 mg, 0.92 mmol) was dissolved in MeOH and then KOH (308 mg, 5.52 mmol) was added. At 60 °C, 30% aqueous solution of $H_2O_2$ (0.52 mL, 4.60 mmol) was slowly added dropwise to the reaction mixture for 10 minutes, and the mixture was stirred for 1 hour, while cooling from 60 °C to room temperature. Upon completion of the reaction, after cooling to 0 °C, 1 N HCl aqueous solution was slowly added dropwise to adjust the pH to 2-3 and stirred. The resulting solid was filtered under reduced pressure and washed with $H_2O$ to obtain 89.6 mg (32.0%) of the final product.

$^1H$ NMR (500 MHz, DMSO-$d_6$): δ 10.37 (s, 1H), 8.04 (d, *J* = 8.6 Hz, 1H), 8.00 (d, *J* = 1.2 Hz, 1H), 7.89 (d, *J* = 7.3 Hz, 2H), 7.47 (dd, *J* = 1.7, 8.6 Hz, 1H), 7.40 - 7.37 (m, 1H); $^{13}C$ NMR (125 MHz, DMSO-$d_6$): δ 172.51, 163.30, 163.20, 161.36, 161.25, 154.44, 142.10, 140.33, 138.35, 134.16, 126.57, 125.20, 119.20, 118.44, 110.48, 110.25, 105.33, 105.12, 104.92.

**<Example 34> 7-chloro-2- (3,4-difluorophenyl) -3-hydroxy-4H-chromen-4-one**

The title compound was prepared using the preparation method described in Synthesis 3. Specifically, (*E*)-1-(4-chloro-2-hydroxyphenyl)-3-(3,4-difluorophenyl)prop-2-en-1-one (700 mg, 2.38 mmol) was dissolved in MeOH, and KOH (800 mg, 14.3 mmol) was added. At 60 °C, 30% aqueous solution of $H_2O_2$ (1.35 mL, 11.9 mmol) was slowly added dropwise to the reaction mixture for 10 minutes, and the mixture was stirred for 1 hour, while cooling from 60 °C to room temperature. Upon completion of the reaction, after cooling to 0 °C, 1 N HCl aqueous solution is slowly added dropwise to adjust the pH to 2-3 and stirred. The resulting solid was filtered under reduced pressure and washed with $H_2O$ to obtain 466 mg (64.0%) of the final product.

$^1H$ NMR (500 MHz, DMSO-$d_6$): δ 9.93 (s, 1H), 8.12 (d, *J* = 5.5 Hz, 2H), 8.10 (d, *J* = 5.9 Hz, 1H), 8.06 (d, *J* = 1.9 Hz, 1H), 7.52 (dd, *J* = 1.9, 8.7 Hz, 1H), 7.39 (t, *J* = 8.7 Hz, 1H); $^{13}C$ NMR (125 MHz, DMSO-$d_6$): δ 172.17, 154.53, 151.97, 150.04, 148.46, 148.37, 144.30, 138.88, 137.92, 126.61, 125.13, 124.85, 123.68, 120.19, 118.38, 114.96, 114.80, 113.72.

**<Example 35> 2-(2,5-difluorophenyl)-7-fluoro-3-hydroxy-4*H*-chromen-4-one**

The title compound was prepared using the preparation method described in Synthesis 3. Specifically, (*E*)-3-(2,5-difluorophenyl)-1-(4-fluoro-2-hydroxyphenyl)prop-2-en-1-one (200 mg, 0.72 mmol) was dissolved in EtOH (9 ml). 5 M aqueous NaOH solution (0.3 mL, 1.44 mmol) and 30% aqueous $H_2O_2$ solution (0.18 mL, 1.58 mmol) were added to the reaction mixture at 0 °C, followed by stirring at room temperature for 17 hours. Upon completion of the reaction, 1 N HCl aqueous solution was slowly added dropwise to the reaction mixture to adjust the pH to 5, followed by stirring for 30 minutes. The resulting solid was filtered to obtain 41.6 mg (20.0%) of the final product.

$^1H$ NMR (500 MHz, $CDCl_3$): δ 8.29 (dd, *J* = 6.2, 8.9 Hz, 1H), 7.54 - 7.26 (m, 1H), 7.24 (d, *J* = 2.3 Hz, 1H), 7.22 (d, *J* = 2.3 Hz, 1H), 7.22 - 7.21 (m, 1H), 7.20 - 7.18 (m, 1H), 6.67 (bs, 1H).

**<Example 36> 2-(3,5-difluorophenyl)-7-fluoro-3-hydroxy-4*H*-chromen-4-one**

The title compound was prepared using the preparation method described in Synthesis 3. Specifically, (*E*)-3-(3,5-difluorophenyl)-1-(4-fluoro-2-hydroxyphenyl)prop-2-en-1-one (300 mg, 1.08 mmol) was dissolved in MeOH, and then KOH (363 mg, 6.48 mmol) was added. At 60 °C, 30% $H_2O_2$ aqueous solution (0.61 mL, 5.40 mmol) was slowly added dropwise to the reaction mixture for 20 minutes, and stirred for 1 hour while cooling from 60 °C to room temperature. Upon completion of the reaction, after cooling to 0 °C, 1 N HCl aqueous solution is slowly added dropwise to adjust the pH to 2-3 and stirred. The resulting solid was filtered under reduced pressure and washed with $H_2O$ to obtain 103 mg (33.0%) of the final product.

$^1H$ NMR (500 MHz, DMSO-$d_6$): δ 10.36 (bs, 1H), 8.16 (dd, *J* = 6.4, 8.9 Hz, 1H), 7.94 (d, *J* = 7.2 Hz, 2H), 7.80 (dd, *J* = 2.3, 9.8 Hz, 1H), 7.46 - 7.40 (m, 1H), 7.37 (ddd, *J* = 2.3, 8.7 Hz, 1H); $^{13}C$ NMR (125 MHz, DMSO-$d_6$): δ 172.53, 165.99, 163.99, 163.36, 163.26, 161.41, 161.31, 155.52, 155.41, 142.41, 140.113, 134.36, 134.28, 134.19, 127.74, 127.65, 118.39, 113.83, 113.65, 110.47, 110.42, 110.30, 110.25, 105.36, 105.20, 105.15, 105.00.

**<Example 37> 2-(2,3-difluorophenyl)-7-fluoro-3-hydroxy-4*H*-chromen-4-one**

The title compound was prepared using the preparation method described in Synthesis 3. Specifically, (*E*)-3-(2,3-difluorophenyl)-1-(4-fluoro-2-hydroxyphenyl)prop-2-en-1-one (700 mg, 2.5 mmol) was dissolved in MeOH and then KOH (847 mg, 15.1 mmol) was added. At 60 °C, 30% aqueous solution of $H_2O_2$ (1.43 mL, 12.6 mmol) was slowly added dropwise over 10 minutes, and stirred for 1 hour while cooling from 60 °C to room temperature. After completion of the reaction, after cooling to 0 °C, 1 N HCl aqueous solution is slowly added dropwise to adjust the pH to 2-3 and stirred. The resulting solid was filtered under reduced pressure and washed with $H_2O$ to obtain 390.4 mg (53.0%) of the final product.

$^1H$ NMR (500 MHz, DMSO-$d_6$): δ 9.43 (bs, 1H), 8.22 (dd, *J* = 6.5, 8.8 Hz, 1H), 7.65 (d, *J* = 9.6 Hz, 1H), 7.49 (dd, *J* = 8.3, 11.7 Hz, 1H), 7.42 - 7.37 (m, 2H), 7.28 - 7.24 (m, 1H); $^{13}C$ NMR (125 MHz, DMSO-$d_6$): δ 172.18, 165.78, 163.78, 156.11, 155.92, 155.80, 154.15, 145.56, 145.49, 145.40, 139.33, 127.87, 127.78, 126.67, 126.64, 125.84, 125.82, 123.96, 123.89, 119.14, 118.91, 118.76, 113.75, 113.56, 105.01, 104.80.

**<Example 38> 2-(2,5-difluorophenyl)-3-hydroxy-7-methoxy-4H-chromen-4-one**

The title compound was prepared using the preparation method described in Synthesis 3. Specifically, (*E*)-3-(2,5-difluorophenyl)-1-(2-hydroxy-4-methoxyphenyl)prop-2-en-1-one (124.3 mg, 0.43 mmol) was dissolved in MeOH, and then KOH (144 mg, 2.6 mmol) was added. At 60 °C, 30% aqueous solution of $H_2O_2$ (0.2 mL, 1.7 mmol) was slowly

added dropwise over 20 minutes, followed by stirring at 30 °C for 30 minutes. Upon completion of the reaction, 1 N HCl is slowly added dropwise to the reaction mixture to adjust the pH to 4-5, followed by stirring for 30 minutes. The resulting solid was washed with $H_2O$ and filtered under reduced pressure to obtain 25 mg (19.0%) of the final product.

$^1H$ NMR (500 MHz, $CDCl_3$): δ 8.16 (d, *J* = 9.0 Hz, 1H), 7.55 - 7.52 (m, 1H), 7.22 - 7.17 (m, 2H), 7.02 (dd, *J* = 2.3, 9.0 Hz, 1H), 6.91 (d, *J* = 2.3 Hz, 1H), 6.70 (bs, 1H), 3.93 (s, 3H).

**<Example 39> 2-(3,5-difluorophenyl)-3-hydroxy-7-methoxy-4*H*-chromen-4-one**

The title compound was prepared using the preparation method described in Synthesis 3. Specifically, (*E*)-3-(3,5-difluorophenyl)-1-(2-hydroxy-4-methoxyphenyl)prop-2-en-1-one (500 mg, 1.7 mmol) was dissolved in MeOH (9 mL), and then KOH (580 mg, 10.3 mmol) was added. At 60 °C, 30% aqueous solution of $H_2O_2$ (0.78 mL, 6.9 mmol) was slowly added dropwise, followed by stirring for 1 hour. Upon completion of the reaction, after cooling to 0 °C, 1 N HCl aqueous solution was added dropwise to adjust the pH to 3-4, followed by stirring for 1 hour. The resulting solid was filtered under reduced pressure to obtain 139.6 mg (27.0%) of the final product.

$^1H$ NMR (500 MHz, DMSO-$d_6$): δ 10.12 (bs, 1H), 7.97 (d, *J* = 8.9 Hz, 1H), 7.93 (d, *J* = 7.6 Hz, 2H), 7.42 - 7.38 (m, 2H), 7.03 (dd, *J* = 2.0, 8.9 Hz), 3.92 (s, 3H); $^{13}C$ NMR (125 MHz, DMSO-$d_6$): δ 172.42, 163.94, 163.52, 163.40, 163.29, 161.45, 161.34, 156.44, 139.93, 134.73, 134.64, 131.52, 126.12, 115.00, 114.96, 110.27, 110.21, 110.04, 105.04, 104.83, 104.63, 100.38, 56.10

**<Synthesis 4: Preparation of chromenone derivative compounds of Examples 40 to 47>**

Based on Scheme 4, the chromenone derivative compounds of Examples 40 to 47 were prepared. Compound 3-b (1.0 eq) prepared based on Scheme 3 was dissolved in acetone, and $K_2CO_3$ (1.5 eq) and MeI (1.2 eq) were added to the reaction mixture, followed by stirring at room temperature to 57 °C for an appropriate time. Upon completion of the reaction, the solvent was removed by distillation under reduced pressure, and the resulting solid was washed with $H_2O$, filtered under reduced pressure, or purified by column chromatography to obtain chromenone derivative compounds 4-a of Examples 40 to 47.

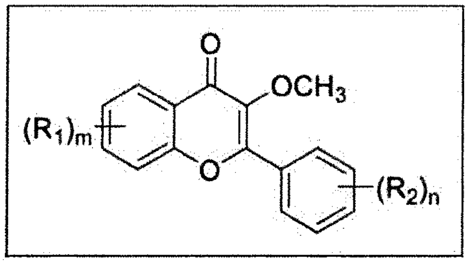

| | | |
|---|---|---|
| **40,** | $(R_1)_m$ = 7-chloro | $(R_2)_n$ = 2,5-difluoro |
| **41,** | $(R_1)_m$ = 7-chloro | $(R_2)_n$ = 3,5-difluoro |
| **42,** | $(R_1)_m$ = 7-fluoro | $(R_2)_n$ = 2,5-difluoro |
| **43,** | $(R_1)_m$ = 7-fluoro | $(R_2)_n$ = 2,4-difluoro |
| **44,** | $(R_1)_m$ = 7-fluoro | $(R_2)_n$ = 3,5-difluoro |
| **45,** | $(R_1)_m$ = 7-methoxy | $(R_2)_n$ = 2,5-difluoro |
| **46,** | $(R_1)_m$ = 7-methoxy | $(R_2)_n$ = 3,5-difluoro |
| **47,** | $(R_1)_m$ = 7-methoxy | $(R_2)_n$ = 2,4-difluoro |

**<Example 40> 7-chloro-2-(2,5-difluorophenyl)-3-methoxy-4*H*-chromen-4-one**

The title compound was prepared using the preparation method described in Synthesis 4. Specifically, the compound of Example 32 (149.7 mg, 0.48 mmol) was dissolved in acetone (3 mL), and $K_2CO_3$ (100.5 mg, 0.72 mmol) and MeI (0.06 mL, 0.96 mmol) were added to the reaction mixture and stirred at 57 °C for 7 hours. Then, THF (2 mL) and MeI (0.06 mL, 0.96 mmol) were further added, followed by stirring for 14 hours. When the reaction was completed, the solvent was removed by distillation under reduced pressure, and the resulting solid was washed with $H_2O$ and filtered under reduced pressure to obtain 146.1 mg (93.0%) of the final product.

$^1H$ NMR (500 MHz, $CDCl_3$): δ 8.22 (d, *J* = 8.6 Hz, 1H), 7.52 (s, 1H), 7.40 (d, *J* = 8.6 Hz, 1H), 7.36 - 7.34 (m, 1H), 7.22 - 7.20 (m, 2H), 3.95 (s, 3H); $^{13}C$ NMR (125 MHz, $CDCl_3$): δ 174.00, 155.56, 142.32, 139.91, 127.31, 125.90, 122.99, 119.27, 119.20, 119.08, 119.01, 118.17, 117.77, 117.70, 117.58, 117.51, 117.37, 117.17, 60.64.

**<Example 41> 7-chloro-2- (3,5-difluorophenyl) -3-methoxy-4*H*-chromen-4-one**

The title compound was prepared using the preparation method described in Synthesis 4. Specifically, the compound of Example 33 (161.4 mg, 0.52 mmol) was dissolved in acetone:THF = 1:1, and then $K_2CO_3$ (87 mg, 0.62 mmol) and MeI (0.098 mL, 1.56 mmol) were added and stirred at 57 °C. MeI (0.098 mL, 1.56 mmol) was added at 1 hour intervals until the reaction was completed. When the reaction was completed, the solvent was removed by distillation under reduced pressure, followed by extraction with EtOAc and $H_2O$. The organic layer was dried over anhydrous $MgSO_4$ and filtered under reduced pressure to remove the solvent. The resulting solid was washed with $H_2O$ and filtered under reduced pressure to obtain 89.5 mg (53.0%) of the final product.

$^1H$ NMR (500 MHz, $CDCl_3$): δ 8.20 (d, *J* = 8.6 Hz, 1H), 7.71 - 7.67 (m, *J* = 1.8, 8.5 Hz, 2H), 7.60 (d, *J* = 1.8 Hz, 1H), 7.40 (dd, *J* = 1.5, 8.6 Hz, 1H), 6.96 (tt, *J* = 2.2, 8.5 Hz, 1H), 3.97 (s, 3H); $^{13}C$ NMR (125 MHz, $CDCl_3$): δ 174.31, 163.97, 163.87, 162.00, 161.90, 155.08, 152.48, 142.34, 140.13, 133.45, 133.37, 127.28, 126.01, 122.59, 118.05, 111.63, 111.58, 111.46, 111.41, 106.49, 106.29, 106.09, 60.31.

**<Example 42> 2-(2,5-difluorophenyl)-7-fluoro-3-methoxy-4*H*-chromen-4-one**

The title compound was prepared using the preparation method described in Synthesis 4. Specifically, the compound of Example 35 (20 mg, 0.07 mmol) is dissolved in acetone, and then $K_2CO_3$ (14.2 mg, 0.047 mmol) and MeI (0.005 mL, 0.077 mmol) were added. The reaction mixture was stirred at room temperature for 2 hours and then at 57 °C for 15 hours. Upon completion of the reaction, the solvent was removed by distillation under reduced pressure after cooling to room temperature. The resulting solid was washed with $H_2O$ and filtered under reduced pressure to obtain 18.5 mg (89.0%) of the final product.

$^1H$ NMR (500 MHz, $CDCl_3$): δ 8.30 (dd, *J* = 6.3, 9.5 Hz, 1H), 7.37 - 7.33 (m, 1H), 7.27 - 7.21 (m, 2H), 7.20 - 7.15 (m, 2H), 3.94 (s, 3H); $^{13}C$ NMR (125 MHz, $CDCl_3$): δ 173.89, 166.72, 164.68, 159.17, 159.15, 157.23, 157.21, 157.00, 156.98, 156.51, 156.40, 155.01, 154.99, 151.24, 142.17, 128.52, 128.43, 121.31, 121.30, 121.30, 119.94, 119.87, 119.81, 119.74, 119.20, 119.13, 119.01, 118.94, 117.73, 117.66, 117.54, 117.47, 117.35, 117.33, 117.14, 117.12, 114.09, 113.91 104.79, 104.58, 60.63, 60.52.

**<Example 43> 2-(2,4-difluorophenyl)-7-fluoro-3-methoxy-4*H*-chromen-4-one**

The title compound was prepared using the preparation method described in Synthesis 4. Specifically, 2-(2,4-difluorophenyl)-7-fluoro-3-hydroxy-4*H*-chromen-4-one (200 mg, 0.68 mmol) was dissolved in acetone (3 mL). $K_2CO_3$ (236 mg, 1.7 mmol) and MeI (0.09 mL, 1.5 mmol) were added to the reaction solution, followed by stirring at 57 °C for 5 hours. Upon completion of the reaction, the solvent was removed by distillation under reduced pressure, followed by extraction with EtOAc and $H_2O$. The organic layer was dried over anhydrous $MgSO_4$ and filtered under reduced pressure to remove the solvent. Purification by column chromatography (n-hexane/EtOAc = 15:1) gave 79.2 mg (38.0%) of the final product.

$^1H$ NMR (500 MHz, $CDCl_3$): δ 8.29 (dd, *J* = 6.3, 9.5 Hz, 1H), 7.37 - 7.33 (m, 1H), 7.23 - 7.19 (m, 2H), 7.18 - 7.15 (m, 2H), 3.95 (s, 3H); $^{13}C$ NMR (125 MHz, $CDCl_3$): δ 173.89, 166.72, 164.69, 159.18, 159.17, 157.25, 157.23, 157.02, 157.00, 156.51, 156.41, 155.03, 155.01,151.25, 142.18, 128.52, 128.44, 121.33, 121.31,119.96, 119.89, 119.83, 119.76, 119.21, 119.14, 119.02, 118.95, 117.74, 117.68, 117.55, 117.48, 117.36, 117.34, 117.16, 117.14, 114.10, 113.91, 104.80, 104.59, 60.63.

**<Example 44> 2-(3,5-difluorophenyl)-7-fluoro-3-methoxy-4*H*-chromen-4-one**

The title compound was prepared using the preparation method described in Synthesis 4. Specifically, the compound of Example 36 (0.05 mg, 0.18 mmol) was dissolved in THF, and then $K_2CO_3$ (37 mg, 0.27 mmol) and MeI (0.017 mL, 0.27 mmol) were added and stirred at room temperature for 1 hour. Acetone and MeI (0.017 mL, 0.27 mmol) were further added to the reaction mixture, and the mixture was stirred at 65 °C for 2 hours. MeI (0.034 mL, 0.54 mmol) was further added to the reaction mixture, and the mixture was stirred for 1 hour and 30 minutes. When the reaction was completed, the solvent was removed by distillation under reduced pressure, followed by extraction with EtOAc and $H_2O$. The organic layer was dried over anhydrous $MgSO_4$ and filtered under reduced pressure to remove the solvent. Purification by column chromatography (n-hexane/EtOAc = 15:1) gave 11.6 mg (42.0%) of the final compound.

$^1H$ NMR (500 MHz, $CDCl_3$): δ 8.28 (dd, *J* = 6.3, 8.9 Hz, 1H), 7.69 (dd, *J* = 2.3, 8.6 Hz, 2H), 7.24 (dd, *J* = 2.3,

8.9 Hz, 1H), 7.17 (ddd, *J* = 1.7, 2.3, 8.5 Hz, 1H). 6.98 (tt, *J* = 2.3, 8.6 Hz, 1H), 3.97 (s, 3H); $^{13}C$ NMR (125 MHz, $CDCl_3$): δ 174.21, 166.88, 164.84, 163.97, 163.87, 161.99,161.89, 156.04, 155.93, 152.66, 142.19, 133.48, 133.40, 133.32, 128.53, 128.44, 120.92, 114.22, 114.03, 111.58, 111.53, 111.42, 111.36, 106.42, 106.22, 106.02, 104.72, 104.51, 60.32.

**<Example 45> 2-(2,5-difluorophenyl)-3,7-dimethoxy-4*H*-chromen-4-one**

The title compound was prepared using the preparation method described in Synthesis 4. Specifically, the compound of Example 38 (150 mg, 0.49 mmol) was dissolved in acetone. $K_2CO_3$ (136 mg, 0.98 mmol) and MeI (0.046 mL, 0.74 mmol) were added and stirred at 57 °C for one day. Upon completion of the reaction, the solvent was removed by distillation under reduced pressure, followed by extraction with EtOAc and $H_2O$. The organic layer was dried over anhydrous $MgSO_4$ and filtered under reduced pressure to remove the solvent. Purification by column chromatography (n-hexane/EtOAc = 8:1) gave 88 mg (56.0%) of the final product.

$^1H$ NMR (500 MHz, $CDCl_3$): δ 8.16 (d, *J* = 9.0 Hz, 1H), 7.37 - 7.33 (m, 1H), 7.21 - 7.18 (m, 2H), 6.98 (dd, *J* = 2.3, 9.0 Hz, 1H), 6.86 (2.3 Hz, 1H), 3.94 (s, 3H), 3.90 (s, 3H); $^{13}C$ NMR (125 MHz, $CDCl_3$): δ 174.10, 164.21, 159.17,159.15, 157.34, 157.23, 157.221, 157.00, 156.98, 155.02, 155.00, 150.42, 142.04, 127.21, 120.39, 120.32, 120.25, 120.18, 118.89, 118.82, 118.70, 118.63, 118.32, 117.64, 117.58, 117.45, 117.38, 117.19, 117.17, 114.81, 99.89, 60.60, 55.88.

**<Example 46> 2-(3,5-difluorophenyl)-3,7-dimethoxy-4*H*-chromen-4-one**

The title compound was prepared using the preparation method described in Synthesis 4. Specifically, 2-(3,5-difluorophenyl)-3-hydroxy-7-methoxy-4*H*-chromen-4-one (150 mg, 0.49 mmol) was dissolved in acetone (3 mL). $K_2CO_3$ (170 mg, 1.2 mmol) and MeI (0.07 mL, 1.1 mmol) were added and stirred at 57 °C for one day. Upon completion of the reaction, the solvent was removed by distillation under reduced pressure, followed by extraction with EtOAc and $H_2O$. The organic layer was dried over anhydrous $MgSO_4$ and filtered under reduced pressure to remove the solvent. Purification by column chromatography (n-hexane/EtOAc = 8:1) gave 29.1 mg (19.0%) of the final product.

1H NMR (500 MHz, CDCl3): δ 8.15 (d, J = 8.9 Hz, 1H), 7.72 - 7.68 (m, 2H), 7.00 (dd, J = 2.3, 9.9 Hz, 1H), 6.94 (tt, J = 2.3, 8.6 Hz, 1H), 6.92 (d, J = 2.2 Hz, 1H), 3.96 (s, 3H), 3.94 (s, 3H); 13C NMR (125 MHz, $CDCl_3$): δ 174.38, 164.38, 163.92, 163.82, 161.95, 161.85, 156.83, 151.83, 151.81, 151.78, 142.10, 133.95, 133.87, 133.78, 127.22, 117.95, 114.87, 111.44, 111.39, 111.27, 111.22, 106.01, 105.81, 105.61, 99.78, 60.24, 55.92.

**<Example 47> 2-(2,4-difluorophenyl)-3,7-dimethoxy-4*H*-chromen-4-one**

The title compound was prepared using the preparation method described in Synthesis 4. Specifically, 2-(2,4-difluorophenyl)-3-hydroxy-7-methoxy-4*H*-chromen-4-one (150 mg, 0.49 mmol) was dissolved in acetone (3 mL). Then, $K_2CO_3$ (170 mg, 1.2 mmol) and MeI (0.07 mL, 1.1 mmol) were added, and the mixture was stirred at 57 °C for 5 hours. Upon completion of the reaction, the solvent was removed by distillation under reduced pressure, followed by extraction with EtOAc and $H_2O$. Purification by column chromatography (n-hexane/EtOAc = 15:1) gave 29.1 mg (19.0%) of the final product.

$^1H$ NMR (500 MHz, $CDCl_3$): δ 8.17 (d, *J* = 9.0 Hz, 1H), 7.37 - 7.33 (m, 1H), 7.21 - 7.18 (m, 2H), 7.00 (dd, *J* = 2.4, 9.0 Hz, 1H), 6.86 (d, *J* = 2.4 Hz, 1H), 3.94 (s, 3H), 3.91 (s, 3H); $^{13}C$ NMR (125 MHz, $CDCl_3$): δ 174.13, 164.22, 159.18, 159.16, 157.35, 157.24, 157.00, 155.01, 150.43, 142.06, 127.25, 120.40, 120.33, 120.26, 120.20, 118.89, 118.82, 118.70, 118.63, 118.34, 117.65, 117.59, 117.46, 117.41, 117.39, 117.21, 117.19, 114.83, 99.90, 60.62, 55.89.

**Preparation of dimer compounds**

**<Example 48> 2,2'-((ethane-1,2-diylbis(oxy))bis(2-fluoro-4,1-phenylene))bis(7-chloro-4-oxo-4*H*-chromene-3-carboxylic acid)**

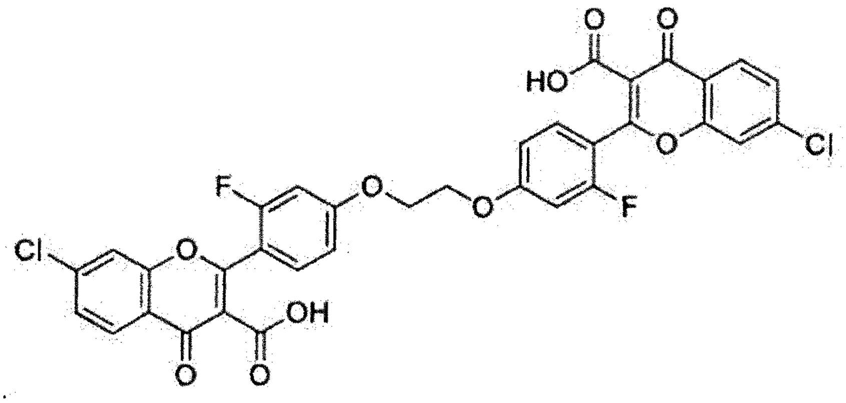

**<Reaction 1> Synthesis of tert-butyl 7-chloro-2-(2-fluoro-4-hydroxyphenyl)-4-oxo-4H-chromene-3-carboxylate**

Tert-butyl 2-(4-acetoxy-2-fluorophenyl)-7-chloro-4-oxo-4*H*-chromene-3-carboxylate (1.6 g, 3.7 mmol, 1.0 eq) was dissolved in dimethylamine solution (2.0 M THF solution, 10 mL, 20 mmol, 5.5 eq) and the solution was stirred at room temperature for 1 hour. Upon completion of the reaction, the mixture was distilled under reduced pressure and extracted with ethyl acetate and 1N HCl aqueous solution. The organic layer was dried over anhydrous $MgSO_4$, and the impurities were purified by column chromatography to obtain 1.03 g (71.8%) of tert-butyl 7-chloro-2-(2-fluoro-4-hydroxyphenyl)-4-oxo-4H chromene-3-carboxylate.

$^1$H NMR (500 MHz, $CDCl_3$): δ 8.18 (d, *J* = 8.6 Hz, 1H), 7.93 (bs, 1H), 7.52 (d, *J* = 1.9 Hz, 1H), 7.42 (dd, *J* = 8.6, 1.9 Hz, 1H), 7.40 (t, *J* = 8.3 Hz, 1H), 1.50 (s, H); $^{13}$C NMR (125 MHz, $CDCl_3$): δ 174.21, 164.27, 160.83, 161.09, 161.00, 160.49, 156.05, 140.44, 131.26, 127.45, 126.59, 122.03, 120.54, 118.23, 111.82, 103.94, 83.71, 27.92.

**<Reaction 2> Synthesis of Di-tert-Butyl 2,2'-((ethane-1,2-diylbis(oxy))bis(2-fluoro-4,1-phenylene))bis(7-chloro-4-oxo-4*H*-chromene-3-carboxylate)**

Tert-butyl 7-chloro-2-(2-fluoro-4-hydroxyphenyl)-4-oxo-4H-chromene-3-carboxylate (148 mg, 0.38 mmol, 1 eq), $K_2CO_3$ (78.5 mg, 0.57 mmol, 1.5 eq) and ethylene glycol di-p-tosylate (64 mg, 0.17 mmol, 0.46 eq) were dissolved in DMF and the mixture was stirred at 60 °C for 12 hours. Upon completion of the reaction, the solvent was removed by distillation under reduced pressure, followed by extraction with ethyl acetate and distilled water. The organic layer was dried over anhydrous $MgSO_4$, and impurities were purified by column chromatography to obtain 252 mg (90.2%) of di-tert-butyl 2,2'-((ethane-1,2-diylbis(oxy))bis(2-fluoro-4,1-phenylene))bis(7-chloro-4-oxo-4*H*-chromene-3-carboxylate).

$^1$H NMR (500 MHz, $CDCl_3$): δ 8.19 (d, *J* = 8.6 Hz, 1H), 7.58 (t, 8.3 Hz, 1H), 7.50 (s, 1H), 7.41 (d, *J* = 8.6 Hz, 1H), 6.87 (d, *J* = 8.9 Hz, 1H), 6.82 (d, *J* = 11.7 Hz, 1H), 4.43 (s, 1H), 1.44 (s, 9H); $^{13}$C NMR (125 MHz, $CDCl_3$): δ 174.04, 162.78, 160.46, 160.4, 159.84, 156.01, 140.30, 131.40, 127.46, 126.49, 122.09, 120.77, 118.19, 112.04, 111.70, 103.88, 83.32, 27.87, 14.19.

**<Reaction 3> Synthesis of 2,2'-((ethane-1,2-diylbis(oxy))bis(2-fluoro-4,1-phenylene))bis(7-chloro-4-oxo-4*H*-chromene-3-carboxylic acid)**

Di-tert-butyl 2,2'-((ethane-1,2-diylbis(oxy))bis(2-fluoro-4,1-phenylene))bis(7-chloro-4-oxo-4*H*-chromene-3-carboxylate) (186.5 mg, 0.23 mmol, 1.0 eq) was dissolved in dichloromethane, and trifluoroacetic acid (0.46 mL, 6.0 mmol, 26.0 eq) and triethylsilane (0.19 mL, 15.0 mmol, 64.0 eq) were added and the mixture was stirred at room temperature for 30 minutes. Upon completion of the reaction, the impurities were removed by diethyl ether (trituration) at 0 °C after distillation under reduced pressure. The resulting solid was dissolved in distilled water and triethylamine (35 mg, 0.35 mmol, 1.5 eq) was added dropwise, followed by stirring at room temperature for 1 hour. At 0 °C, 1 N HCl solution was slowly added dropwise to adjust the pH of the solution to 1-2, and the resulting solid was filtered with distilled water to obtain 97.5 mg (60.7%) of the compound of Example 48.

$^1$H NMR (500 MHz, DMSO-$d_6$) δ 13.35 (s, 1H), 8.12 (d, *J* = 8.6 Hz, 1H), 7.95 (d, *J* = 1.9 Hz, 1H), 7.69 (t, *J* = 8.6 Hz, 1H), 7.63 (dd, *J* = 8.6, 1.9 Hz, 1H), 7.17 (dd, *J* = 12.4, 2.2 Hz, 1H), 7.08 (dd, *J* = 8.7, 2.2 Hz, 1H), 4.51 (s, 2H); $^{13}$C NMR (125 MHz, DMSO-$d_6$) δ 174.00, 165.04, 162.86, 160.75, 159.77, 156.17, 139.83, 132.03, 127.56, 127.17, 121.93, 120.70, 119.03, 112.50, 111.94, 103.20, 67.53.

**<Example 49> 2,2'-((propane-1,3-diylbis(oxy))bis(2-fluoro-4,1-phenylene))bis(7-chloro-4-oxo-4*H*-chromene-3-carboxylic acid)**

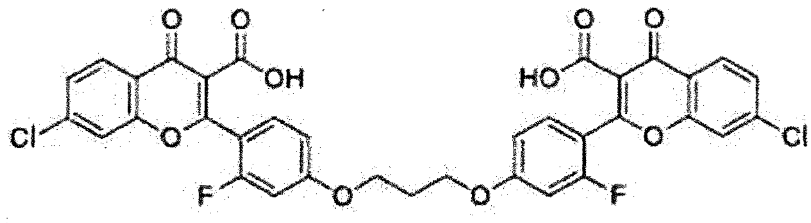

**<Reaction 1> Synthesis of di-tert-Butyl 2,2'-((propane-1,3-diylbis(oxy))bis(2-fluoro-4,1-phenylene))bis(7-chloro-4-oxo-4*H* -chromene-3-carboxylate)**

Tert-butyl 7-chloro-2-(2-fluoro-4-hydroxyphenyl)-4-oxo-4*H*-chromene-3-carboxylate (200 mg, 0.51 mmol, 1.0 eq), $K_2CO_3$ (105 mg, 0.77 mmol, 1.5 eq) and 1,3-propanediol di-p-tosylate (83 mg, 0.22 mmol, 0.4 eq) were dissolved in DMF and stirred at 60 °C. for 12 hours. Upon completion of the reaction, the solvent was removed by distillation under reduced pressure, followed by extraction with ethyl acetate and distilled water. The organic layer was dried over anhydrous $MgSO_4$, and the impurities were purified by column chromatography to obtain 194.5 mg (92.5%) of di-tert-butyl 2,2'-((propane-1,3-diylbis(oxy))bis(2-fluoro-4,1)-phenylene))bis(7-chloro-4-oxo-4*H*-chromene-3-carboxylate).

$^1H$ NMR (500 MHz, $CDCl_3$): $\delta$ 8.18 (d, *J* = 8.7 Hz, 1H), 7.55 (t, *J* = 8.4 Hz, 1H), 7.49 (d, *J* = 1.9 Hz, 1H), 7.40 (dd, *J* = 8.6, 1.9 Hz, 1H), 6.82 (dd, *J* = 8.7, 2.3 Hz, 1H), 6.77 (dd, *J* = 11.9, 2.3 Hz, 1H), 4.24 (t, *J* = 6.0 Hz, 2H), 2.40-2.32 (m, 1H), 1.57 (s, 1H), 1.43 (s, 9H); $^{13}C$ NMR (125 MHz, $CDCl_3$): $\delta$ 173.93, 163.01, 162.68, 160.91, 159.74, 155.99, 140.21, 131.36, 127.51, 126.44, 122.17, 121.07, 118.14, 112.76, 110.63, 102.70, 82.84, 64.87, 28.83, 27.88.

**<Reaction 2> Synthesis of 2,2'-((propane-1,3-diylbis(oxy))bis(2-fluoro-4,1-phenylene))bis(7-chloro-4-oxo-4*H*-chromene-3-carboxylic acid)**

Di-tert-butyl 2,2'-((propane-1,3-diylbis(oxy))bis(2-fluoro-4,1-phenylene))bis(7-chloro-4-oxo-4*H*-chromene-3-carboxylate) (195 mg, 0.28 mmol, 1.0 eq) was dissolved in dichloromethane, and trifluoroacetic acid (0.46 mL, 6 mmol, 21.0 eq) and triethylsilane (0.185 mL, 1.16 mmol, 4.3 eq) were added and the mixture was stirred at room temperature for 30 minutes. When the reaction was completed, the impurities were removed by diethyl ether (trituration) at 0 °C after distillation under reduced pressure. The resulting solid was dissolved in distilled water and triethylamine (35 mg, 0.35 mmol, 1.5 eq) was added dropwise, followed by stirring at room temperature for 1 hour. At 0 °C, 1 N HCl solution was slowly added dropwise to adjust the pH of the solution to 1-2, and the resulting solid was filtered with distilled water to obtain 52.7 mg (31.5%) of the compound of Example 49.

$^1H$ NMR (500 MHz, DMSO-$d_6$) $\delta$ 13.36 (bs, 1H), 8.11 (d, *J* = 8.6 Hz, 1H), 7.94 (d, *J* = 1.9 Hz, 1H), 7.67 (t, *J* = 8.6 Hz, 1H), 7.63 (dd, *J* = 8.6, 1.9 Hz, 1H), 7.12 (dd, *J*= 12.5, 2.3 Hz, 1H), 7.04 (dd, *J* = 8.7, 2.3 Hz, 1H), 4.29 (t, *J* = 6.2 Hz, 2H), 2.29-2.25 (m, 1H); $^{13}C$ NMR (125 MHz, DMSO-$d_6$) $\delta$ 173.99, 165.03, 163.06, 162.46, 159.79, 156.15, 139.81, 131.98, 127.55, 127.15, 121.92, 120.68, 119.00, 112.30, 111.86, 103.11, 65.74, 28.62.

**<Example 50> 2,2'-((butane-1,4-diylbis(oxy))bis(2-fluoro-4,1-phenylene))bis(7-chloro-4-oxo-4*H*-chromene-3-carboxylic acid)**

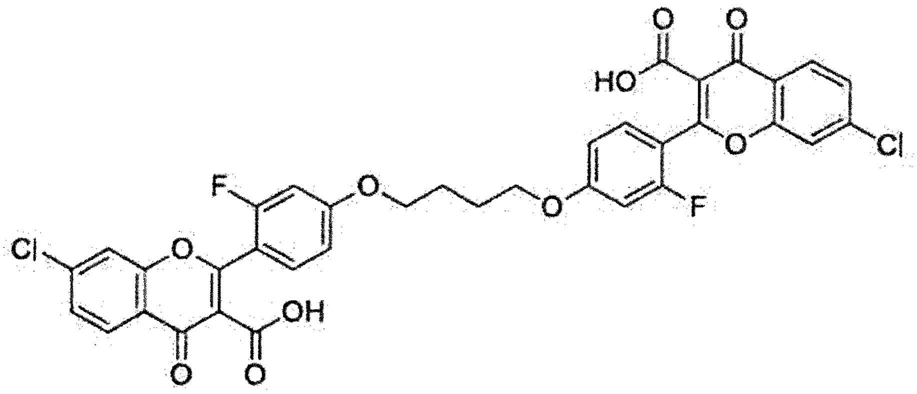

**<Reaction 1> Synthesis of di-tert-butyl 2,2'-((butane-1,4-diylbis(oxy))bis(2-fluoro-4,1-phenylene))bis(7-chloro-4-oxo-4*H*-chromene-3-carboxylate)**

Tert-butyl 7-chloro-2-(2-fluoro-4-hydroxyphenyl)-4-oxo-4*H*-chromene-3-carboxylate (200 mg, 0.51 mmol, 1.0 eq), $K_2CO_3$ (105 mg, 0.77 mmol, 1.5 eq) and 1,4-bromobutane (47.5 mg, 0.22 mmol, 0.4 eq) were dissolved in DMF and stirred at 60 °C for 12 hours. Upon completion of the reaction, the solvent was removed by distillation under reduced pressure, followed by extraction with ethyl acetate and distilled water. The organic layer was dried over anhydrous $MgSO_4$, and the impurities were purified by column chromatography to obtain 195 mg (91.2%) of di-tert-butyl 2,2'-((butane-1,4-diylbis(oxy))bis(2-fluoro-4,1-phenylene))bis(7-chloro-4-oxo-4*H*-chromene-3 -carboxylate).

$^1H$ NMR (500 MHz, $CDCl_3$): $\delta$ 8.18 (d, *J* = 8.6 Hz, 1H), 7.54 (t, *J* = 8.4 Hz, 1H), 7.49 (d, *J* = 1.9 Hz, 1H), 7.40 (dd, *J* = 8.6, 1.9 Hz, 1H), 6.80 (dd, *J* = 8.6, 2.4 Hz, 1H), 6.74 (dd, *J* = 11.9, 2.4 Hz, 1H), 4.12 (s, 2H), 2.05 (s, 2H), 1.55 (s, 2H), 1.44 (s, 9H); $^{13}C$ NMR (125 MHz, $CDCl_3$): $\delta$ 173.94, 163.01, 162.90, 160.98, 160.93, 159.82, 156.00, 140.19, 131.33, 131.30, 127.51, 126.42, 122.17, 121.05, 118.15, 112.55, 110.63, 82.81, 68.13, 27.88, 25.57.

**<Reaction 2> Synthesis of 2,2'-((butane-1,4-diylbis(oxy))bis(2-fluoro-4,1-phenylene))bis(7-chloro-4-oxo-4*H*-chromene-3-carboxylic acid)**

Di-tert-butyl 2,2'-((butane-1,4-diylbis(oxy))bis(2-fluoro-4,1-phenylene))bis(7-chloro-4-oxo-4H-chromene-3-carboxylate) (195 mg, 0.23 mmol, 1.0 eq) was dissolved in dichloromethane, and TFA (0.46 mL, 6 mmol, 26.0 eq) and triethylsilane (0.185 mL, 1.16 mmol, 5.0) eq) were added and the mixture was stirred at room temperature for 30 minutes. When the reaction was completed, the impurities were removed by diethyl ether (trituration) at 0 °C after distillation under reduced pressure. The resulting solid was dissolved in distilled water and triethylamine (35 mg, 0.35 mmol, 1.5 eq) was added dropwise, followed by stirring at room temperature for 1 hour. At 0 °C, 1 N HCl aqueous solution was slowly added dropwise to adjust the pH of the solution to 1-2, and the resulting solid was filtered with distilled water to obtain 87.5 mg (51.8%) of the compound of Example 50.

$^1H$ NMR (500 MHz, DMSO-$d_6$) $\delta$ 13.36 (bs, 1H), 8.11 (d, *J* = 8.6 Hz, 1H), 7.95 (d, *J* = 1.8 Hz, 1H), 7.66 (t, *J* = 8.6 Hz, 1H), 7.62 (dd, *J* = 8.7, 1.8 Hz, 1H), 7.09 (dd, *J* = 12.6, 2.1 Hz, 1H), 7.01 (dd, *J* = 8.7, 2.1 Hz, 1H), 4.19 (m, 2H), 1.94 (m, 2H); $^{13}C$ NMR (125 MHz, DMSO-$d_6$) $\delta$ 173.49, 164.55, 162.83, 162.02, 159.29, 155.66, 139.30, 131.42, 127.05, 126.64, 121.42, 120.21, 118.51, 111.63, 111.36, 102.50, 68.11, 25.00.

**<Example 51> 2,2'-(((oxybis(ethane-2,1-diyl))bis(oxy))bis(2-fluoro-4,1-phenylene))bis(7-chloro-4-oxo-4*H*-chromene-3-carboxylic acid)**

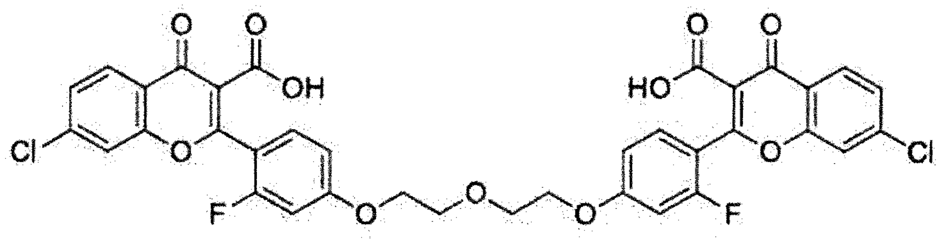

**<Reaction 1> Synthesis of tert-butyl 2,2'-(((oxybis(ethane-2,1-diyl))bis(oxy))bis(2-fluoro-4,1-phenylene))bis(7-chloro-4-oxo-4*H*-chromene-3-carboxylate)**

Tert-butyl 7-chloro-2-(2-fluoro-4-hydroxyphenyl)-4-oxo-4*H*-chromene-3-carboxylate (150 mg, 0.38 mmol, 1.0 eq), $K_2CO_3$ (79.5 mg, 0.58 mmol, 1.5 eq) and di(ethylene glycol) di-p-tosylate (71.6 mg, 0.17 mmol, 0.45 eq) were dissolved in DMF and stirred at 60 °C for 5 hours. When the reaction was completed, the solvent was removed by distillation under reduced pressure, followed by extraction with ethyl acetate and distilled water. The organic layer was dried over anhydrous $MgSO_4$, and the impurities were purified by column chromatography to obtain 98 mg (30.1%) of tert-butyl 2,2'-(((oxybis(ethane-2,1-diyl))bis(oxy))bis(2-fluoro-4,1-phenylene))bis(7-chloro-4-oxo-4*H*-chromene-3-carboxylate).

**<Reaction 2> Synthesis of 2,2'-(((oxybis(ethane-2,1-diyl))bis(oxy))bis(2-fluoro-4,1-phenylene))bis(7-chloro-4-oxo-4*H*-chromene-3-carboxylic acid)**

Tert-butyl 2,2'-(((oxybis(ethane-2,1 -diyl))bis(oxy))bis(2-fluoro-4,1 -phenylene))bis(7-chloro-4-oxo-4*H*-chromene-3-carboxylate) (150 mg, 0.38 mmol, 1.0 eq), $K_2CO_3$ (79.5 mg, 0.58 mmol, 1.5 eq) and di(ethylene glycol) di-p-tosylate (71.6 mg, 0.17 mmol, 0.45 eq) were dissolved in DMF and stirred at 60 °C for 12 hours. Upon completion of the reaction, the solvent was removed by distillation under reduced pressure, followed by extraction with ethyl acetate and distilled water. The organic layer was dried over $MgSO_4$, and impurities were purified and removed by column chromatography, and then the next step was performed. Purified di-tert-butyl 2,2'-(((oxybis(ethane-2,1-diyl))bis(oxy))bis(2-fluoro-4,1-phenylene))bis(7-chloro-4-oxo-4*H*-chromene-3-carboxylate) (98 mg, 0.12 mmol, 1.0 eq) was dissolved in dichloromethane, and TFA (0.23 mL, 3 mmol, 26.0 eq) and triethylsilane (0.09 mL, 0.58 mmol, 4.3 eq) were added and stirred at room temperature for 30 minutes. When the reaction was completed, the impurities were removed by diethyl ether (trituration) at 0 °C after distillation under reduced pressure. The resulting solid was dissolved in distilled water and triethylamine (35 mg, 0.35 mmol, 1.5 eq) was added dropwise, followed by stirring at room temperature for 1 hour. At 0 °C, 1 N HCl aqueous solution was slowly added dropwise to adjust the pH of the solution to 1-2, and the resulting solid was filtered with distilled water to obtain 51.1 mg (60.1%) of the compound of Example 51.

$^1H$ NMR (500 MHz, $CDCl_3$): $\delta$ 13.99 (bs, 1H), 8.26 (d, *J* = 8.6 Hz, 1H), 7.63 (d, *J* = 1.8 Hz, 1H), 7.53 (dd, *J* = 8.6, 1.8 Hz, 1H), 7.49 (t, *J* = 8.3 Hz, 1H), 6.87 (dd, *J* = 8.7, 2.3 Hz, 1H), 6.76 (dd, *J* = 11.9, 2.3 Hz, 1H), 4.24 (t, *J* = 4.5 Hz, 2H), .97 (t, *J* = 4.5 Hz, 2H); $^{13}C$ NMR (125 MHz, $CDCl_3$): $\delta$ 179.67, 168.63, 163.47, 162.89, 161.29,155.74, 142.36, 130.87, 127.75, 120.61, 118.45, 113.37, 111.92, 110.98, 102.70, 69.68, 68.20.

**<Example 52> N,N'-(ethane-1,2-diyl)bis(7-chloro-2-(2,5-difluorophenyl)-4-oxo-4*H*-chromene-3-carboxamide)**

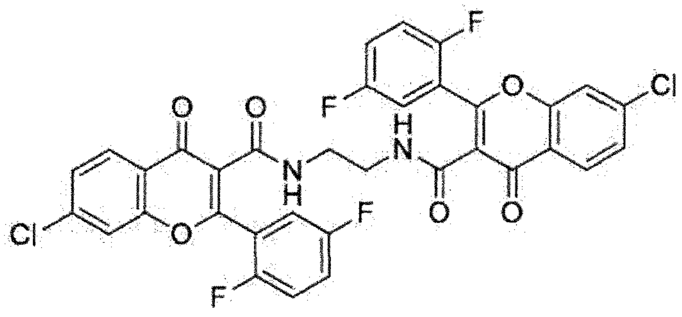

**<Reaction 1> Synthesis of 7-chloro-2-(2,5-difluorophenyl)-4-oxo-4*H*-chromene-3-carbonyl chloride**

7-chloro-2-(2,5-difluorophenyl)-4-oxo-4*H*-chromene-3-carboxylic acid (310 mg, 0.92 mmol, 1.0 eq), oxalyl chloride (3.7 g, 2.92 mmol, 3.2 eq) and 1 drop of dimethylformamide were dissolved in dichloromethane and stirred at room temperature for 2 hours. When the reaction was completed, the solvent was removed by distillation under reduced pressure to obtain 326 mg (99.0%) of 7-chloro-2-(2,5-difluorophenyl)-4-oxo-4*H*-chromene-3-carbonyl chloride.

$^1H$ NMR (500 MHz, $CDCl_3$): $\delta$ 8.24 (d, *J* = 8.6 Hz, 1H), 7.57 (d, *J* = 1.9 Hz, 1H), 7.50 (dd, *J* = 8.6, 1.9 Hz, 1H), 7.35-7.30 (m, 2H), 7.27-7.23 (m, *J* = 1H).

**<Reaction 2> Synthesis of N,N'-(ethane-1,2-diyl)bis(7-chloro-2-(2,5-difluorophenyl)-4-oxo-4*H*-chromene-3-carboxamide)**

TEA (0.1 mL, 0.72 mmol, 1.2 eq) and anhydrous ethylenediamine (0.02 mL, 0.30 mmol, 0.5 eq) were dissolved in dichloromethane and then 7-chloro-2-(2,5-difluorophenyl)-4-oxo-4H-chromene-3-carbonyl chloride (210 mg, 0.60 mmol, 1.0 eq) dissolved in dichloromethane was slowly added dropwise followed by stirring at room temperature for 2 hours. The solid filtrate produced during the reaction was filtered with dichloromethane to obtain 201 mg (97.1%) of the compound of Example 52.

$^1$H NMR (500 MHz, $CDCl_3$): $\delta$ 9.04 (bs, 1H), 8.20 (d, *J* = 8.6 Hz, 1H), 7.55 (d, *J* = 1.9 Hz, 1H), 7.46 (dd, *J* = 8.6, 1.9 Hz, 1H), 7.28-7.25 (m, 1H), 7.20-7.15 (m, 1H), 7.10 (td, *J* = 9.0, 4.3 Hz, 1H), .56 (t, *J* = 2.8 Hz, 2H); $^{13}$C NMR (125 MHz, $CDCl_3$): $\delta$ 176.57, 163.59, 162.58, 158.20, 155.62, 155.52, 141.10, 127.78, 127.13, 123.49, 122.07, 119.01, 118.18, 117.33, 116.91, 116.32, 9.14.

**<Example 53> N,N'-(propane-1,3-diyl)bis(7-chloro-2-(2,5-difluorophenyl)-4-oxo-4*H*-chromene-3-carboxamide)**

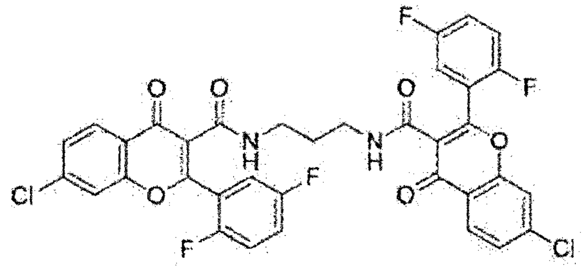

TEA (0.61 mL, 4.4 mmol, 5 eq) and 1,3-diaminopropane (0.04 mL, 0.44 mmol, 0.5 eq) were dissolved in dichloromethane and then 7-chloro-2-(2,5-difluorophenyl)-4-oxo-4*H*-chromene-3-carbonyl chloride (316 mg, 0.89 mmol, 1.0 eq) dissolved in dichloromethane was slowly added dropwise followed by stirring at room temperature for 2 hours. When the reaction was completed, the reaction was washed with 1N HCl aqueous solution and saturated $NaHCO_3$ solution. Then, the organic layer was dried over anhydrous $MgSO_4$, filtered, and distilled under reduced pressure to obtain 87 mg (29.0%) of N,N'-(propane-1,3-diyl)bis(7-chloro-2-(2,5-difluorophenyl)-4-oxo-477-chromene-3-carboxamide).

$^1$H NMR (500 MHz, $CDCl_3$): $\delta$ 8.74 (t, *J* = 5.7 Hz, 1H), 7.99 (d, *J* = 8.6 Hz, 1H), 7.49 (d, *J* = 1.9 Hz, 1H), 7.33 (dd, *J* = 8.6, 1.9 Hz, 1H), 7.32-7.30 (m, 1H), 7.22-7.13 (m, 2H), .47 (q, *J* = 6.3 Hz, 2H), 1.87 (quintet, *J* = 6.3 Hz, 1H); $^{13}$C NMR (125 MHz, $CDCl_3$): $\delta$ 176.40, 162.36, 162.25, 158.21, 155.71, 155.54, 141.07, 127.33, 126.93, 122.94, 121.88, 119.21, 118.77, 118.11, 117.11, 116.55, 7.79, 28.76.

**<Example 54> N,N'-(butane-1,4-diyl)bis(7-chloro-2-(2,5-difluorophenyl)-4-oxo-4*H*-chromene-3-carboxamide)**

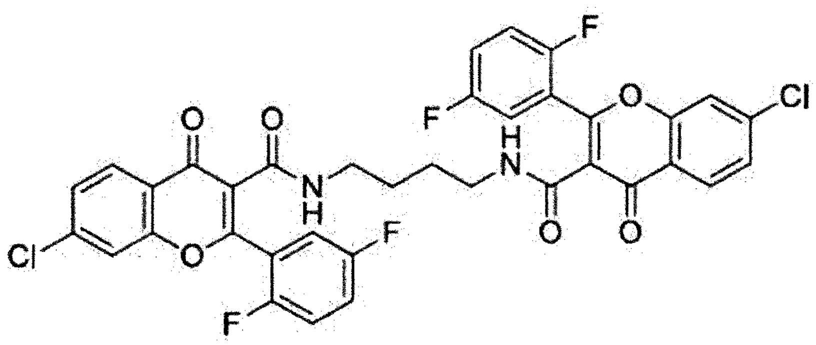

TEA (0.61 mL, 4.4 mmol, 5 eq) and 1,4-diaminobutane (0.04 mL, 0.44 mmol, 0.5 eq) were dissolved in dichloromethane and then 7-chloro-2-(2,5-difluorophenyl)-4-oxo-4H-chromene-3-carbonyl chloride (316 mg, 0.89 mmol, 1.0 eq) dissolved in dichloromethane was slowly added dropwise followed by stirring at room temperature for 2 hours. Upon completion of the reaction, the reaction mixture was washed with 1N HCl aqueous solution and saturated $NaHCO_3$ solution. Then,

the organic layer was dried over anhydrous $MgSO_4$, filtered, and distilled under reduced pressure to obtain 20 mg (6.5%) of N,N'-(butane-1,4-diyl)bis(7-chloro-2-(2,5-difluorophenyl)-4-oxo-4*H*-chromene-3-carboxamide) was obtained.

$^1H$ NMR (500 MHz, $CDCl_3$): $\delta$ 8.87 (t, *J* = 5.4 Hz, 1H), 8.18 (d, *J* = 8.6 Hz, 1H), 7.53 (d, *J* = 1.9 Hz, 1H), 7.45 (dd, *J* = 8.6, 1.9 Hz, 1H), 7.26-7.21 (m, 1H), 7.21-7.17 (m, 1H), 7.13 (td, *J* = 9.0, 4.3 Hz, 1H), .39-3.38 (m, 2H), 1.67 (t, *J* = 2.9 Hz, 2H); $^{13}C$ NMR (125 MHz, $CDCl_3$): $\delta$ 176.73, 163.42, 161.97, 158.20, 155.59, 155.46, 141.10, 127.62, 127.12, 123.52, 122.01, 119.01, 118.17, 117.58, 116.92, 116.26, 9.22, 26.80.

**<Example 55> 3,3'-(piperazine-1,4-dicarbonyl)bis(7-chloro-2-(2,5-difluorophenyl)-4*H*-chromen-4-one)**

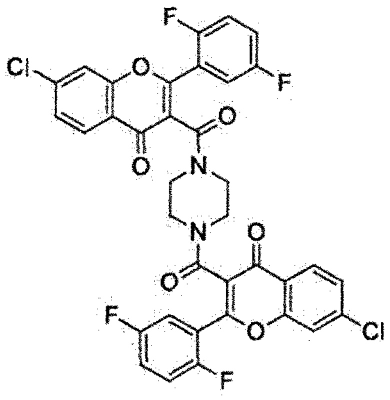

TEA (0.11 mL, 0.80 mmol, 1.2 eq) and piperazine (32.0 mg, 0.37 mmol, 0.5 eq) were dissolved in dichloromethane and then 7-chloro-2-(2,5-difluorophenyl)-4-oxo-4*H*-chromene-3-carbonyl chloride (316 mg, 0.89 mmol, 1.0 eq) dissolved in dichloromethane was slowly added dropwise followed by stirring at room temperature for 2 hours. Upon completion of the reaction, the reaction mixture was washed with 1N HCl aqueous solution and saturated $NaHCO_3$ solution. Then, the organic layer was dried over anhydrous $MgSO_4$, filtered, and distilled under reduced pressure to obtain 175.4 mg (65.3%) of 3,3'-(piperazine-1,4-dicarbonyl)bis(7-chloro-2-(2,5-difluorophenyl)-4*H*-chromen-4-one).

$^1H$ NMR (500 MHz, $CDCl_3$): $\delta$ 8.20-8.12 (m, 2H), 7.56-7.51 (m, 2H), 7.47-7.41 (m, 1H), 7.40-7.6 (m, 1H), 7.28-7.19 (m, 2H), 4.09-3.81 (m, 1H), 3.59-3.35 (m, 3H); $^{13}C$ NMR (125 MHz, $CDCl_3$): $\delta$ 174.01, 162.20, 158.35, 157.48, 156.19, 155.66, 140.95, 127.42, 126.97, 122.47, 121.57, 120.29, 118.32, 117.78, 117.24, 47.18, 46.84, 41.83, 41.57.

**<Experiment 1> Measurement of TNF-induced apoptosis inhibition activity (*in vitro* assay)**

In this experiment, the TNF-induced apoptosis inhibitory activity by the compound of the present invention was investigated. To this end, mouse fibroblasts were treated with the chromenone and quinolone derivative compounds prepared in the above Examples, and then cell viability was measured by a conventionally known MTS assay.

**(1) Preparation of mouse fibroblasts**

Mouse fibroblast L929 cells were purchased from the American Type Culture Collection (ATCC Manassas, VA), and then cultured in RPMI medium containing 10% Fetal Bovine Serum (FBS) and 1% antibiotics at the incubator in which 5% $CO_2$ and humidity at 37 °C were maintained.

**(2) Determination of TNF-induced apoptosis inhibitory activity of the compounds in L929 cells**

Mouse fibroblast L929 cells were treated with recombinant TNF and actinomycin D, a sensitizer, to induce apoptosis, with which an assay capable of confirming apoptosis inhibitory activity was established. In particular, apoptosis conditions were induced by adding actinomycin D as a sensitizer and TNF (20 ng/mL) to L292, and then L929 cells were pretreated with the compounds of Examples 52, 54, and 55 at a concentration of 0 to 40 μM for 24 hours. After 24 hours, they were treated with MTS for 3 hours, and then the absorbance was measured at 490 nm to calculate $IC_{50}$ values. % Inhibition of TNF was calculated by the following formula.

% Inhibition of TNF = (OD value of the group treated with the compound – OD value of the group treated with TNF and actinomycin D)/(OD value of the group treated with actinomycin D – OD value of the group treated with TNF and actinomycin D) × 100

$IC_{50}$ values were statistically calculated using Prism 6 (GraphPad) ($IC_{50}$: 50% inhibitory concentration).

The results are as shown in Table 1 and Fig. 1, and when treated with the compound of the present invention, apoptosis was inhibited by up to 100%.

[Table 1]

| Example compound | $IC_{50}$ (μM) |
|---|---|
| 52 | 1.482 |
| 54 | 7.185 |
| 55 | 7.628 |

This result demonstrates that the compound of the present invention has excellent effect of TNF inhibition activity by inhibiting TNF-induced apoptosis in mouse fibroblasts.

**<Experiment 2> Inhibition of TNF response in HEK-Blue TNF cells (*in vitro* assay)**

In this experiment, the TNF-inhibitory activity of the compound of the present invention was evaluated in HEK-Blue TNF cells. To this end, the transformed HEK-Blue TNF cells that respond specifically to the binding of TNF to TNFRs on the cell surface were treated with the compound of Example 48, followed by a reporter gene assay.

**(1) Preparation of HEK-Blue TNF Cells**

HEK-Blue TNF cells are human embryonic kidney cells that were transformed to express SEAP (secreted embryonic alkaline phosphatase) reporter gene having the binding site for activated NF-κB when TNF binds to TNFR on the cell surface and NF-κB is activated in the cell. They were purchased from Invivogen (San Diego, CA, USA) and were cultured in DMEM medium containing Normocin™ (100 μg/mL) (Invivogen Cat# ant-nr-1), Puromycin (1 μg/mL) (Invivogen Cat# ant-pr-1), Zeocin™ (100 μg/mL) (Invivogen Cat# ant-zn-1), 10% Fetal Bovine Serum (FBS) and 1% antibiotics, by using an incubator in which 5% $CO_2$ and humidity at 37 °C were maintained. When treating with the compound, 0.5% of fetal bovine serum was used, and the experiment was carried out after normocin, puromycin, and zeocin were removed.

**(2) Inhibition of TNF-dependent activity in HEK-Blue TNF cells by the compounds**

An experimental method was used in which SEAP, a reporter gene having the binding site for activated NF-κB in the intracellular signal transduction process, is expressed when human embryonic kidney cells, HEK-Blue TNF cells, are treated with recombinant TNF. In particular, SEAP is expressed when TNF (0.2 ng/mL) is added to HEK-Blue TNF cells, and the degree of color development is measured by treating the SEAP-specific QUANTI-Blue™ solution followed by reading absorbance at 620 nm to obtain $IC_{50}$ value. The inhibition rate (%) of TNF was calculated by the following formula.

$$\% \text{ Inhibition of TNF} = (\text{OD value of the group treated with TNF} - \text{OD value of the group treated with the compound})/(\text{OD value of the group treated with TNF} - \text{OD value of the group not treated with TNF}) \times 100$$

$IC_{50}$ values were statistically calculated using Prism 6 (GraphPad).

The results are as shown in Table 2 and Fig. 2. When treated with the compound of the present invention, TNF reactivity was inhibited by up to 100%.

[Table 2]

| Example compound | $IC_{50}$ (μM) |
|---|---|
| 48 | 9.67 |

The result demonstrates that the compound of the present invention has excellent effect of TNF-inhibitory activity by specifically inhibiting intracellular signal transduction by TNF in TNF-dependently transformed human embryonic kidney cells.

**<Experiment 3> Determination of direct binding activity to TNF, TNFR1 or TNFR2 using SPR assay**

In this experiment, the direct binding activity (binding kinetics) of the compound of the present invention with TNF, TNFR1, or TNFR2 was measured using surface plasmon resonance (SPR) analysis.

In particular, pH scouting (10 mM acetate buffer, pH 4.0, 4.5, 5.0, 5.5) was first performed to determine an appropriate buffer pH. After recombinant TNF, TNFR1, and TNFR2 were fixed on the CM5 chip using the buffer (2~3,000 RU), a solvent correction standard was set up to correct the DMSO effect, and DMSO was added PBS-TT (PBS + 0.05% Tween20 + 0.01% Triton x-100) buffer to be 5%. Compounds were diluted to various concentrations (3.125, 6.25, 10, 12.5, 20, 25, 50 μM), the diluted compounds were injected onto the chip (30 μL/min, 150 s), and allowed to dissociate (500 s). The sensorgram results were analyzed through a dedicated program, and an affinity value (KD) was obtained. The analysis instrument was a Biacore T200 (GE).

As shown in Fig. 3 (a, b, c), as a result of measuring the degree of direct binding to TNF, TNFR1, or TNFR2 of the compound of Example 48 using SPR assay, the binding affinity (KD) to TNF, TNFR1, and TNFR2 were 3.603 M, 2.871 M, and 0.7665 M, respectively.

Therefore, it was confirmed that the compound of the present invention directly binds to TNF with a higher binding affinity than TNFR1 or TNFR2.

**<Experiment 4> Measurement of inhibitory activity against binding of TNF with TNFR1 or TNFR2 using the FRET assay**

In this experiment, the inhibitory activity of the compound of the present invention was evaluated using a fluorescence resonance energy transfer (FRET) assay to measure the binding of TNF-TNFR1 or TNF-TNFR2.

In particular, biotin-labeled 0.25 nM TNF, XL-665-labeled streptavidin, 0.25 nM His-labeled TNFR1, 1 nM His-labeled TNFR2, and Europium-Gold-labeled anti-his Ab were used. First, each 2 μL (10% DMSO) of the compounds of Examples 48 and 51 was added in 384 wells. After 4 μL of 0.25 nM TNF was added, 4 μL of each of 0.25 nM His-labeled TNFR1 or 1 nM His-labeled TNFR2 was added thereto and incubated at room temperature for 1 hour. After 1 hour incubation, 5 μL of XL-665-labeled streptavidin and 5 μL of Europium-Gold-labeled anti-his Ab were added and further incubated at room temperature. Absorbance was measured at 622 nm and 665 nm. The reagent used at this time was prepared by dissolving in PPI-europium detection buffer.

Two experiments were performed using the above protocol.

First, binding inhibitory activity of the compounds of the present invention to TNF and TNFR1 or TNFR2 was evaluated at a single concentration (10 μM) of the compounds. As a result, as shown in Fig. 4 (a, b), the compounds of Examples 48 and 51 were found to inhibit the binding to TNF and TNFR1 or TNF and TNFR2. In particular, in the case of the compound of Example 48 showed a higher binding inhibitory effect than SPD304 compound, known as a TNF inhibitor, and the flavonoid Quercetin.

Then, $IC_{50}$ values were obtained with the various concentrations of the compounds of Examples 48 and 51. At this time, the value was confirmed through the HTRF ratio, and the HTRF ratio was calculated by the following formula.

$$\text{HTRF ratio} = O.D_{665nm} / O.D_{662\ nm} \times 10^4$$

$IC_{50}$ values were statistically calculated using Prism 6 (GraphPad).
The results are shown in Table 3 and Fig. 5 (a, b).

[Table 3]

| $IC_{50}$ (μM) | TNF-TNFR1 | TNF-TNFR2 |
|---|---|---|
| Example 48 | 3.56 | 3.92 |
| Example 51 | 14.50 | 22.30 |

The result confirmed that the compounds of the present invention inhibit the binding of TNF with TNFR1 or TNFR2, and demonstrated that the effect of inhibiting TNF activity is excellent.

**<Experiment 5> TNF-cell signal transduction inhibitory activity assay**

Two experiments were conducted to examine the TNF-cell signal transduction inhibitory activity by the compound of the present invention. First, immunoprecipitation was performed to evaluate the ubiquitination of an upstream protein of the TNF signaling pathway, that is, RIP1.
In particular, human peripheral blood T cells were treated with the compound of Example 48 (0, 0.1 μM) for 24 hours followed by with TNF for 10 minutes, and then immunoprecipitation was performed with TNFR1 protein.
As a result, as shown in Fig. 6a, it was confirmed that the degree of RIP1 ubiquitination activated by TNF treatment was reduced in the experimental group treated with the compound of Example 48 compared to the group treated with hTNF alone.
Second, the inhibition of TNF signal transduction activity of Example 48 was confirmed. In order to examine the TNF-cell signal transduction inhibitory activity by the compound of the present invention, Western blot analysis was performed on proteins of the signal transduction pathway, namely phospho-p65, phospho-IKKα/β, phospho-SAPK/JNK, and phospho-AKT.
In particular, hTNF alone (10 ng/ml) or a mixture of hTNF and the compound of Example 48 (0.01, 0.1, 1, 10 μM) was incubated for 30 minutes, and then the incubated mixture was applied to mouse fibroblast L929 cells for 10 minutes. After the treatment, Western blot analysis was performed for phospho-p65, phospho-IKKα/β, phospho-SAPK/JNK, and phospho-AKT.
As a result, as shown in Fig. 6b, it was confirmed that the levels of phospho-p65, phospho-IKKα/β, phospho-SAPK/JNK, and phospho-AKT proteins in the TNF-cell signal transduction pathway activated by TNF treatment was reduced in the experimental group treated with the compound of Example 48 compared to the group treated with hTNF alone.
Therefore, the result demonstrates that the compound of the present invention is excellent in preventing or treating TNF-related diseases by inhibiting cell signal transduction by TNF.

**<Experiment 6> Measurement of inhibition activity in TNF-induced mouse lethality assay**

In this experiment, the TNF-induced mouse death inhibition activity by the compound of the present invention was investigated. To this end, mice were pretreated with the compound of the present invention, and the survival rate of the mice was measured against TNF-induced death.
In particular, after C57BL/6 mice were fasted for 24 hours, 100 μL of the compounds of Examples 2, 32, 39, 40, 44, 47, and 48 in a buffer composition of 10% DMSO in 90% PBS was orally administered at a concentration of 20 or 40 mg/kg. After 30 minutes, a mixed solution of human recombinant TNF 8 μg/kg and D-galactosamine 700 mg/kg in PBS buffer was intraperitoneally administered at 100 μL per mouse, the survival rate of mice was measured every 3 hours from the time of intraperitoneal administration and the experiment was terminated 24 hours later. The mice were divided into 5 groups, the experiment was repeated 5 times, and the Example compounds used for each experiment are as shown in Fig. 7(a, b).
As a result, as shown in Table 4 and Fig. 7 (a, b), it was confirmed that the survival rate of mice against the death by treatment with TNF and D-galactosamine was increased in the group pre-treated with the compound of Example

2, 32, 39, 40, 44, 47 and 48 compared to the control group.

[Table 4]

| Comp. No. | Molecular weight | Survival (%) |
|---|---|---|
| Example 2 | 335.69 | 60 |
| Example 32 | 308.66 | 90 *** |
| Example 39 | 304.25 | 40 |
| Example 40 | 322.69 | 50 * |
| Example 44 | 306.24 | 60 ** |
| Example 47 | 318.27 | 40 * |
| Example 48 | 695.4 | 50 * |
| Mouse: C57BL/6 (Male) / Age: 7 weeks<br>Number of mice: 10/group<br>hTNF (R&D): 8 μg/kg, i.p.<br>D-galactosamine: 700 mg/kg, i.p.<br>Treatment: 30 min before TNF + D-Gal, p.o. | | |

Therefore, the result demonstrates that the compound of the present invention inhibits TNF-induced mouse death by TNF, and shows that the effect of preventing or treating TNF-related diseases is excellent.

As the specific parts of the present invention have been described in detail above, it is clear for a person skilled in the art that these specific descriptions are only preferred embodiments, and the scope of the present invention is not limited thereby. That is, the substantial scope of the present invention is defined by the appended claims and their equivalents.

## Claims

**1.** A compound of Formula I below or a pharmaceutically acceptable salt thereof:

[Formula I]

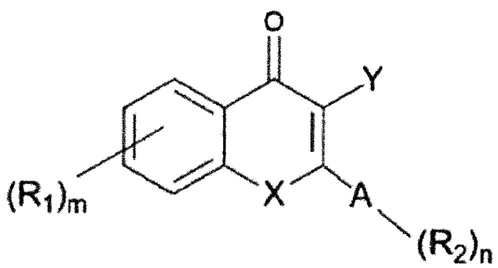

wherein,

X is $-C(R_3)_2-$, $-N(R_3)-$, -O- or -S-,
Y is $-OR_a$, $-SR_a$, $-C(O)R_a$, $-C(O)OR_a$, $-C(O)N(R_a)(R_b)$, $-N(R_a)(R_b)$, $-N(R_a)C(O)R_b$, $-N(R_a)C(O)OR_b$, or $-N(R_a)S(O)OR_b$,
A is $C_3$-$C_8$ cycloalkyl; 4- to 10-membered heterocycloalkyl containing 1 to 4 heteroatoms independently selected from the group consisting of N, O and S; $C_6$-$C_{10}$ aryl; or 5- to 10-membered heteroaryl containing 1 to 4 heteroatoms independently selected from the group consisting of N, O and S,
$R_1$, $R_2$ and $R_3$ may be the same or different and are each independently H, halo, cyano, - ORc, nitro, $C_1$-$C_6$ alkyl, $C_2$-$C_6$ alkenyl, $C_2$-$C_6$ alkynyl, $-SR_c$, $-C(O)R_c$, $-C(O)OR_c$, $-C(O)N(R_c)(R_d)$, $-N(R_c)(R_d)$, $-N(R_c)C(O)R_d$, $-N(R_c)C(O)OR_d$, or $-N(R_c)S(O)OR_d$,
$R_a$, $R_b$, $R_c$ and $R_d$ may be the same or different and are each independently H or $C_1$-$C_6$ alkyl, and

m and n are each independently an integer from 0 to 4.

2. The compound or pharmaceutically acceptable salt thereof according to claim 1, wherein X is -N($R_3$)- or -O-, and $R_3$ is H or $C_1$-$C_6$ alkyl.

3. The compound or pharmaceutically acceptable salt thereof according to claim 1, wherein Y is -$OR_a$, -C(O)$R_a$, -C(O)$OR_a$, or -C(O)N($R_a$)($R_b$).

4. The compound or pharmaceutically acceptable salt thereof according to claim 1, wherein A is phenyl.

5. The compound or pharmaceutically acceptable salt thereof according to claim 1, wherein $R_1$ and $R_2$ are the same or different, and are each independently H, halo or -$OR_c$.

6. A compound of Formula II below or a pharmaceutically acceptable salt thereof

[Formula II]

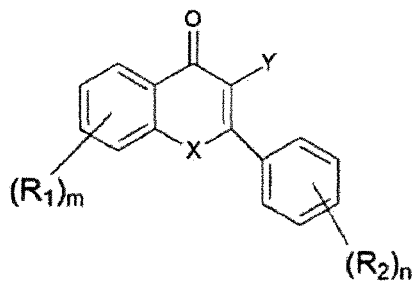

wherein,

X is -N($R_3$)- or -O-,
Y is -$OR_a$, -C(O)$R_a$, -C(O)$OR_a$, or -C(O)N($R_a$)($R_b$),
$R_1$ and $R_2$ can be the same or different, and are each independently H, halo, or -$OR_c$,
$R_3$ is H or $C_1$-$C_6$ alkyl,
$R_a$, $R_b$ and $R_c$ can be the same or different, and are each independently H or $C_1$-$C_6$ alkyl, and
m and n are each independently an integer 1 or 2.

7. A compound selected from the group consisting of

(1) 2-(2,4-difluorophenyl)-3-carboxy-7-chloro-4-(1*H*)-quinolone;
(2) 2-(2,5-difluorophenyl)-3-carboxy-7-chloro-4-(1*H*)-quinolone;
(3) 2-(3,4-difluorophenyl)-3-carboxy-7-chloro-4-(1*H*)-quinolone;
(4) 2-(3,5-difluorophenyl)-3-carboxy-7-chloro-4-(1*H*)-quinolone;
(5) 2-(2,6-difluorophenyl)-3-carboxy-7-chloro-4-(1*H*)-quinolone;
(6) 2-(2,5-difluorophenyl)-3-carboxy-6-chloro-4-(1*H*)-quinolone;
(7) 2-(3,4-difluorophenyl)-3-carboxy-6-chloro-4-(1*H*)-quinolone;
(8) 2-(3,5-difluorophenyl)-3-carboxy-6-chloro-4-(1*H*)-quinolone;
(9) 2-(2,6-difluorophenyl)-3-carboxy-6-chloro-4-(1*H*)-quinolone;
(10) 2-(2,5-difluorophenyl)-3-carboxy-8-chloro-4-(1*H*)-quinolone;
(11) 2-(2,5-difluorophenyl)-3-carboxy-7-bromo-4-(1*H*)-quinolone;
(12) 2-(3,4-difluorophenyl)-3-carboxy-7-bromo-4-(1*H*)-quinolone;
(13) 2-(3,5-difluorophenyl)-3-carboxy-7-bromo-4-(1*H*)-quinolone;
(14) 2-(2,6-difluorophenyl)-3-carboxy-7-bromo-4-(1*H*)-quinolone;
(15) 2-(2,5-difluorophenyl)-3-carboxy-7-methoxy-4-(1*H*)-quinolone;
(16) 2-(3,5-difluorophenyl)-3-carboxy-7-methoxy-4-(1*H*)-quinolone;
(17) 2-(2,6-difluorophenyl)-3-carboxy-7-methoxy-4-(1*H*)-quinolone;
(18) 2-(2,5-difluorophenyl)-3-carboxy-7-fluoro-4-(1*H*)-quinolone;
(19) 1-methyl-2-(2,4-difluorophenyl)-3-carboxy-7-chloro-4-(1*H*)-quinolone;
(20) 1-methyl-2-(2,5-difluorophenyl)-3-carboxy-7-chloro-4-(1*H*)-quinolone;

(21) 1-methyl-2-(3,4-difluorophenyl)-3-carboxy-7-chloro-4-(1*H*)-quinolone;
(22) 1-methyl-2-(3,5-difluorophenyl)-3-carboxy-7-chloro-4-(1*H*)-quinolone;
(23) 1-methyl-2-(2,6-difluorophenyl)-3-carboxy-7-chloro-4-(1*H*)-quinolone;
(24) 1-methyl-2-(2,5-difluorophenyl)-3-carboxy-6-chloro-4-(1*H*)-quinolone;
(25) 1-methyl-2-(3,4-difluorophenyl)-3-carboxy-6-chloro-4-(1*H*)-quinolone;
(26) 1-methyl-2-(3,5-difluorophenyl)-3-carboxy-6-chloro-4-(1*H*)-quinolone;
(27) 1-methyl-2-(2,6-difluorophenyl)-3-carboxy-6-chloro-4-(1*H*)-quinolone;
(28) 1-methyl-2-(2,5-difluorophenyl)-3-carboxy-8-chloro-4-(1*H*)-quinolone;
(29) 1-methyl-2-(2,5-difluorophenyl)-3-carboxy-7-bromo-4-(1*H*)-quinolone;
(30) 1-methyl-2-(3,5-difluorophenyl)-3-carboxy-7-bromo-4-(1*H*)-quinolone;
(31) 1-methyl-2-(2,6-difluorophenyl)-3-carboxy-7-bromo-4-(1*H*)-quinolone;
(32) 7-chloro-2-(2,5-difluorophenyl)-3-hydroxy-4*H*-chromen-4-one;
(33) 7-chloro-2-(3,5-difluorophenyl)-3-hydroxy-4*H*-chromen-4-one;
(34) 7-chloro-2-(3,4-difluorophenyl)-3-hydroxy-4*H*-chromen-4-one;
(35) 2-(2,5-difluorophenyl)-7-fluoro-3-hydroxy-4H-chromen-4-one;
(36) 2-(3,5-difluorophenyl)-7-fluoro-3-hydroxy-4*H*-chromen-4-one;
(37) 2-(2,3-difluorophenyl)-7-fluoro-3-hydroxy-4*H*-chromen-4-one;
(38) 2-(2,5-difluorophenyl)-3-hydroxy-7-methoxy-4*H*-chromen-4-one;
(39) 2-(3,5-difluorophenyl)-3-hydroxy-7-methoxy-4*H*-chromen-4-one;
(40) 7-chloro-2-(2,5-difluorophenyl)-3-methoxy-4*H*-chromen-4-one;
(41) 7-chloro-2-(3,5-difluorophenyl)-3-methoxy-4*H*-chromen-4-one;
(42) 2-(2,5-difluorophenyl)-7-fluoro-3-methoxy-4*H*-chromen-4-one;
(43) 2-(2,4-difluorophenyl)-7-fluoro-3-methoxy-4*H*-chromen-4-one;
(44) 2-(3,5-difluorophenyl)-7-fluoro-3-methoxy-4*H*-chromen-4-one;
(45) 2-(2,5-difluorophenyl)-3,7-dimethoxy-4*H*-chromen-4-one;
(46) 2-(3,5-difluorophenyl)-3,7-dimethoxy-4*H*-chromen-4-one; and
(47) 2-(2,4-difluorophenyl)-3,7-dimethoxy-4*H*-chromen-4-one,

or a pharmaceutically acceptable salt thereof.

**8.** A compound of Formula III below or a pharmaceutically acceptable salt thereof

[Formula III] $M_1$-L-$M_2$

wherein,

$M_1$ and $M_2$ may be the same or different, and are each independently a monomer radical of Formula Ia, and L is a linking group covalently bonding $M_1$ and $M_2$, and is selected from the following Formulas IVa to IVc,

[Formula Ia]

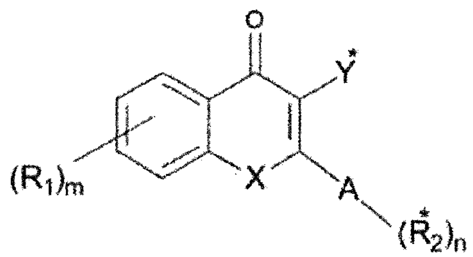

(wherein,

X is -$C(R_3)_2$-, -$N(R_3)$-, -O- or -S-,
Y is -$OR_a$, -$SR_a$, -$C(O)R_a$, -$C(O)OR_a$, -$C(O)N(R_a)(R_b)$, -$N(R_a)(R_b)$, -$N(R_a)C(O)R_b$, - $N(R_a)C(O)OR_b$, or -$N(R_a)S(O)OR_b$,
A is $C_3$-$C_8$ cycloalkyl; 4- to 10-membered heterocycloalkyl containing 1 to 4 heteroatoms independently selected from the group consisting of N, O and S; $C_6$-$C_{10}$ aryl; or 5- to 10-membered heteroaryl containing

1 to 4 heteroatoms independently selected from the group consisting of N, O and S,
$R_1$, $R_2$ and $R_3$ may be the same or different and each independently H, halo, cyano, $-OR_c$, nitro, $C_1$-$C_6$ alkyl, $C_2$-$C_6$ alkenyl, $C_2$-$C_6$ alkynyl, $-SR_c$, $-C(O)R_c$, $-C(O)OR_c$, $-C(O)N(R_c)(R_d)$, $-N(R_c)(R_d)$, $-N(R_c)C(O)R_d$, $-N(R_c)C(O)OR_d$, or $-N(R_c)S(O)OR_d$,
$R_a$, $R_b$, $R_c$ and $R_d$ may be the same or different and are each independently H or $C_1$-$C_6$ alkyl,
m and n are each independently an integer of 0 to 4,
the monomer unit $M_1$ or $M_2$ is bonded to the linking group L through a bonding site * located in either Y or $R_2$, and
when Y or $R_2$ has the above bonding site *, Y or $R_2$ contains N, O or S atoms, and at the bonding site *, -C(O)-, -NH-, $-N(C_1$-$C_6$ alkyl)-, -O- or -S- radical is located),

[Formula IVa] *R-Z-R'*

[Formula IVb] $*-[(CH_2)_2-O]_p-(CH_2)_2-*$

[Formula IVc]

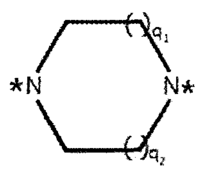

(wherein,

* represents a bonding site between the monomer unit $M_1$ or $M_2$ and the linking group L,
R or R' is each independently $C_1$-$C_{10}$ alkylene, $C_2$-$C_{10}$ alkenylene, or $C_2$-$C_{10}$ alkynylene;
Z is a single bond; -O-; -S-; -NH-; $-N(C_1$-$C_6$ alkyl)-; $C_3$-$C_8$ cycloalkylene; 4- to 10-membered heterocycloalkyl containing 1 to 4 heteroatoms independently selected from the group consisting of N, O and S; $C_6$-$C_{10}$ aryl; or 5- to 10-membered heteroaryl containing 1 to 4 heteroatoms independently selected from the group consisting of N, O and S;
p is an integer from 1 to 5, and
$q_1$ and $q_2$ are each independently an integer from 0 to 5, but $q_1$ and $q_2$ are not 0 at the same time).

**9.** The compound or pharmaceutically acceptable salt thereof according to claim 8, wherein $M_1$ and $M_2$ may be the same or different, and are each independently a monomer radical of Formula IIa below or a pharmaceutically acceptable salt thereof:

[Formula IIa]

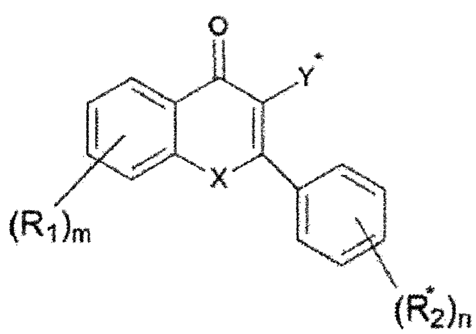

wherein,

X is N-$R_3$ or O,
Y is -$OR_a$, -C(O)$OR_a$, or -C(O)N($R_a$)($R_b$),
$R_1$ and $R_2$ can be the same or different, and are each independently H, halo, or -$OR_c$,
$R_3$ is H or $C_1$-$C_6$ alkyl,
$R_a$, $R_b$ and $R_c$ can be the same or different, and are each independently H or $C_1$-$C_6$ alkyl, and
m and n are each independently an integer 1 or 2.

**10.** The compound or pharmaceutically acceptable salt thereof according to claim 8, wherein L is $C_2$-$C_4$ alkylene, -$(CH_2)_2$-O-$(CH_2)_2$-, or

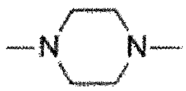 .

**11.** A compound selected from the group consisting of

(48) 2,2'-((ethane-1,2-diylbis(oxy))bis(2-fluoro-4,1-phenylene))bis(7-chloro-4-oxo-4H-chloromene-3-carboxylic acid);
(49) 2,2'-((propane-1,3-diylbis(oxy))bis(2-fluoro-4,1-phenylene))bis(7-chloro-4-oxo-4H-chloromene-3-carboxylic acid);
(50) 2,2'-((butane-1,4-diylbis(oxy))bis(2-fluoro-4,1-phenylene))bis(7-chloro-4-oxo-4H-chloromene-3-carboxylic acid);
(51) 2,2'-(((oxybis(ethane-2,1-diyl))bis(oxy))bis(2-fluoro-4,1-phenylene))bis(7-chloro-4 -oxo-4H-chromene-3-carboxylic acid);
(52) N,N'-(ethane-1,2-diyl)bis(7-chloro-2-(2,5-difluorophenyl)-4-oxo-4H-chromene-3-carboxamide);
(53) N,N'-(propane-1,3-diyl)bis(7-chloro-2-(2,5-difluorophenyl)-4-oxo-4H-chromene-3-carboxamide);
(54) N,N'-(butane-1,4-diyl)bis(7-chloro-2-(2,5-difluorophenyl)-4-oxo-4H-chromene-3-carboxamide); and
(55) 3,3'-(piperazine-1,4-dicarbonyl)bis(7-chloro-2-(2,5-difluorophenyl)-4H-chromene-4-one),

or a pharmaceutically acceptable salt thereof.

**12.** A pharmaceutical composition for preventing or treating a TNF-related disease, comprising the compound or pharmaceutically acceptable salt thereof according to any one of claims 1 to 11, as an active ingredient.

**13.** The pharmaceutical composition according to claim 12, wherein the TNF-related disease is selected from the group consisting of rheumatoid arthritis, juvenile rheumatoid arthritis, inflammatory bowel disease, Crohn's disease, ulcerative colitis, psoriasis, plaque psoriasis, pediatric plaque psoriasis, psoriatic arthritis, polyarticular juvenile idiopathic arthritis, Behcet's enteritis, ankylosing spondylitis, axial spondylarthritis, pediatric osteochondritis-related arthritis, polymyalgia rheumatica, multiple sclerosis, thyroiditis, delayed hypersensitivity, allergy, contact dermatitis, atopic dermatitis, systemic lupus erythematosus, systemic sclerosis, adult-onset Still's disease, asthma, autoimmune thyroid disorder, Sjogren's syndrome, Kawasaki disease, pancreatitis, nephritis, hepatitis, pneumonia, chronic obstructive pulmonary disease, otitis media, angioproliferative nephritis, myelodysplastic syndrome, osteoarthritis, sarcoidosis, granuloma annulus, Wegener's granulomatosis, lupus, hemolytic uremic syndrome, arteriosclerosis, vasculitis, heart failure, stroke, myocardial infarction, myocardial ischemia-reperfusion injury, sexual dysfunction, obesity, hypertension, diabetes and diabetic complications, hyperlipidemia, preeclampsia, kidney disease, liver disease, kidney damage, liver damage, snake bite, allograft rejection, organ transplantation, graft-versus-host disease, dementia, Alzheimer's disease, Parkinson's disease, amyotrophic lateral sclerosis, pain, central nervous system disease, uveitis, Behcet's disease, diabetic macular edema, macular degeneration, orbitopathy, glaucoma, hidradenitis suppurativa, multicentral reticulocytosis, pityriasis rubra pilaris, eosinophilic fasciitis, panniculitis, necrobiosis lipoidica diabeticorum, cicatrical pemphigoid, pyoderma gangrenosum, Sweet's syndrome, subcorneal pustular dermatosis, scleroderma, neutrophilic dermatosis, toxic epidermal necrolysis, pustular dermatosis, dermatomyositis, polymyositis, bullous dermatosis, erythema nodosum, alopecia, depression, bipolar disorder, anxiety disorder, tuberculosis, viral infection, bacterial infection, fungal infection, protozoan infection, cerebral malaria, sepsis, septic shock, prostate cancer, skin cancer, colorectal cancer, kidney cancer, pancreatic cancer, ovarian cancer, breast cancer, bladder cancer, prostate cancer, lymphoma, glioma, osteosarcoma, leukemia, multiple myeloma and cachexia,

**14.** A health functional food composition for preventing or improving a TNF-related disease, comprising the compound

or pharmaceutically acceptable salt thereof according to any one of claims 1 to 11.

**15.** A reagent composition for inhibiting the activity of TNF *in vitro,* comprising the compound or pharmaceutically acceptable salt thereof according to any one of claims 1 to 11.

【Fig. 1】

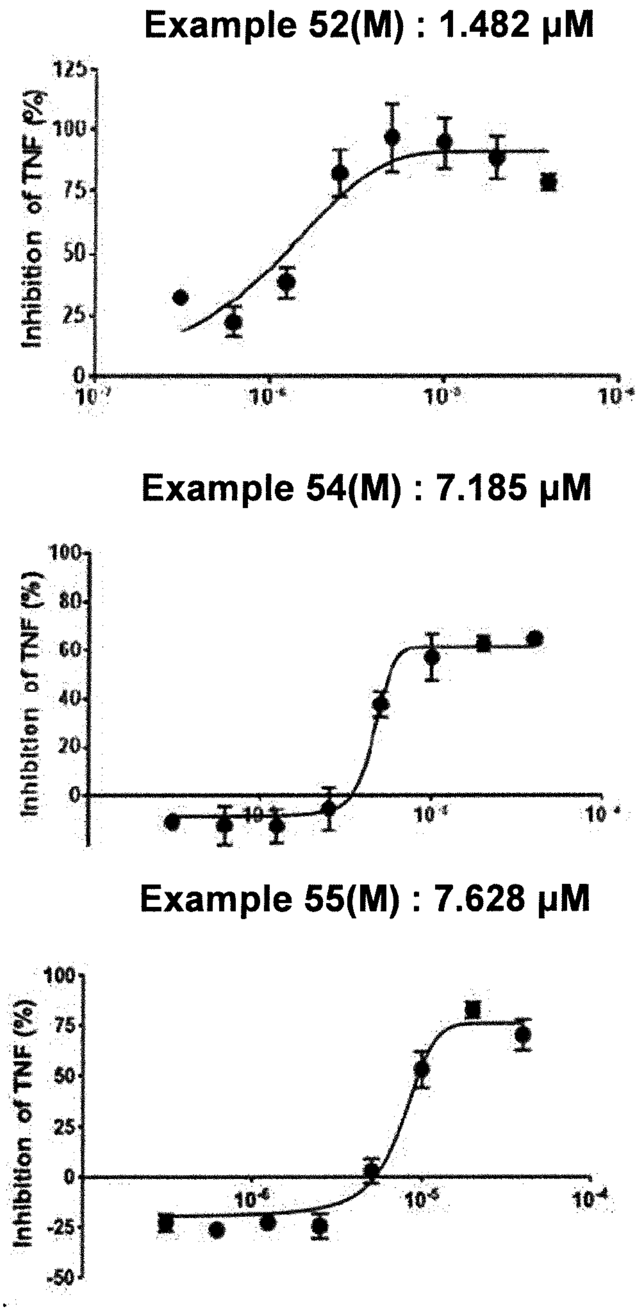
Example 52(M) : 1.482 µM
Inhibition of TNF (%)
125
100
75
50
25
0
Example 54(M) : 7.185 µM
Inhibition of TNF (%)
100
80
60
40
20
0
Example 55(M) : 7.628 µM
Inhibition of TNF (%)
100
75
50
25
0
-25
-50

【Fig. 2】

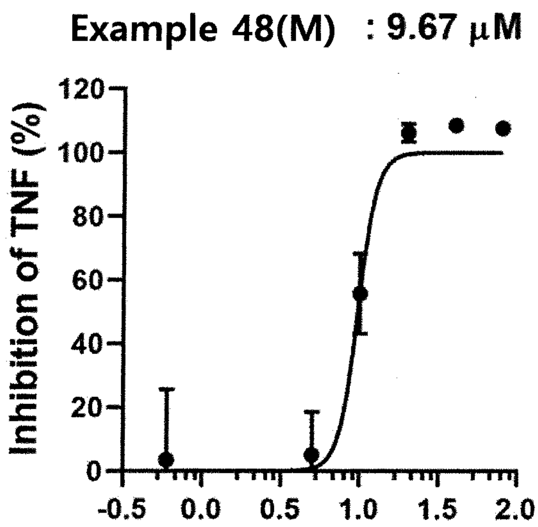
Example 48(M) : 9.67 μM
Inhibition of TNF (%)
120
100
80
60
40
20
0
-0.5
0.0
0.5
1.0
1.5
2.0

【Fig. 3a】

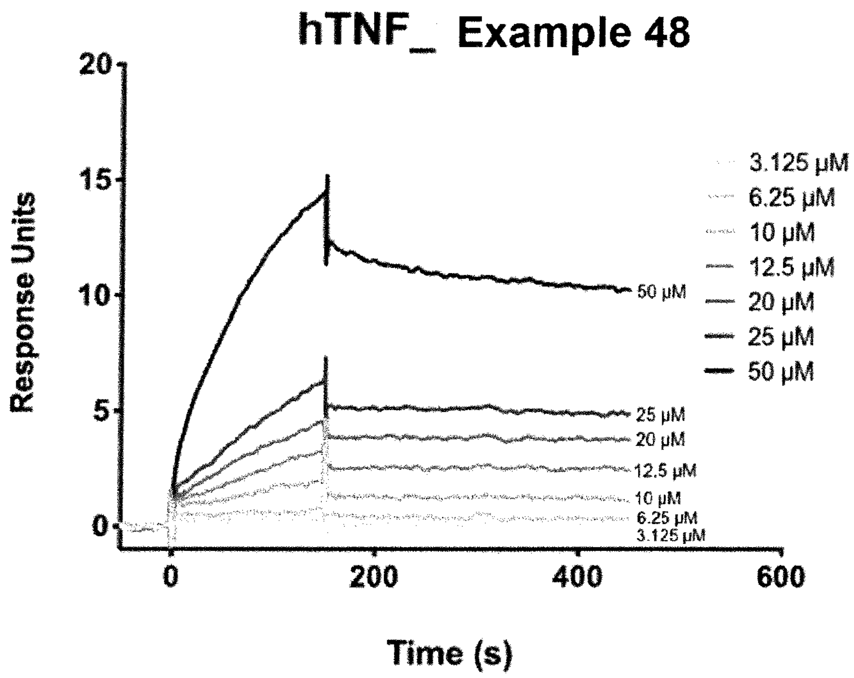
hTNF_ Example 48
Response Units
Time (s)
3.125 μM
6.25 μM
10 μM
12.5 μM
20 μM
25 μM
50 μM

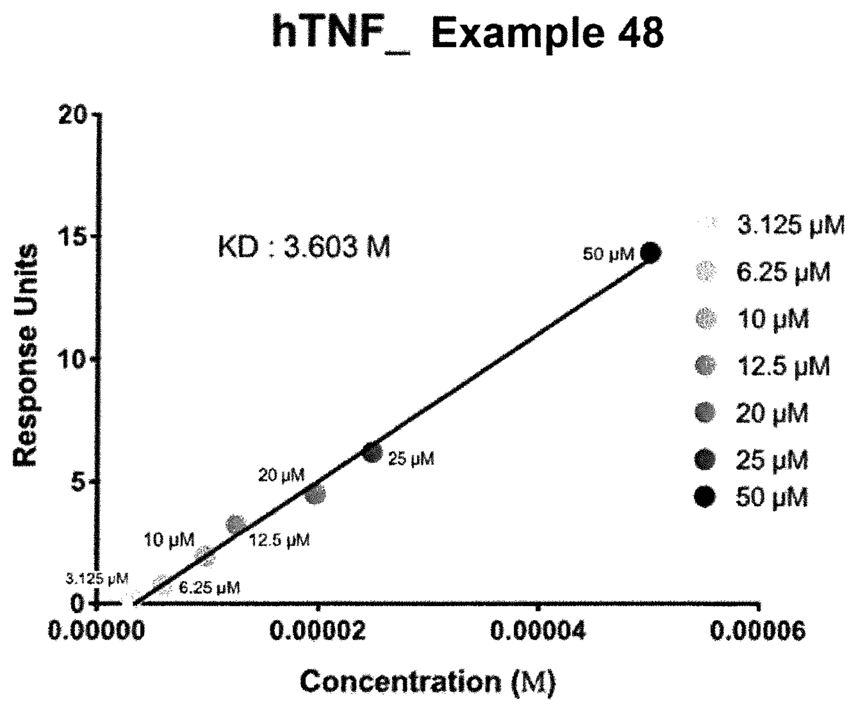
hTNF_ Example 48
KD : 3.603 M
Response Units
Concentration (M)
3.125 μM
6.25 μM
10 μM
12.5 μM
20 μM
25 μM
50 μM

【Fig. 3b】

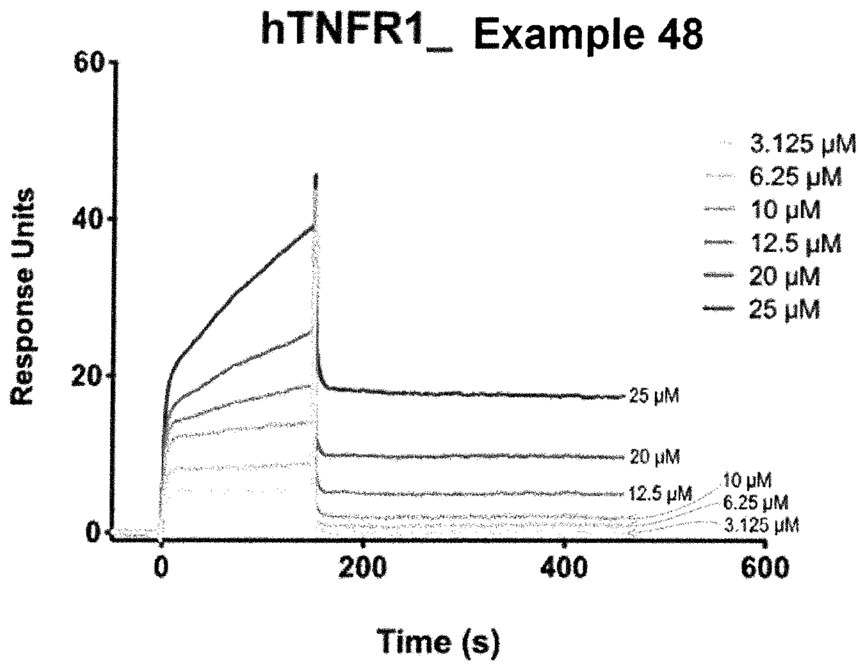
hTNFR1_ Example 48
Response Units
Time (s)
3.125 µM
6.25 µM
10 µM
12.5 µM
20 µM
25 µM

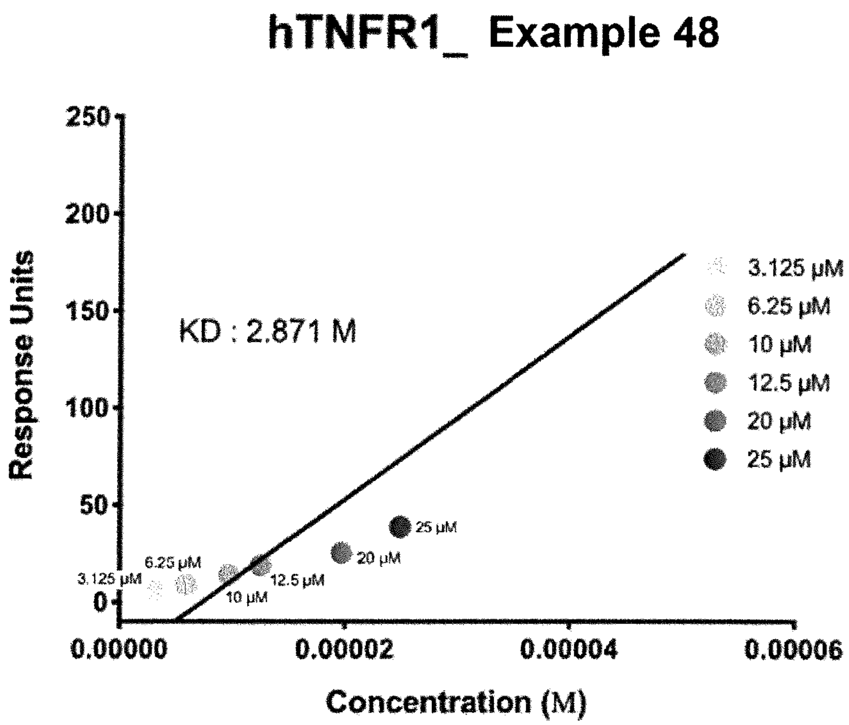
hTNFR1_ Example 48
Response Units
KD : 2.871 M
Concentration (M)
3.125 µM
6.25 µM
10 µM
12.5 µM
20 µM
25 µM

【Fig. 3c】

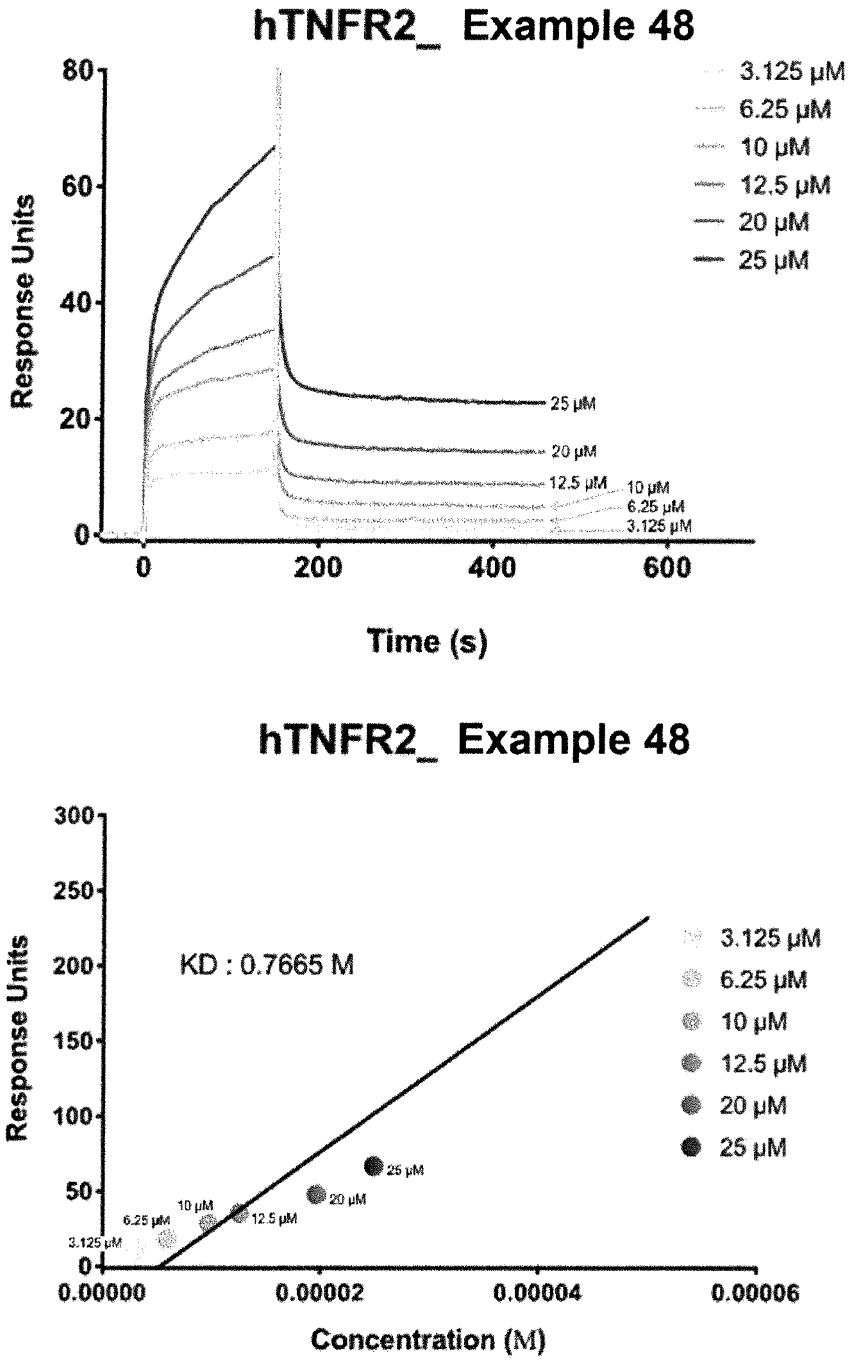
hTNFR2_ Example 48
Response Units
Time (s)
3.125 μM
6.25 μM
10 μM
12.5 μM
20 μM
25 μM
0
20
40
60
80
200
400
600
hTNFR2_ Example 48
Response Units
Concentration (M)
KD : 0.7665 M
0
50
100
150
200
250
300
0.00000
0.00002
0.00004
0.00006

【Fig. 4a】

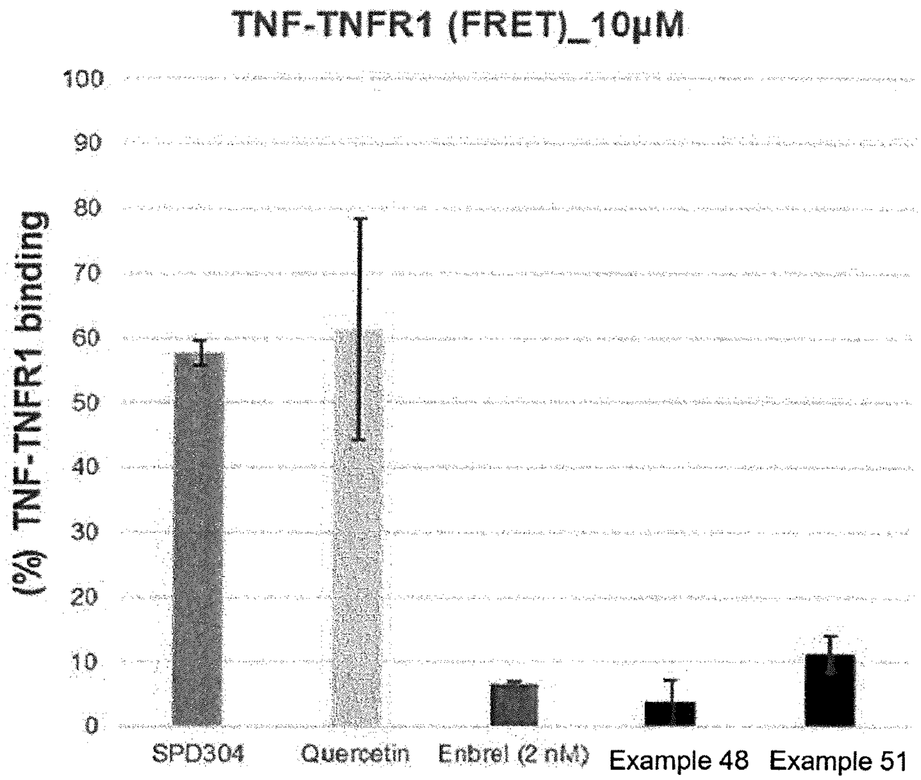
TNF-TNFR1 (FRET)_10μM
(%) TNF-TNFR1 binding
100
90
80
70
60
50
40
30
20
10
0
SPD304
Quercetin
Enbrel (2 nM)
Example 48
Example 51

【Fig. 4b】

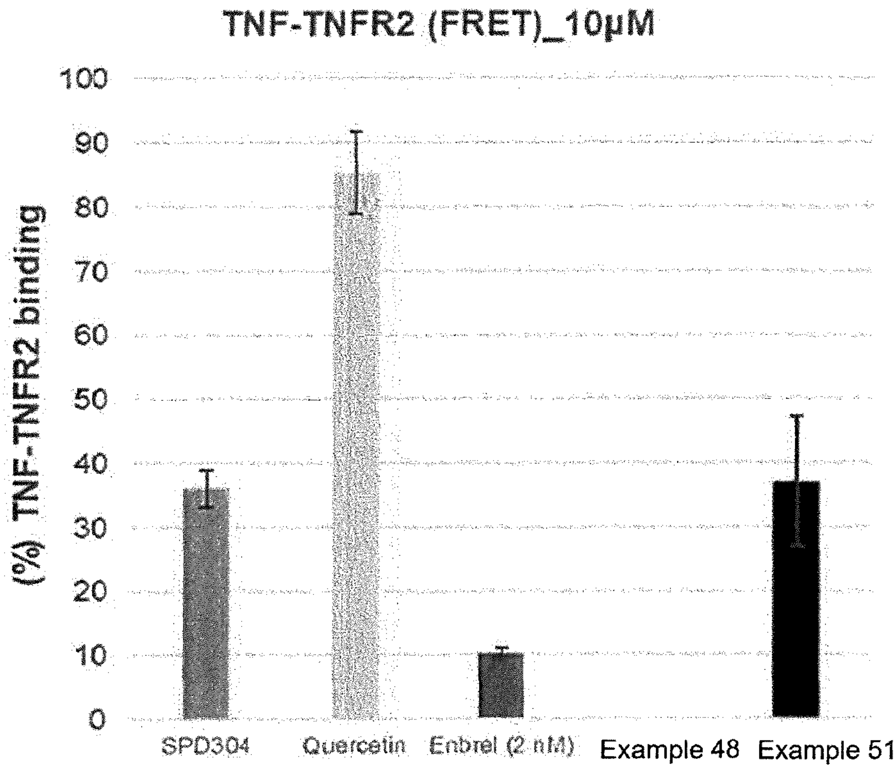
TNF-TNFR2 (FRET)_10μM
(%) TNF-TNFR2 binding
100
90
80
70
60
50
40
30
20
10
0
SPD304
Quercetin
Enbrel (2 nM)
Example 48
Example 51

【Fig. 5a】

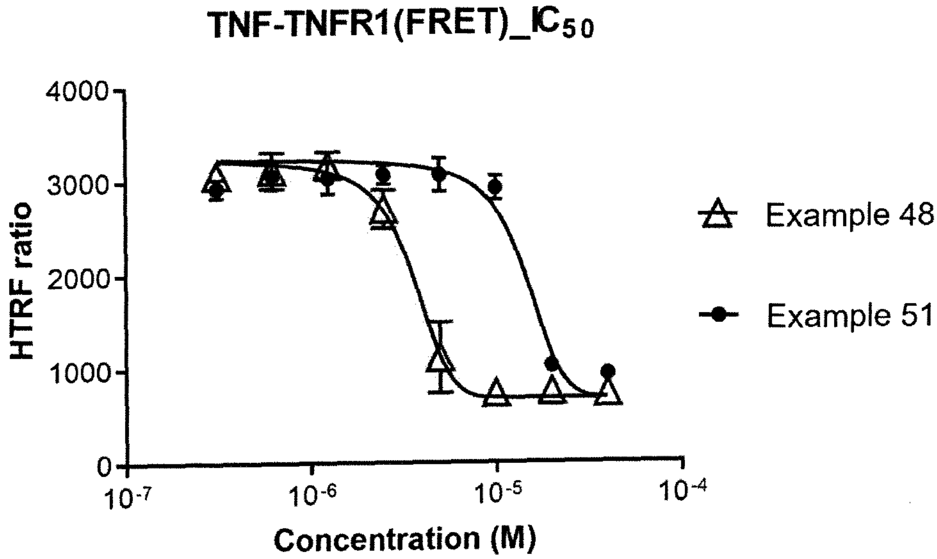
TNF-TNFR1(FRET)_IC50
4000
3000
2000
1000
0
HTRF ratio
10-7
10-6
10-5
10-4
Concentration (M)
Example 48
Example 51

【Fig. 5b】

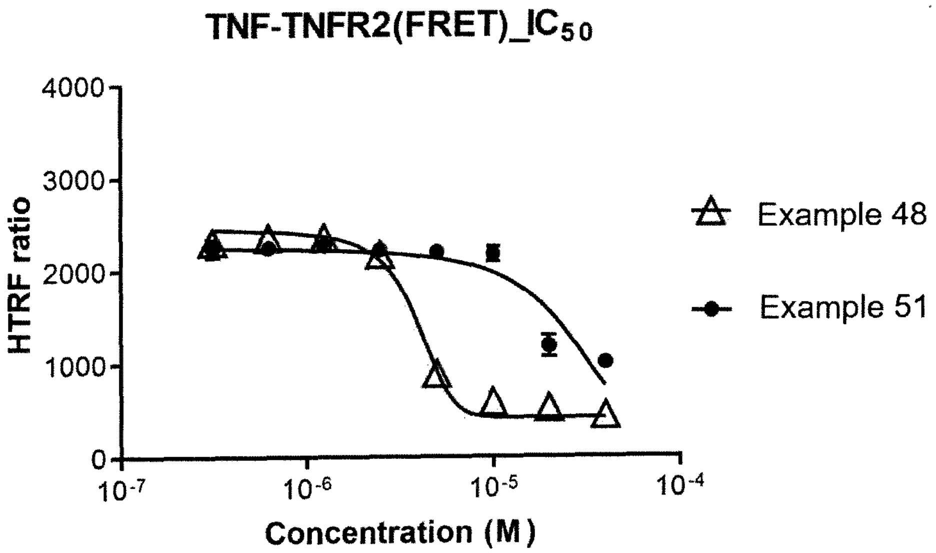
TNF-TNFR2(FRET)_IC50
HTRF ratio
4000
3000
2000
1000
0
10-7
10-6
10-5
10-4
Concentration (M)
Example 48
Example 51

【Fig. 6a】

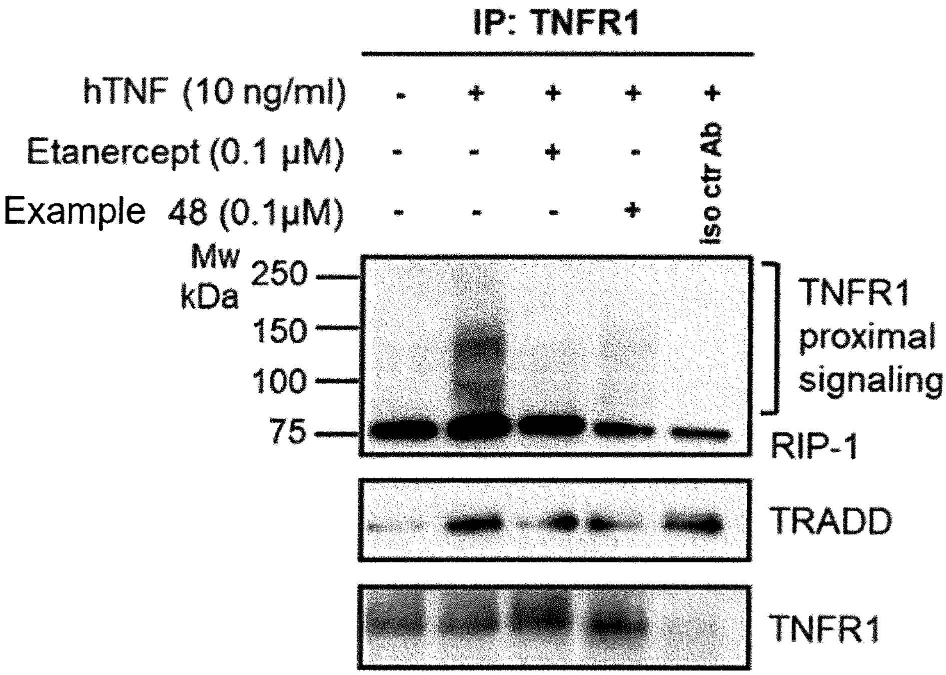
IP: TNFR1
hTNF (10 ng/ml) - + + + +
Etanercept (0.1 μM) - - + -
Example 48 (0.1μM) - - - +
Iso ctr Ab
Mw kDa
250
150
100
75
TNFR1 proximal signaling
RIP-1
TRADD
TNFR1

【Fig. 6b】

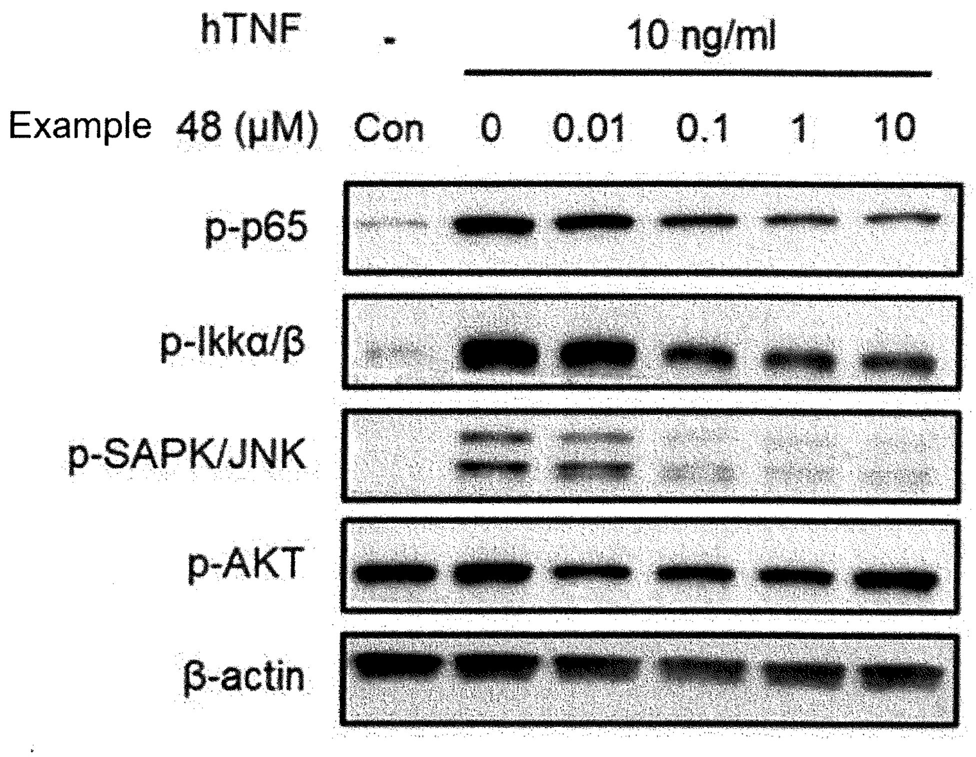
hTNF
-
10 ng/ml
Example 48 (μM)
Con
0
0.01
0.1
1
10
p-p65
p-Ikkα/β
p-SAPK/JNK
p-AKT
β-actin

【Fig. 7a】

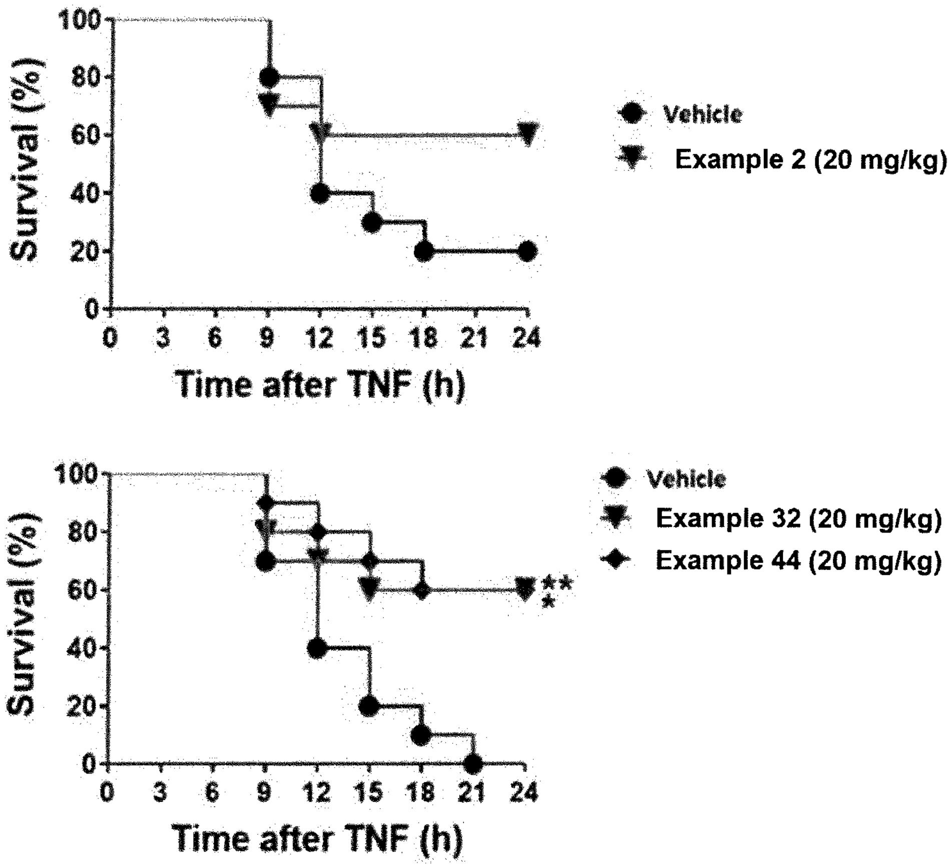
Survival (%)
100
80
60
40
20
0
0 3 6 9 12 15 18 21 24
Time after TNF (h)
Vehicle
Example 2 (20 mg/kg)
Survival (%)
100
80
60
40
20
0
0 3 6 9 12 15 18 21 24
Time after TNF (h)
Vehicle
Example 32 (20 mg/kg)
Example 44 (20 mg/kg)
**
*

【Fig. 7b】

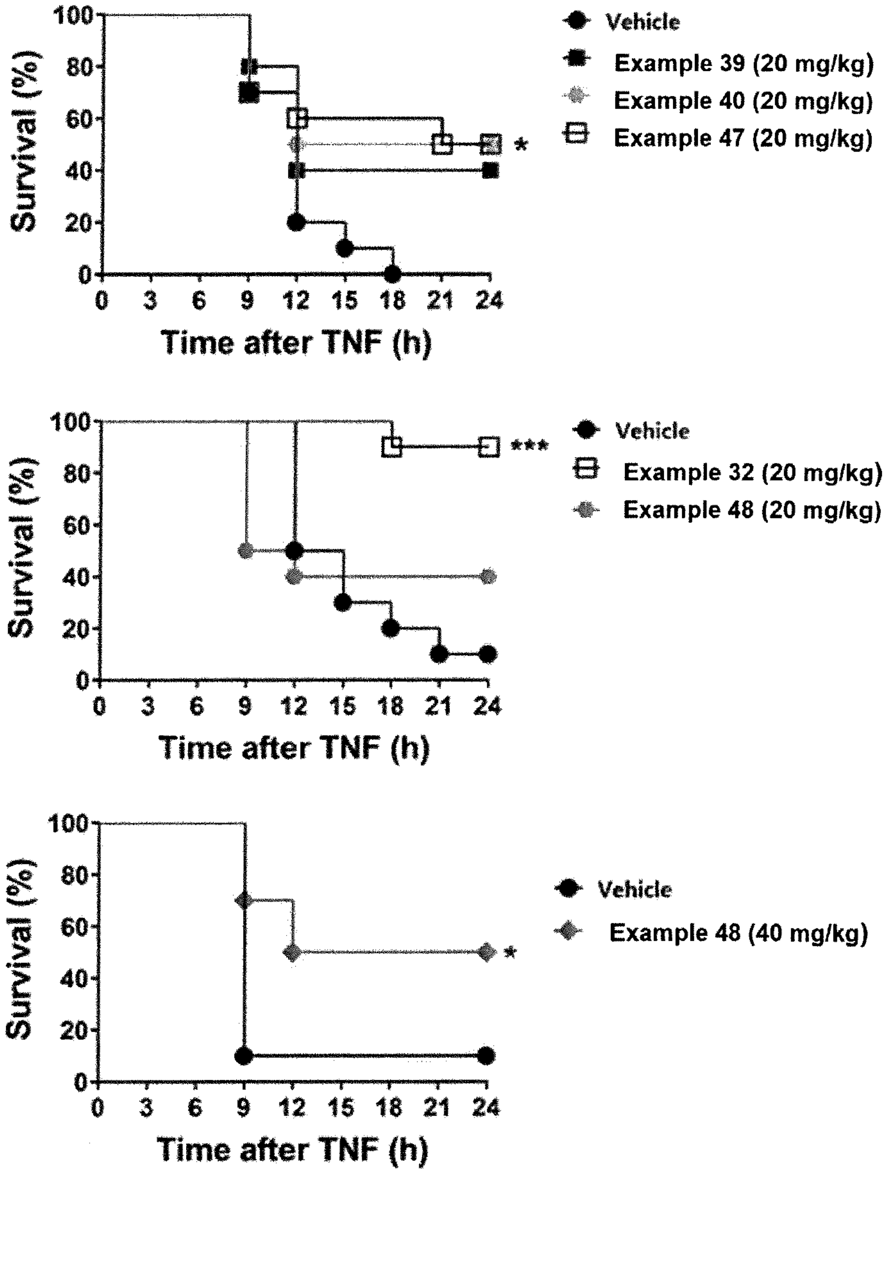
Survival (%)
Time after TNF (h)
Vehicle
Example 39 (20 mg/kg)
Example 40 (20 mg/kg)
Example 47 (20 mg/kg)
*
Survival (%)
Time after TNF (h)
Vehicle
Example 32 (20 mg/kg)
Example 48 (20 mg/kg)
***
Survival (%)
Time after TNF (h)
Vehicle
Example 48 (40 mg/kg)
*

# INTERNATIONAL SEARCH REPORT

International application No.
**PCT/KR2021/008079**

## A. CLASSIFICATION OF SUBJECT MATTER

**C07D 215/233**(2006.01)i; **A61K 31/47**(2006.01)i; **A61K 31/352**(2006.01)i; **C07D 311/22**(2006.01)i; **C07D 407/12**(2006.01)i

According to International Patent Classification (IPC) or to both national classification and IPC

## B. FIELDS SEARCHED

Minimum documentation searched (classification system followed by classification symbols)

C07D 215/233(2006.01); A23L 33/10(2016.01); A61K 31/352(2006.01); C07D 311/24(2006.01); C07D 311/30(2006.01); C07D 405/12(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus, Marpat), Google & keywords: 종양괴사인자 (tumor necrosis factor), 퀴놀론 (quinolone), 크로멘온 (chromenone)

## C. DOCUMENTS CONSIDERED TO BE RELEVANT

| Category* | Citation of document, with indication, where appropriate, of the relevant passages | Relevant to claim No. |
|---|---|---|
| X | CN 107056739 A (CHONGQING UNIVERSITY) 18 August 2017 (2017-08-18)<br>See paragraphs [0004], [0018], [0058], [0062], [0071] and [0083]-[0087]; and claims 1-8. | 1-15 |
| DX | KR 10-1982667 B1 (THE CATHOLIC UNIVERSITY OF KOREA INDUSTRY-ACADEMIC COOPERATION FOUNDATION) 27 May 2019 (2019-05-27)<br>See claims 4, 5, 11, 12 and 14; and paragraphs [0314]-[0316]. | 1-7,12-15 |
| X | KR 10-2008-0013162 A (KOREA INSTITUTE OF SCIENCE AND TECHNOLOGY) 13 February 2008 (2008-02-13)<br>See claim 1; and paragraphs [0002] and [0086]. | 1-7,12-15 |
| X | GHOSH, A. et al. A novel biflavonyloxymethane from Pongamia pinnata and its radical Quenching activity. Natural Product Communications. 2011, vol. 6, no. 5, pp. 625-656.<br>See abstract; figure 1, and table 1. | 8-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 October 2021** | **05 October 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2021/008079**

**C. DOCUMENTS CONSIDERED TO BE RELEVANT**

| Category* | Citation of document, with indication, where appropriate, of the relevant passages | Relevant to claim No. |
|---|---|---|
| A | US 2016-0280676 A1 (VIRGINIA COMMONWEALTH UNIVERSITY) 29 September 2016 (2016-09-29)<br>See entire document. | 1-15 |

Form PCT/ISA/210 (second sheet) (July 2019)

**INTERNATIONAL SEARCH REPORT**
**Information on patent family members**

International application No.
**PCT/KR2021/008079**

| Patent document cited in search report | Publication date (day/month/year) | Patent family member(s) | Publication date (day/month/year) |
|---|---|---|---|
| CN 107056739 A | 18 August 2017 | CN 107056739 B | 02 August 2019 |
| KR 10-1982667 B1 | 27 May 2019 | AU 2018-353722 A1 | 25 April 2019 |
| | | AU 2018-353722 B2 | 29 July 2021 |
| | | CA 3074993 A1 | 25 April 2019 |
| | | CN 111315730 A | 19 June 2020 |
| | | EP 3699174 A1 | 26 August 2020 |
| | | JP 2020-536977 A | 17 December 2020 |
| | | KR 10-1934651 B1 | 02 January 2019 |
| | | KR 10-2019-0044025 A | 29 April 2019 |
| | | US 2020-0246304 A1 | 06 August 2020 |
| | | WO 2019-078452 A1 | 25 April 2019 |
| KR 10-2008-0013162 A | 13 February 2008 | None | |
| US 2016-0280676 A1 | 29 September 2016 | US 9850221 B2 | 26 December 2017 |
| | | WO 2015-069979 A2 | 14 May 2015 |
| | | WO 2015-069979 A3 | 02 July 2015 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 101282801
- KR 101934651
- KR 101982667
- US 6875751 B
- US 7585851 B
- US 7964580 B


### Non-patent literature cited in the description


- **HE et al.** *Science,* 2005, vol. 310 (5750), 1022-1025
- *Pure Appl. Chem.,* 1976, vol. 45, 11-30
- Design of Prodrugs. Elsevier, 1985